(19) Europäisches Patentamt
European Patent Office
Office européen des brevets

(11) Veröffentlichungsnummer: **0 192 611**
**A2**

(12) # EUROPÄISCHE PATENTANMELDUNG

(21) Anmeldenummer: 86810087.6

(22) Anmeldetag: 17.02.86

(51) Int. Cl.[4]: C 07 K 9/00
A 61 K 37/02

(30) Priorität: 20.02.85 CH 773/85
31.10.85 CH 4679/85

(43) Veröffentlichungstag der Anmeldung:
27.08.86 Patentblatt 86/35

(84) Benannte Vertragsstaaten:
AT BE CH DE FR GB IT LI LU NL SE

(71) Anmelder: CIBA-GEIGY AG
Klybeckstrasse 141
CH-4002 Basel(CH)

(72) Erfinder: Wacker, Oskar, Dr.
Löwenbergstrasse 60
CH-4059 Basel(CH)

(72) Erfinder: Hartmann, Albert, Dr.
Steingasse 21A
D-7889 Grenzach(DE)

(72) Erfinder: Stanek, Jaroslav, Dr.
Hangstrasse 9
CH-4144 Arlesheim(CH)

(72) Erfinder: Fechtig, Bruno, Dr.
Hinterlindenweg 1
CH-4153 Reinach(CH)

(72) Erfinder: Baschang, Gerhard, Dr.
Bückenweg 7
CH-4126 Bettingen(CH)

(54) **Acylierte Hexosederivate und Verfahren zu ihrer Herstellung.**

(57) Beschrieben sind Verbindungen der Formel I,

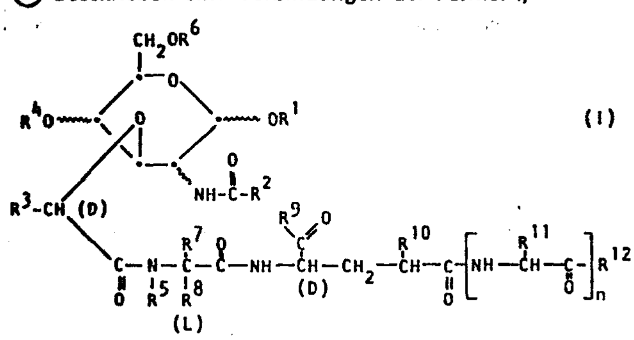

(I)

worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy, 3-Cholesteryloxy oder 2-Trimethylammonio-ethyloxy, $R^{10}$ Wasserstoff, Carboxy, Niederalkoxycarbonyl oder Arylniederalkoxycarbonyl und $R^{11}$ Wasserstoff oder unsubstituiertes oder durch Amino, Hydroxy, Niederalkanoylamino, Niederalkanoyloxy, 2-Benzyloxycarbonylamino-ethyl-sulfinyl, 2-Benzyloxycarbonylamino-ethyl-sulfonyl, 2-Niederalkoxycarbonylamino-ethyl-sulfinyl, 2-Niederalkoxycarbonylamino-ethyl-sulfonyl oder Guanidino substituiertes Niederalkyl bedeuten, mit der Massgabe, dass mindestens einer der Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im Alkoxyteil verschieden ist, und Salze von solchen Verbindungen mit mindestens einer salzbildenden Gruppe, die zur Prophylaxe und Therapie von Virusinfektionen verwendet werden können.

EP 0 192 611 A2

0192611

CIBA-GEIGY AG
Basel (Schweiz)

4-15272/1+2/+

# Acylierte Hexosederivate und Verfahren zu ihrer Herstellung

Die Erfindung betrifft 1,4,6-tri-O-acylierte Muramylpeptid- und analoge D-Mannose- oder D-Galactosederivate, Verfahren zu ihrer Herstellung, pharmazeutische Präparate, enthaltend diese Derivate, und deren Verwendung als Arzneimittel.

Die Erfindung betrifft insbesondere die Verbindungen der Formel I,

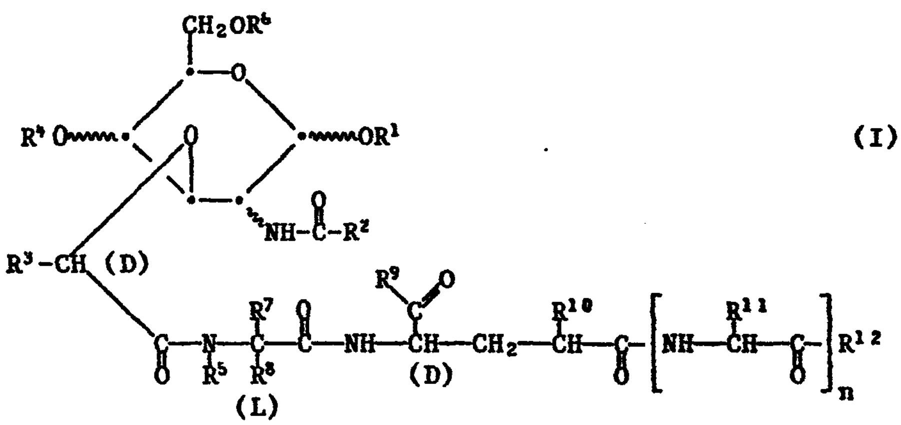

(I)

worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unab-

hängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy, 3-Cholesteryloxy oder 2-Trimethylammonio-ethyloxy, $R^{10}$ Wasserstoff, Carboxy, Niederalkoxycarbonyl oder Arylniederalkoxycarbonyl und $R^{11}$ Wasserstoff oder unsubstituiertes oder durch Amino, Hydroxy, Niederalkanoylamino, Niederalkanoyloxy, 2-Benzyloxycarbonylamino-ethyl-sulfinyl, 2-Benzyloxycarbonylamino-ethyl-sulfonyl, 2-Niederalkoxycarbonylamino-ethyl-sulfinyl, 2-Niederalkoxycarbonylamino-ethyl-sulfonyl oder Guanidino substituiertes Niederalkyl bedeuten, mit der Massgabe, dass mindestens einer der Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im Alkoxyteil verschieden ist, und Salze von solchen Verbindungen mit mindestens einer salzbildenden Gruppe.

Vorzugsweise leitet sich der Hexoseteil von D-Glucose ab.

Im Falle asymmetrischer Substitution ist die Konfiguration an den Atomen C-$R^3$, C-$R^8$ beziehungsweise C-CO-$R^9$ (D), (L) bzw. (D), wie in Formel I angegeben. Die Konfiguration am C-$R^{11}$ ist im Falle asymmetrischer Substitution (L) oder (D), vorzugsweise (L).

Niederalkanoyl $R^1$, $R^4$ und $R^6$ ist insbesondere $C_{2-6}$-Alkanoyl, z.B. n-Hexanoyl, vorzugsweise $C_{2-4}$-Alkanoyl, z.B. Butyryl, Propionyl oder vorzugsweise Acetyl.

Niederalkyl $R^2$ ist vorzugsweise $C_{1-4}$-, insbesondere $C_{1-2}$-Alkyl.

Niederalkyl $R^3$, $R^5$ oder $R^7$ ist vorzugsweise $C_{1-3}$-Alkyl, insbesondere Methyl.

Unsubstituiertes Niederalkyl $R^8$ oder $R^{11}$ ist vorzugsweise $C_{1-4}$-Alkyl, z.B. Ethyl, Isopropyl, 2-Methylpropyl, sek.Butyl oder insbesondere Methyl.

Durch Phenyl, Hydroxy, Mercapto oder Niederalkylthio, wie insbesondere Methylthio, substituiertes Niederalkyl $R^8$ ist vorzugsweise entsprechend substituiertes $C_{1-2}$-Alkyl, z.B. Benzyl, Hydroxymethyl, 1-Hydroxy-ethyl, Mercaptomethyl oder 2-Methylthio-ethyl.

Alkoxy $R^9$ oder $R^{12}$ ist vorzugsweise $C_{1-4}$-Alkoxy, z.B. Methoxy, n-Butyloxy oder tert.Butyloxy.

Aryl als Teil von Arylniederalkoxy, Arylniederalkoxycarbonyl oder Aroyloxyniederalkoxy ist insbesondere unsubstituiertes oder durch einen oder mehrere, z.B. 1-3, der untengenannten Arylsubstituenten substituiertes Phenyl oder Naphthyl, vorzugsweise unsubstituiertes Phenyl.

Arylsubstituenten sind insbesondere Niederalkyl, z.B. Methyl, Phenyl, Halogen, Hydroxy, Niederalkoxy, z.B. Methoxy, Niederalkanoyloxy, z.B. Acetoxy, Amino, Mono- oder Diniederalkylamino, z.B. Mono- oder Dimethylamino oder Niederalkanoylamino, z.B. Acetylamino.

Arylniederalkoxy $R^9$ und/oder $R^{12}$ ist durch einen oder mehrere, z.B. 1-3, vorzugsweise 1 oder 2, Arylreste substituiertes Niederalkoxy, z.B. Arylmethoxy, wie insbesondere Benzyloxy oder Benzhydryloxy.

Arylniederalkoxycarbonyl $R^{10}$ ist durch einen oder mehrere, z.B. 1-3, vorzugsweise 1 oder 2, Arylreste substituiertes Niederalkoxycarbonyl, z.B. Arylmethoxycarbonyl, wie insbesondere Benzyloxycarbonyl oder Benzhydryloxycarbonyl.

Niederalkoxycarbonyl $R^{10}$ ist vorzugsweise Alkoxycarbonyl mit bis zu 5 C-Atomen, z.B. Methoxycarbonyl, n-Butyloxycarbonyl oder tert. Butyloxycarbonyl.

Durch Amino oder Niederalkanoylamino substituiertes Niederalkyl $R^{11}$ ist vorzugsweise 4-Amino-n-butyl oder 4-Niederalkanoylamino-n-butyl. Durch 2-Benzyloxycarbonylamino-ethyl-sulfinyl oder -sulfonyl oder

durch 2-Niederalkoxycarbonylamino-ethyl-sulfinyl oder -sulfonyl substituiertes Niederalkyl $R^{11}$ ist vorzugsweise entsprechend substituiertes Methyl, z.B. $C_6H_5-CH_2-O-C(=O)-NH-CH_2-CH_2-S(=O)-CH_2-$ oder $C_6H_5-CH_2-O-C(=O)-NH-CH_2-CH_2-SO_2-CH_2-$. Durch Guanidino substituiertes Niederalkyl $R^{11}$ ist vorzugsweise 3-Guanidino-n-propyl.

Alkanoyloxyniederalkoxy $R^9$ oder $R^{12}$ ist insbesondere Niederalkanoyloxymethoxy, z.B. Pivaloyloxymethoxy.

Aroyloxyniederalkoxy $R^9$ oder $R^{12}$ ist insbesondere Aroyloxymethoxy, z.B. Benzoyloxymethoxy.

Durch Hydroxy oder Niederalkanoyloxy substituiertes Niederalkyl $R^{11}$ ist insbesondere entsprechend substituiertes $C_{1-2}$-Alkyl, z.B. Hydroxymethyl, 1-Hydroxy-ethyl, Niederalkanoyloxymethyl oder 1-Niederalkanoyloxy-ethyl.

3-Cholesteryloxy ist das durch Abstraktion von Wasserstoff von der Hydroxygruppe des Cholesterins gebildete Radikal.

Die vor- und nachstehend verwendeten Allgemeinbegriffe haben vorzugsweise folgende Bedeutungen:

Das Präfix "Nieder" bezeichnet Reste bis und mit 7, insbesondere bis und mit 4, Kohlenstoffatomen.

Halogen ist insbesondere Chlor oder Brom, ferner Fluor oder Iod.

Salzbildende Gruppen in einer Verbindung der Formel I sind saure Gruppen, z.B. freie Carboxylgruppen, oder basische Gruppen, wie insbesondere freie Amino- oder Guanidinogruppen. Verbindungen der Formel I, die eine Trimethylammoniogruppe aufweisen, liegen in Salzform vor. Je nach der Art der salzbildenden Gruppe bilden die Verbindungen der Formel I Metall-oder Ammoniumsalze oder Säureadditionssalze. Salze einer Verbindung der Formel I sind vorzugsweise pharmazeutisch verwendbar und nicht toxisch, z.B. Alkalimetall- oder

Erdalkalimetallsalze, z.B. Natrium-, Kalium-, Magnesium- oder Calciumsalze, oder Salze mit Ammoniak oder geeigneten organischen Aminen, wobei in erster Linie aliphatische, cycloaliphatische, cycloaliphatisch-aliphatische oder araliphatische primäre, sekundäre oder tertiäre Mono-, Di- oder Polyamine, sowie heterocyclische Basen für die Salzbildung in Frage kommen, wie Niederalkylamine, z.B. Triethylamin, Hydroxyniederalkylamine, z.B. 2-Hydroxy-ethylamin, Bis-(2-hydroxyethyl)-amin, 2-Hydroxy-ethyl-diethyl-amin oder Tri-(2-hydroxyethyl)-amin, basische aliphatische Ester von Carbonsäuren, z.B. 4-Amino-benzoesäure-2-diethylaminoethylester, Niederalkylenamine, z.B. 1-Ethylpiperidin, Cycloalkylamine, z.B. Dicyclohexylamin, oder Benzylamine, z.B. N,N'-Dibenzylethylendiamin, ferner Basen vom Pyridintyp, z.B. Pyridin, Collidin oder Chinolin. Verbindungen der Formel I mit mindestens einer basischen Gruppe können Säureadditionssalze, z.B. mit anorganischen Säuren, wie Salzsäure, Schwefelsäure oder Phosphorsäure, oder mit geeigneten organischen Carbon- oder Sulfonsäuren, z.B. Trifluoressigsäure, sowie mit Aminosäuren, wie Arginin und Lysin, bilden. Bei Anwesenheit von mehreren sauren oder basischen Gruppen können Mono-oder Polysalze gebildet werden. Verbindungen der Formel I mit einer sauren, z.B. freien Carboxylgruppe, und einer freien basischen, z.B. einer Aminogruppe, können auch in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze Verwendung finden. Zur therapeutischen Anwendung gelangen jedoch nur die pharmazeutisch verwendbaren, nicht-toxischen Salze, die deshalb bevorzugt werden.

Aus der Französischen Patentanmeldung Nr. 74 22 909 mit der Publikationsnummer 2 292 486 sind Muramylpeptide vom Typ der Verbindung N-Acetyl-muramyl-L-alanyl-D-isoglutamin ("MDP") bekannt. Diese Verbindungen sind als immunologische Adjuvanzien beschrieben, d.h. sie können in Mischung mit Impfstoffen dazu benützt werden, den Impferfolg zu verbessern. Eigene Untersuchungen, z.B. bei mit

Influenza-Viren infizierten Mäusen, haben ergeben, dass N-Acetyl-muramyl-L-alanyl-D-isoglutamin per se, d.h. ohne Beimengung von Impfstoffen, bei der Prophylaxe und Therapie von Virusinfektionen unwirksam ist. Auch ist die Verwendbarkeit strukturell einfacher Muramylpeptide und ihrer Analogen gegen Virusinfektionen bisher nicht beschrieben.

Der vorliegenden Erfindung liegt die Aufgabe zugrunde, strukturell verhältnismässig einfache und daher relativ leicht herstellbare Muramylpeptid-Derivate zur Verfügung zu stellen, die nach Verabreichung an Warmblüter hinsichtlich der Prophylaxe und Therapie von Virusinfektionen hochwirksam sind.

Erfindungsgemäss wurde überraschenderweise gefunden, dass die obengenannten Verbindungen der Formel I und ihre pharmazeutisch verwendbaren Salze sowohl zur Prophylaxe als auch zur Therapie von Virusinfektionen hervorragend geeignet sind, wie sich z.B. aus Tierversuchen, wie sie im Beispielteil exemplifiziert sind, ergibt. In diesen Tierversuchen werden Tiere, wie Mäuse oder Meerschweinchen, mit den verschiedensten Virusarten in einer Dosis, die für alle oder die grosse Mehrzahl der unbehandelten (Kontroll)Tiere letal ist, z.B. $LD_{80-90}$, infiziert und der Infektionsverlauf bei den unbehandelten Kontrolltieren im Vergleich zu Tieren beobachtet, die vor, gleichzeitig mit oder nach der Infektion mit einer der obengenannten Verbindungen oder einem Salz davon behandelt werden.

Dabei zeigt sich, dass ein prophylaktischer Effekt bei Verabreichung der Verbindungen der Formel I schon mehrere Tage bis zu einigen, z.B. vier, Wochen vor der Infektion, und ein therapeutischer Effekt noch bei Verabreichung mehrere Tage, z.B. 1 Woche, nach der Infektion, eintritt.

Die Verbindungen der Formel I sind im obengenannten Test an der Maus bereits im Dosisbereich zwischen 0,0001 mg/kg und 0,1 mg/kg wirksam.

Bemerkenswert ist auch das breite virale Spektrum, gegen das die obengenannten Verbindungen wirksam sind.

Die Verbindungen der Formel I können insbesondere zur Prophylaxe und Therapie von Krankheiten verwendet werden, die durch die nachstehend näher bezeichneten Viren hervorgerufen werden [zur Nomenklatur vgl. J.L.Melnick, Prog. med. Virol. 26, 214-232 (1980) und 28, 208-221 (1982)]:

DNA-Viren mit kubischer Symmetrie und nacktem Nukleokapsid, DNA-Viren mit umhülltem Virion sowie RNA-Viren mit kubischer und solche mit helikaler Symmetrie des Kapsids.

Bevorzugterweise verwendet man die Verbindungen der Formel I im Falle von DNA-Viren mit umhülltem Virion und kubischer Symmetrie des Kapsids, im Falle von RNA-Viren mit kubischer Symmetrie des Kapsids und nacktem Virion und im Falle von RNA-Viren mit helikaler Symmetrie des Kapsids, in denen die Nukleokapsidhülle bei der Oberflächenmembran gelegen ist, aber auch im Falle von Adenoviridae, Poxviridae und Coronaviridae, wie insbesondere menschlichen Coronaviren.

In erster Linie verwendet man die Verbindungen der Formel I im Falle von Herpesviridae, Picornaviridae und Myxoviren, aber auch im Falle von Mastadenoviren, wie insbesondere menschlichen Adenoviren, im Falle von Chordopoxvirinae, wie hauptsächlich Orthopoxviren, wie insbesondere z.B. Vacciniaviren, im Falle von Reoviridae, vornehmlich (insbesondere menschlichen) Rotaviren, sowie im Falle von Caliciviridae und Rhabdoviridae, wie in erster Linie Vesiculoviren des Menschen sowie von Pferden, Rindern und Schweinen.

Hauptsächlich verwendet man die Verbindungen der Formel I im Falle von Alphaherpesvirinae, wie Varicellaviren, z.B. menschlichen Varicella-Zoster Viren, Rhinoviren, Cardioviren und Orthomyxoviridae, aber auch im Falle von Betaherpesvirinae, wie insbesondere menschlichen Cytomegaloviren, im Falle von Aphthoviren, in erster

Linie Apthoviren von Paarhufern, wie hauptsächlich von Rindern, sowie im Falle von Paramyxoviridae, wie in erster Linie Pneumoviren, z.B. respiratorischen Syncitialviren des Menschen, und wie daneben Morbilliviren oder Paramyxoviren, wie Parainfluenzaviren, z.B. menschlichen Parainfluenzaviren, einschliesslich der Sendaiviren sowie im Falle von Arboviren oder Vesiculoviren, z.B. Vesicular stomatitis Viren.

In allererster Linie verwendet man die Verbindungen der Formel I im Falle von Simplexviren, z.B. menschlichen Herpes simplex Viren der Typen 1 und 2, im Falle von menschlichen Encephalomyocarditisviren, im Falle von Influenzaviren, wie hauptsächlich Influenza A und Influenza B Viren, im Falle von Vaccinia und Parainfluenza Viren und ganz besonders im Falle der in den Beispielen genannten Viren.

Die Verbindungen der Formel I können zur Prophylaxe und Therapie von Virusinfektionen, insbesondere bei Warmblütern einschliesslich des Menschen, verwendet werden, indem man sie enteral oder parenteral, in erster Linie zusammen mit geeigneten Hilfs- oder Trägerstoffen, appliziert. Bevorzugterweise appliziert man sie auf die Schleimhaut, z.B. intranasal, rektal, vaginal oder auf die Bindehaut des Auges, oder oral. Der antivirale Effekt tritt jedoch auch bei Applikation auf anderen Wegen, z.B. subkutan, intravenös, intramuskulär oder bei Applikation auf die normale Haut ein.

Die Dosierung des Wirkstoffes hängt u.a. von der Warmblüterspezies, der Abwehrlage des Organismus, der Applikationsweise und der Art des Virus ab. Die Dosis-Wirkungsbeziehung ist relativ schwach ausgeprägt.

Zur Vorbeugung appliziert man eine einmalige Dosis von etwa 0,01 mg bis etwa 10 mg, vorzugsweise 0,05 bis 1 mg, z.B. 0,2 mg, Wirkstoff an einen Warmblüter von etwa 70 kg Körpergewicht, z.B. den Menschen. Die prophylaktische Wirkung dieser Dosis erstreckt sich über mehrere Wochen. Bei Bedarf, z.B. in Zeiten erhöhter Ansteckungsgefahr, kann man die Verabreichung dieser Dosis wiederholen.

Die therapeutische Dosis für Warmblüter von etwa 70 kg Körpergewicht liegt zwischen 0,1 mg und 25 mg, vorzugsweise zwischen 0,1 und 1 mg, z.B. bei 0,5 mg, insbesondere bei oraler Applikation. Die Dosierung bei topischer, insbesondere intranasaler Applikation, liegt bis zum Faktor 10 niedriger. Bei Bedarf kann man die Verabreichung der Verbindungen der Formel I bis zum Eintritt einer Besserung der Erkrankung wiederholen. Oft genügt jedoch eine einmalige Applikation.

Die Verbindungen der Formel I besitzen ausserdem Antitumoreigenschaften. Diese beruhen auf ihrer Fähigkeit, z.B. inkorporiert in multilamellären Liposomen oder in phosphatgepufferter physiologischer Kochsalzlösung (PBS), Makrophagen derart zu aktivieren, dass diese körpereigenen Abwehrzellen in der Lage sind, Tumorzellen abzutöten (Zytotoxizität) oder an ihrem Wachstum zu hindern (Zytostase). Die Induktion tumorizider und tumoristatischer Alveolarmakrophagen der Ratte in vitro und in situ lässt sich z.B. mit folgendem Versuch zeigen:

Alveolarmakrophagen werden durch Lungenspülung mit Kulturmedium erhalten. Diese Makrophagen werden entweder durch Injektion der Testsubstanzen in die Ratten (intravenös oder intranasal, in situ-Aktivierung) oder durch eine 24stündige Vorinkubation mit einer Verbindung der Formel I im $CO_2$-Inkubator (in vitro-Aktivierung) aktiviert. Die so aktivierten Makrophagen werden nun für weitere 72 Stunden mit Tumorzellen inkubiert.

Um tumorizide Aktivitäten der Makrophagen zu messen, werden die Tumorzellen vor der 72 Stunden-Inkubation mit $^{125}$I-Iododeoxyuridin markiert. Die nicht abgetöteten Tumorzellen können nach Wegwaschen der durch lysierte Tumorzellen freigewordenen Radioaktivität anhand der verbleibenden Radioaktivität gemessen werden.

Um tumoristatische Aktivitäten der Makrophagen zu erfassen, wird den Kulturen 8 Stunden vor dem Ende der 72 Stunden-Inkubation $^3H$-Thymidin zugesetzt und danach die $^3H$-Thymidininkorporation in die Tumorzellen gemessen. In vitro können die Substanzen sowohl gelöst in PBS als auch inkorporiert in Liposomen bereits in Dosen von 20 Nanogramm/0,2 ml Kultur tumorizide Ratten-Alveolarmakrophagen induzieren. Bei Ratten bewirkt eine einmalige intravenöse Applikation der Verbindungen inkorporiert in Liposomen bei einer Dosis von 160 µg/Tier eine Induktion von tumoriziden und tumoristatischen Alveolarmakrophagen. Darüberhinaus bewirkt eine einmalige intranasale Applikation der Substanzen in PBS bei einer Dosis von 25 µg/Ratte die Induktion von tumoriziden Alveolarmakrophagen.

Die Verbindungen der Formel I können somit bei Warmblütern einschliesslich des Menschen auch zur Therapie von Tumorerkrankungen verwendet werden, insbesondere z.B. zur Vermeidung der Bildung von Metastasen, z.B. bei operativer Entfernung des Primärtumors.

Die Erfindung betrifft insbesondere solche Verbindungen der Formel I, worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy oder 3-Cholesteryloxy, $R^{10}$ Wasserstoff, Carboxy, Niederalkoxycarbonyl oder Arylniederalkoxycarbonyl und $R^{11}$ Wasserstoff oder unsubstituiertes oder durch Amino oder Hydroxy substituiertes Niederalkyl bedeuten, mit der Massgabe, dass mindestens einer der Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im Alkoxyteil verschieden ist, und Salze von solchen Verbindungen mit mindestens einer salzbildenden Gruppe.

Bevorzugt sind die obengenannten Verbindungen der Formel I, worin $R^9$ und $R^{12}$ unabhängig voneinander Hydroxy, Amino, $C_{1-10}$-Alkoxy, Alkanoyloxyniederalkoxy mit bis zu 16 C-Atomen, 3-Cholesteryloxy oder im Phenylteil jeweils unsubstituiertes oder durch Niederalkyl, z.B. Methyl, Phenyl, Halogen, Hydroxy, Niederalkoxy, z.B. Methoxy, Niederalkanoyloxy, z.B. Acetoxy, Amino, Mono- oder Diniederalkylamino, z.B. Mono- oder Dimethylamino, oder Niederalkanoylamino, z.B. Acetylamino, substituiertes Phenyl- oder Benzoyloxy-niederalkoxy, und $R^{10}$ Wasserstoff, Carboxy, Niederalkoxycarbonyl oder im Phenylteil unsubstituiertes oder durch Niederalkyl, z.B. Methyl, Phenyl, Halogen, Hydroxy, Niederalkoxy, z.B. Methoxy, Niederalkanoyloxy, z.B. Acetoxy, Amino, Mono- oder Diniederalkylamino, z.B. Mono- oder Dimethylamino, oder Niederalkanoylamino, z.B. Acetylamino, substituiertes Phenylniederalkoxycarbonyl bedeuten, und die übrigen Substituenten die obengenannten Bedeutungen haben, mit der Massgabe, dass mindestens einer der Reste $R^9$ und $R^{12}$ von Hydroxy, Amino und Niederalkoxy oder $R^{10}$ von Wasserstoff, Carboxy und Niederalkoxycarbonyl verschieden ist, und Salze von solchen Verbindungen mit mindestens einer salzbildenden Gruppe.

Besonders bevorzugt sind Verbindungen der Formel I, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-4}$-Alkyl oder Phenyl, $R^3$, $R^5$ und $R^7$ unabhängig voneinander Wasserstoff oder Methyl, oder $R^5$ mit $R^8$ zusammen Trimethylen und $R^7$ Wasserstoff, $R^8$ Wasserstoff, $C_{1-4}$-Alkyl oder durch Phenyl, Hydroxy, Mercapto oder Methylthio substituiertes $C_{1-2}$-Alkyl, $R^9$ und $R^{12}$ unabhängig voneinander Hydroxy, Amino, Niederalkoxy, Phenylniederalkoxy, Niederalkanoyloxyniederalkoxy, Benzoyloxyniederalkoxy oder 3-Cholesteryloxy, $R^{10}$ Wasserstoff, Carboxy, Niederalkoxycarbonyl oder Phenylniederalkoxycarbonyl und $R^{11}$ Wasserstoff oder unsubstituiertes oder durch Amino oder Hydroxy substituiertes $C_{1-4}$-Alkyl bedeuten, mit der Massgabe, dass mindestens einer der Reste $R^9$ und $R^{12}$ von Hydroxy, Amino und Niederalkoxy

oder $R^{10}$ von Wasserstoff, Carboxy und Niederalkoxycarbonyl verschieden ist, und Salze von solchen Verbindungen mit mindestens einer salzbildenden Gruppe.

Sehr bevorzugt sind Verbindungen der Formel I, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-2}$-Alkyl oder Phenyl, $R^3$, $R^5$ und $R^7$ unabhängig voneinander Wasserstoff oder Methyl, $R^8$ $C_{1-4}$-Alkyl, $R^9$ und $R^{12}$ unabhängig voneinander Hydroxy, Amino, $C_{1-4}$-Alkoxy, Phenyl-methoxy, Niederalkanoyloxymethoxy, Benzoyloxymethoxy oder 3-Cholesteryloxy, $R^{10}$ Wasserstoff, Carboxy, Alkoxycarbonyl mit bis zu 5 C-Atomen oder Phenylmethoxycarbonyl und $R^{11}$ $C_{1-4}$-Alkyl bedeuten, mit der Massgabe, dass mindestens einer der Reste $R^9$ und $R^{12}$ von Hydroxy, Amino und $C_{1-4}$-Alkoxy oder $R^{10}$ von Wasserstoff, Carboxy und Alkoxycarbonyl mit bis zu 5 C-Atomen verschieden ist, und Salze von solchen Verbindungen mit mindestens einer salzbildenden Gruppe.

Ganz besonders bevorzugt sind Verbindungen der Formel I, worin sich der Hexoseteil von D-Glucose ableitet, n für 0 oder 1 steht, $R^1$, $R^4$ und $R^6$ Acetyl oder Butyryl, $R^2$ $C_{1-2}$-Alkyl oder Phenyl, $R^3$ Wasserstoff oder Methyl, $R^5$ und $R^7$ Wasserstoff, $R^8$ $C_{1-3}$-Alkyl, $R^9$ Amino, $C_{1-4}$-Alkoxy, Pivaloyloxymethoxy oder Mono- oder Diphenylmethoxy, $R^{10}$ Wasserstoff, $R^{11}$ Methyl und $R^{12}$ Mono- oder Diphenylmethoxy oder 3-Cholesteryloxy bedeuten.

Ganz besonders bevorzugt sind auch Verbindungen der Formel I, worin sich der Hexoseteil von D-Glucose ableitet, n für 0 oder 1 steht, $R^1$, $R^4$ und $R^6$ $C_{2-6}$-Alkanoyl, $R^2$ $C_{1-4}$-Alkyl oder Phenyl, $R^3$, $R^5$ und $R^7$ unabhängig voneinander Wasserstoff oder Methyl, $R^8$ $C_{1-4}$-Alkyl, $R^9$ Amino, Niederalkoxy, Pivaloyloxymethoxy, Diphenylmethoxy, Benzyloxy oder 2-Trimethylammonio-ethoxy, $R^{10}$ Wasserstoff oder Niederalkoxycarbonyl, $R^{11}$ $C_{1-4}$-Alkyl, Niederalkanoyloxymethyl oder (2-Benzyloxycarbonylamino-ethyl)-sulfonyl-methyl und $R^{12}$ Amino, Niederalkoxy, Pivaloyloxymethoxy, Diphenylmethoxy, Benzyloxy, 2-Trimethylammonioethoxy, 3-Cholesteryloxy oder Benzoyloxymethoxy bedeuten, mit der

Massgabe, dass mindestens einer der Reste $R^9$ und $R^{12}$ von Amino und Niederalkoxy verschieden ist, und Salze von solchen zur Salzbildung fähigen Verbindungen.

Ganz besonders bevorzugt sind vor allem Verbindungen der Formel I, worin mindestens einer der Reste $R^9$ und $R^{12}$ für Pivaloyloxymethoxy, Diphenylmethoxy, Benzyloxy, 2-Trimethylammonio-ethoxy, 3-Cholesteryloxy oder Benzoyloxymethoxy steht und der andere der Reste $R^9$ und $R^{12}$ die obengenannte Bedeutung hat, und Salze von solchen zur Salzbildung fähigen Verbindungen.

Am meisten bevorzugt sind die in den Beispielen beschriebenen Verbindungen der Formel I.

Die Verbindungen der Formel I werden in an sich bekannter Weise hergestellt:

Sie werden z.B. hergestellt, indem man

a) eine Verbindung der Formel II,

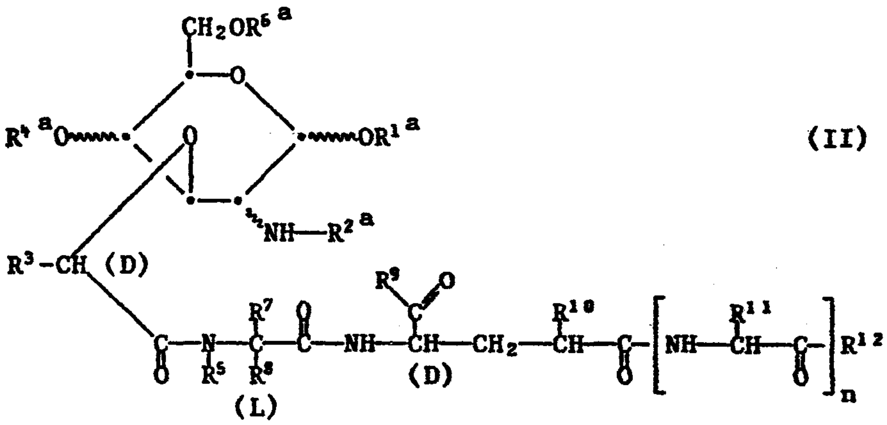

(II)

worin mindestens einer der Reste $R^{1a}$, $R^{2a}$, $R^{4a}$ und $R^{6a}$ für Wasserstoff steht und die übrigen dieser Reste die Bedeutungen von $R^1$, der Gruppe $R^2$-C(=O)-, $R^4$ bzw. $R^6$ haben, und die restlichen Substituenten die obengenannten Bedeutungen haben, wobei in einer Verbindung der Formel II vorhandene freie funktionelle Gruppen mit

Ausnahme der Gruppen, die an der Reaktion teilnehmen sollen, erforderlichenfalls durch leicht abspaltbare Schutzgruppen geschützt sind, mit einem den einzuführenden Rest $R^1$, $R^2$-C(=O)-, $R^4$ oder $R^6$ übertragenden Acylierungsmittel umsetzt und vorhandene Schutzgruppen erforderlichenfalls abspaltet, oder

b) eine Verbindung der Formel III,

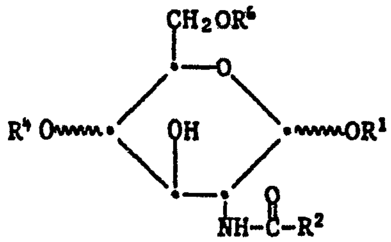

(III)

worin die Substituenten die obengenannten Bedeutungen haben, oder ein reaktionsfähiges Derivat davon mit einer Verbindung der Formel IV,

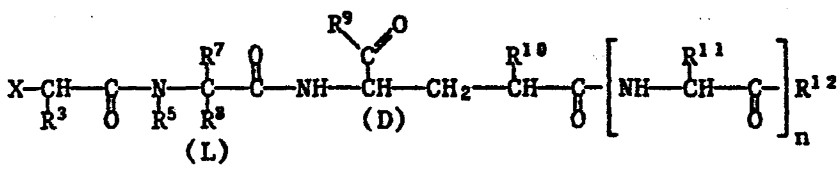

(IV)

worin X eine reaktionsfähige veresterte Hydroxygruppe bedeutet, und die restlichen Substituenten die obengenannten Bedeutungen haben, wobei in einer Verbindung der Formel IV vorhandene freie funktionelle Gruppen mit Ausnahme von X erforderlichenfalls durch leicht abspaltbare Schutzgruppen geschützt sind, umsetzt und vorhandene Schutzgruppen erforderlichenfalls abspaltet, oder

c) eine Verbindung der Formel V,

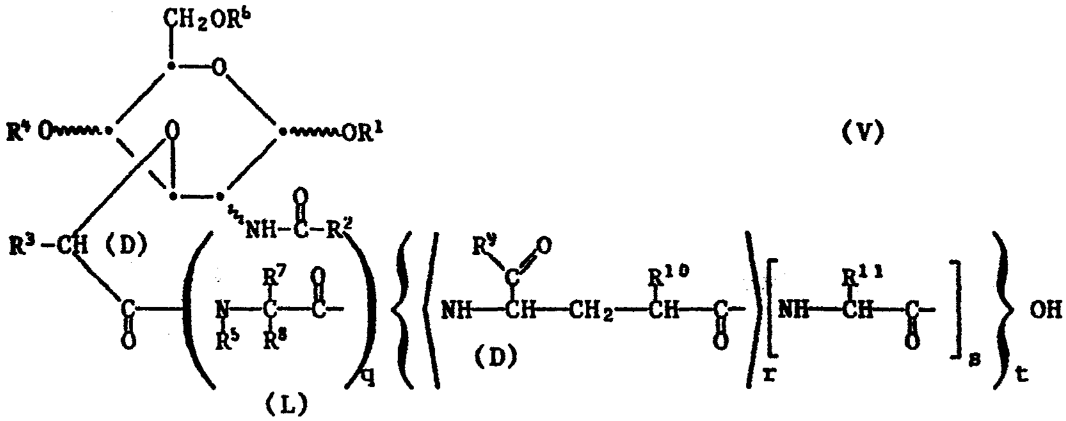

(V)

worin q, r, s und t unabhängig voneinander für 0 oder 1 stehen und worin die Substituenten die obengenannten Bedeutungen haben, wobei in einer Verbindung der Formel V vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit einer Verbindung der Formel VI,

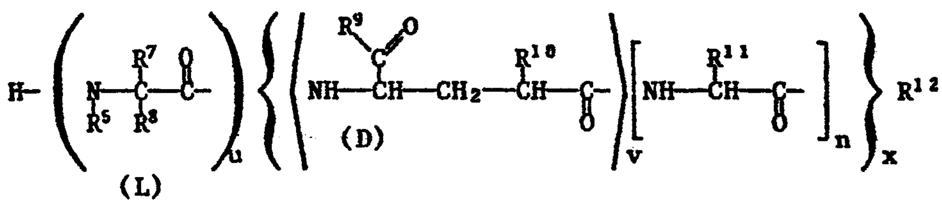

(VI)

worin u, v und x unabhängig voneinander für 0 oder 1 stehen und die übrigen Symbole und Substituenten die obengenannten Bedeutungen haben, wobei in einer Verbindung der Formel VI vorhandene freie funktionelle Gruppen mit Ausnahme der Gruppe, die an der Reaktion teilnehmen soll, erforderlichenfalls durch leicht abspaltbare Schutzgruppen geschützt sind, wobei u, v und x für 1 stehen, wenn im Reaktionspartner der Formel V q und t für 0 stehen, oder u für 0 und v und x für 1 stehen, wenn q für 1 und t für 0 stehen, oder u und v für 0 und x für 1 stehen, wenn q, r und t für 1 und s für 0 stehen oder (zur Herstellung von Verbindungen der Formel I, worin n für 1

steht) u und x für 0 stehen, wenn q, r, s und t für 1 stehen, oder mit einem reaktionsfähigen Derivat davon umsetzt und vorhandene Schutzgruppen erforderlichenfalls abspaltet, oder

d) zur Herstellung einer Verbindung der Formel I, worin $R^9$ eine der obengenannten Bedeutungen ausser Hydroxy und Amino hat und/oder $R^{10}$ für Niederalkoxycarbonyl oder Arylniederalkoxycarbonyl steht und die übrigen Substituenten die obengenannten Bedeutungen haben, eine Verbindung der Formel VII,

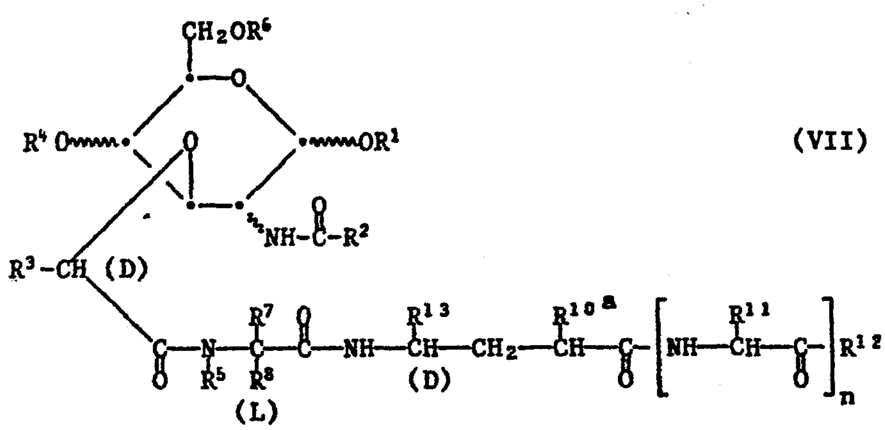

(VII)

worin mindestes einer der Reste $R^{10a}$ und $R^{13}$ für Carboxy steht und der andere der Reste $R^{10a}$ und $R^{13}$ die obengenannte Bedeutung von $R^{10}$ beziehungsweise von der Gruppe $R^9$-C(=O)- hat, und worin die übrigen Substituenten die obengenannten Bedeutungen haben, wobei in einer Verbindung der Formel VII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon verestert und vorhandene Schutzgruppen erforderlichenfalls abspaltet, oder

e) in einer Verbindung der Formel I, worin mindestens einer der Reste $R^8$, C(=O)-$R^9$, $R^{10}$, $R^{11}$ und C(=O)-$R^{12}$ in einer geschützten Form vorliegt, die nicht der Definition des gewünschten Endstoffes entspricht, die entsprechende(n) Schutzgruppe(n) abspaltet, oder

f) zur Herstellung einer Verbindung der Formel I, worin $R^9$ Amino bedeutet und die übrigen Substituenten die obengenannten Bedeutungen haben, eine Verbindung der Formel VIII,

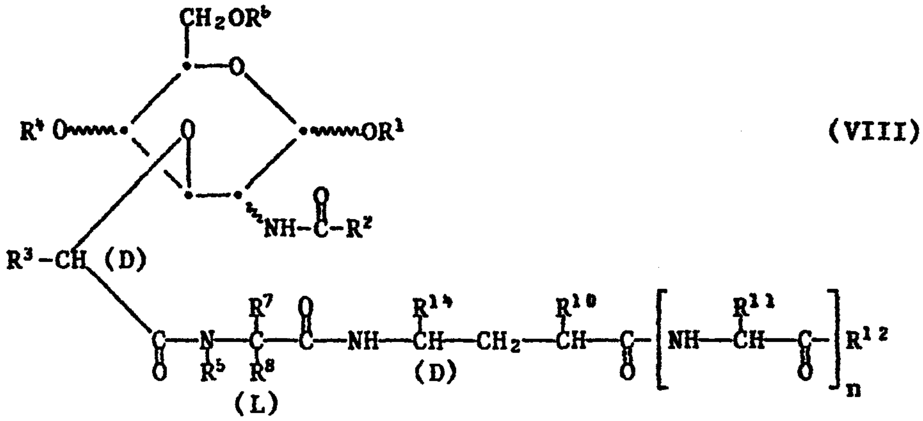

(VIII)

worin der Rest $R^{14}$ für Carboxy steht und die übrigen Substituenten die obengenannten Bedeutungen haben, wobei in einer Verbindung der Formel VIII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon amidiert und vorhandene Schutzgruppen erforderlichenfalls abspaltet,
und, wenn erwünscht, nach Durchführung eines der Verfahren a - f) eine erhaltene Verbindung der Formel I mit mindestens einer salzbildenden Gruppe in ihr Salz überführt oder ein erhaltenes Salz einer Verbindung der Formel I in die freie Verbindung umwandelt, und/oder ein erhaltenes Isomerengemisch auftrennt.

Bevorzugt sind die Verfahren a, c, d und e, ferner Verfahren f.

Die Durchführung der obengenannten Verfahrensvarianten wird im folgenden näher erläutert:

Verfahren a:
Bevorzugterweise wird Verfahren a) zur Einführung eines Acylrestes $R^1$, $R^4$ und/oder $R^6$ verwendet. Dabei geht man vorzugsweise von Verbindungen der Formel II aus, worin der Rest $R^{2a}$ für die Gruppe $R^2$-C(=O) steht.

In einer Verbindung der Formel II gegebenenfalls vorhandene freie funktionelle Gruppen, die vorzugsweise durch leicht abspaltbare Schutzgruppen geschützt werden, sind insbesondere freies Hydroxy oder Mercapto im Rest $R^8$, freies Carboxy $R^9$-C(=O)-, $R^{10}$ oder $R^{12}$-C(=O)- sowie freies Amino, Hydroxy oder Guanidino im Rest $R^{11}$. Fakultativ ist der Schutz von freiem Carbamoyl $R^9$-C(=O)- oder $R^{12}$-C(=O)-.

Schutzgruppen, ihre Einführung und Abspaltung sind beispielsweise beschrieben in "Protective Groups in Organic Chemistry", Plenum Press, London, New York 1973, und in "Methoden der organischen Chemie", Houben-Weyl, 4. Auflage, Bd. 15/1, Georg-Thieme-Verlag, Stuttgart 1974 sowie in Theodora W. Greene, "Protective Groups in Organic Synthesis, John Wiley & Sons, New York 1981. Charakteristisch für Schutzgruppen ist, dass sie leicht, d.h. ohne dass unerwünschte Nebenreaktionen stattfinden, z.B. solvolytisch, reduktiv, photolytisch oder auch unter physiologischen Bedingungen abspaltbar sind.

Hydroxyschutzgruppen sind z.B. Acylreste, wie gegebenenfalls, z.B. durch Halogen, substituiertes Niederalkanoyl, wie 2,2-Dichloracetyl, oder Acylreste von Kohlensäurehalbestern, insbesondere tert.-Butyloxycarbonyl, gegebenenfalls substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl, oder organische Silyl- oder Stannylreste, ferner leicht abspaltbare verethernde Gruppen, wie tert.-Niederalkyl, z.B. tert.-Butyl, 2-oxa- oder 2-thia-aliphatische oder cycloaliphatische Kohlenwasserstoffreste, in erster Linie 1-Niederalkoxyniederalkyl oder 1-Niederalkylthio-niederalkyl, z.B. Methoxymethyl, 1-Methoxy-ethyl, 1-Ethoxy-ethyl, Methylthiomethyl, 1-Methylthioethyl oder 1-Ethylthioethyl, oder 2-Oxa- oder 2-Thiacycloalkyl mit 5-6 Ringatomen, z.B. Tetrahydrofuryl oder 2-Tetrahydropyranyl oder entsprechende Thiaanaloge, sowie gegebenenfalls substituiertes 1-Phenylniederalkyl, wie gegebenenfalls substituiertes Benzyl oder Diphenylmethyl, wobei als Substituenten der Phenylreste z.B. Halogen, wie Chlor, Niederalkoxy, wie Methoxy und/oder Nitro in Frage kommen.

Carboxylgruppen sind üblicherweise in veresterter Form geschützt, wobei solche Estergruppierungen unter schonenden Bedingungen leicht spaltbar sind. In dieser Art geschützte Carboxylgruppen enthalten als veresternde Gruppen in erster Linie in 1-Stellung verzweigte oder in 1- oder 2-Stellung geeignet substituierte Niederalkylgruppen. Bevorzugte, in veresterter Form vorliegende Carboxylgruppen sind u.a. tert.-Niederalkoxycarbonyl, z.B. tert.-Butyloxycarbonyl, Arylmethoxycarbonyl mit einem oder zwei Arylresten, wobei diese gegebenenfalls z.B. durch Niederalkyl, wie tert.-Niederalkyl, z.B. tert.-Butyl, Niederalkoxy, wie Methoxy, Hydroxy, Halogen, z.B. Chlor, und/oder Nitro, mono- oder polysubstituierte Phenylreste darstellen, wie gegebenenfalls, z.B. wie oben erwähnt, substituiertes Benzyloxycarbonyl, z.B. 4-Methoxybenzyloxycarbonyl, oder 4-Nitrobenzyloxycarbonyl, oder gegebenenfalls, z.B. wie oben erwähnt, substituiertes Diphenylmethoxycarbonyl, z.B. Diphenylmethoxycarbonyl oder Di-(4-methoxyphenyl)-methoxycarbonyl, 1-Niederalkoxyniederalkoxycarbonyl, wie Methoxymethoxycarbonyl, 1-Methoxyethoxycarbonyl oder 1-Ethoxymethoxycarbonyl, 1-Niederalkylthioniederalkoxycarbonyl, wie 1-Methylthiomethoxycarbonyl oder 1-Ethylthioethoxycarbonyl, Aroylmethoxycarbonyl, worin die Aroylgruppe gegebenenfalls, z.B. durch Halogen, wie Brom, substituiertes Benzoyl darstellt, z.B. Phenacyloxycarbonyl, 2-Halogenniederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy,

Aryl, Halogen, und/oder Nitro substituierten, aliphatischen, araliphatischen, cycloaliphatischen oder aromatischen Kohlenwasserstoffrest, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Die oben und im folgenden erwähnten organischen Silyl- oder Stannylreste enthalten vorzugsweise Niederalkyl, insbesondere Methyl, als Substituenten der Silizium- oder Zinn-Atome. Entsprechende Silyl- oder Stannylgruppen sind in erster Linie Triniederalkylsilyl, insbesondere Trimethylsilyl, ferner Dimethyl-tert.-butyl-silyl, oder entsprechend substituiertes Stannyl, z.B. Tri-n-butylstannyl.

Bevorzugte geschützte Carboxylgruppen sind tert.-Niederalkoxycarbonyl, wie tert.-Butoxycarbonyl, und in erster Linie gegebenenfalls, z.B. wie oben erwähnt, substituiertes Benzyloxycarbonyl, wie 4-Nitrobenzyloxycarbonyl, oder Diphenylmethoxycarbonyl, vor allem 2-(Trimethylsilyl)-ethoxycarbonyl.

Eine geschützte Aminogruppe kann z.B. in Form einer leicht spaltbaren Acylamino-, Arylmethylamino-, verätherten Mercaptoamino-, 2-Acyl-niederalk-1-en-yl-amino-, Silyl- oder Stannylaminogruppe oder als Azidogruppe vorliegen.

In einer entsprechenden Acylaminogruppe ist Acyl beispielsweise der Acylrest einer organischen Carbonsäure mit z.B. bis zu 18 Kohlenstoffatomen, insbesondere einer gegebenenfalls,z.B. durch Halogen oder Aryl, substituierten Alkancarbonsäure oder gegebenenfalls, z.B. durch Halogen, Niederalkoxy oder Nitro, substituierten Benzoesäure, oder eines Kohlensäurehalbesters. Solche Acylgruppen sind beispielsweise Niederalkanoyl, wie Formyl, Acetyl oder Propionyl, Halogenniederalkanoyl, wie 2-Halogenacetyl, insbesondere 2-Chlor-, 2-Brom-, 2-Iod-, 2,2,2-Trifluor- oder 2,2,2-Trichloracetyl, gegebenenfalls, z.B. durch Halogen, Niederalkoxy oder Nitro substituiertes Benzoyl,

z.B. Benzoyl, 4-Chlorbenzoyl, 4-Methoxybenzoyl oder 4-Nitrobenzoyl, oder in 1-Stellung des Niederalkylrestes verzweigtes oder in 1- oder 2-Stellung geeignet substituiertes Niederalkoxycarbonyl, insbesondere tert.-Niederalkoxycarbonyl, z.B. tert.-Butyloxycarbonyl, Arylmethoxycarbonyl mit einem oder zwei Arylresten, die vorzugsweise gegebenenfalls, z.B. durch Niederalkyl, insbesondere tert.-Niederalkyl, wie tert.Butyl, Niederalkoxy, wie Methoxy, Hydroxy, Halogen, z.B. Chlor, und/oder Nitro, mono- oder polysubstituiertes Phenyl darstellen, wie gegebenenfalls substituiertes Benzyloxycarbonyl, z.B. 4-Nitro-benzyloxycarbonyl, oder substituiertes Diphenylmethoxycarbonyl, z.B. Benzhydryloxycarbonyl oder Di-(4-methoxyphenyl)-methoxycarbonyl, Aroylmethoxycarbonyl, worin die Aroylgruppe vorzugsweise gegebenenfalls, z.B. durch Halogen, wie Brom, substituiertes Benzoyl darstellt, z.B. Phenacyloxycarbonyl, 2-Halogen-niederalkoxycarbonyl, z.B. 2,2,2-Trichlorethoxycarbonyl, 2-Bromethoxycarbonyl oder 2-Iodethoxycarbonyl, oder 2-(trisubstituiertes Silyl)-ethoxycarbonyl, worin die Substituenten unabhängig voneinander je einen gegebenenfalls substituierten, z.B. durch Niederalkyl, Niederalkoxy, Aryl, Halogen oder Nitro substituierten, aliphatischen, araliphatischen, cycloaliphatischen oder aromatischen Kohlenwasserstoffrest mit bis zu 15 C-Atomen, wie entsprechendes, gegebenenfalls substituiertes Niederalkyl, Phenylniederalkyl, Cycloalkyl oder Phenyl, bedeuten, z.B. 2-Triniederalkylsilylethoxycarbonyl, wie 2-Trimethylsilylethoxycarbonyl oder 2-(Di-n-butyl-methyl-silyl)-ethoxycarbonyl, oder 2-Triarylsilylethoxycarbonyl, wie 2-Triphenylsilylethoxycarbonyl.

Weitere, als Aminoschutzgruppen in Frage kommende Acylreste sind auch entsprechende Reste organischer Phosphor-, Phosphon- oder Phosphinsäuren, wie Diniederalkylphosphoryl, z.B. Dimethylphosphoryl, Diethylphosphoryl, Di-n-propylphosphoryl oder Diisopropylphosphoryl, Dicycloalkylphosphoryl, z.B. Dicyclohexylphosphoryl, gegebenenfalls substituiertes Diphenylphosphoryl, z.B. Diphenylphosphoryl, gegebenenfalls, z.B. durch Nitro substituiertes Di-(phenylniederalkyl)-phosphoryl, z.B. Dibenzylphosphoryl oder Di-(4-nitrobenzyl)-phosphoryl, gegebenenfalls substituiertes

Phenyloxy-phenyl-phosphonyl, z.B. Phenyloxyphenyl-phosphonyl, Diniederalkylphosphinyl, z.B. Diethylphosphinyl, oder gegebenenfalls substituiertes Diphenylphosphinyl, z.B. Diphenylphosphinyl.

In einer Arylmethylaminogruppe, die eine Mono-, Di- oder insbesondere Triarylmethylaminogruppe darstellt, sind die Arylreste insbesondere gegebenenfalls substituierte Phenylreste. Solche Gruppen sind beispielsweise Benzyl-, Diphenylmethyl- und insbesondere Tritylamino.

Eine verätherte Mercaptogruppe in einer mit einem solchen Rest geschützten Aminogruppe ist in erster Linie Arylthio oder Arylniederalkylthio, worin Aryl insbesondere gegebenenfalls, z.B. durch Niederalkyl, wie Methyl oder tert.-Butyl, Niederalkoxy, wie Methoxy, Halogen, wie Chlor, und/oder Nitro substituiertes Phenyl ist. Eine entsprechende Aminoschutzgruppe ist z.B. 4-Nitrophenylthio.

In einem als Aminoschutzgruppe verwendbaren 2-Acyl-niederalk-1-enl-yl-rest ist Acyl z.B. der entsprechende Rest einer Niederalkancarbonsäure, einer gegebenenfalls, z.B. durch Niederalkyl, wie Methyl oder tert.-Butyl, Niederalkoxy, wie Methoxy, Halogen, wie Chlor, und/oder Nitro substituierten Benzoesäure, oder insbesondere eines Kohlensäurehalbesters, wie eines Kohlensäure-niederalkylhalbesters. Entsprechende Schutzgruppen sind in erster Linie 1-Niederalkanoyl-prop-1-en-2-yl, z.B. 1-Acetyl-prop-1-en-2-yl, oder 1-Niederalkoxycarbonyl-prop-1-en-2-yl, z.B. 1-Ethoxycarbonyl-prop-1-en-2-yl.

Eine Aminogruppe kann auch in protonierter Form geschützt werden; als entsprechende Anionen kommen in erster Linie diejenigen von starken anorganischen Säuren, wie von Halogenwasserstoffsäuren, z.B. das Chlor- oder Bromanion, oder von organischen Sulfonsäuren, wie p-Toluolsulfonsäure, in Frage.

Bevorzugte Aminoschutzgruppen sind Acylreste von Kohlensäurehalbestern, insbesondere tert.-Butyloxycarbonyl, gegebenenfalls, z.B. wie angegeben, substituiertes Benzyloxycarbonyl, z.B. 4-Nitro-benzyloxycarbonyl, oder Diphenylmethoxycarbonyl, oder 2-Halogen-niederalkoxycarbonyl, wie 2,2,2-Trichlorethoxycarbonyl, ferner Trityl oder Formyl.

Eine Mercaptogruppe, wie z.B. in Cystein, kann insbesondere durch S-Alkylierung mit gegebenenfalls substituierten Alkylresten, Thioacetalbildung, S-Acylierung oder durch das Erstellen asymmetrischer Disulfid-Gruppierungen geschützt werden.Bevorzugte Mercaptoschutzgruppen sind z.B. gegebenenfalls im Phenylrest, z.B. durch Methoxy oder Nitro, substituiertes Benzyl, wie 4-Methoxybenzyl, gegebenenfalls im Phenylteil, z.B. durch Methoxy, substituiertes Diphenylmethyl, wie 4,4'-Dimethoxydiphenyl-methyl, Triphenylmethyl, Trimethylsilyl, Benzyl-thiomethyl, Tetrahydropyranyl, Acylaminomethyl, Benzoyl, Benzyloxycarbonyl oder Aminocarbonyl, wie Ethylaminocarbonyl.

Primäre Carbonsäureamidgruppen (Carbamoylgruppen, $-CONH_2$) sind z.B. in Form von N-(9-Xanthenyl)-Derivaten oder in Form von N-(Mono-, Di- oder Triarylmethyl)-Derivaten geschützt, wobei Aryl insbesondere unsubstituiertes oder mit bis zu 5 gleichen oder verschiedenen Substituenten substituiertes Phenyl bedeutet. Solche Phenylsubstituenten sind vorzugsweise Niederalkyl, wie Methyl, oder Niederalkoxy, wie Methoxy. Als Beispiele für solche Arylmethyl-Schutzgruppen seien genannt: 4-Methoxy-benzyl, 2,4,6-Trimethoxy-benzyl, Diphenyl-methyl, Di-(4'-methoxy-phenyl)-methyl und Di-(4-methylphenyl)-methyl.

Der Schutz von Carbamoylgruppen ist fakultativ, d.h. bei geeigneten Reaktionsbedingungen, z.B. bei Verwendung geeigneter Kondensationsmittel, nicht zwingend erforderlich.

Guanidinogruppen sind z.B. in Form des Säureadditionssalzes, insbesondere als Hydrochlorid oder als Toluolsulfonat geschützt.

Ein den Rest $R^1$, $R^2$-C(=O)-, $R^4$ oder $R^6$ übertragendes Acylierungsmittel ist insbesondere die entsprechende Carbonsäure, d.h. $R^1$-OH, $R^2$-COOH, $R^4$-OH oder $R^6$-OH, oder vorzugsweise ein reaktionsfähiges Säurederivat derselben, wobei die Aktivierung der als Acylierungsmittel verwendeten Carbonsäure auch in situ in Gegenwart der Verbindung der Formel II erfolgen kann.

Die Erfindung betrifft insbesondere diejenigen Ausführungsformen des Verfahrens a), wobei ein Acylrest $R^1$, $R^4$ und/oder $R^6$ eingeführt wird.

Als Acylierungsmittel verwendbare aktivierte Carbonsäurederivate sind in erster Linie reaktionsfähige aktivierte Ester oder reaktionsfähige Anhydride, ferner reaktionsfähige cyclische Amide.

Aktivierte Ester von Säuren sind insbesondere am Verknüpfungskohlenstoffatom des veresternden Restes ungesättigte Ester, z.B. vom Vinylester-Typ, wie eigentliche Vinylester (die man z.B. durch Umesterung eines entsprechenden Esters mit Vinylacetat erhalten kann; Methode des aktivierten Vinylesters), Carbamoylvinylester (die man z.B. durch Behandeln der entsprechenden Säure mit einem Isoxazoliumreagens erhalten kann; 1,2-Oxazolium- oder Woodward-Methode), oder 1-Niederalkoxyvinylester (die man z.B. durch Behandeln der entsprechenden Säure mit einem Niederalkoxyacetylen erhalten kann; Ethoxyacetylen-Methode), oder Ester vom Amidinotyp, wie N,N'-disubstituierte Amidinoester (die man z.B. durch Behandeln der entsprechenden Säure mit einem geeigneten N,N'-disubstituierten Carbodiimid, z.B. N,N'-Dicyclohexylcarbodiimid, erhalten kann; Carbodiimid-Methode), oder N,N-disubstituierte Amidinoester (die man z.B. durch Behandeln der entsprechenden Säure mit einem N,N-disubstituierten Cyanamid erhalten kann; Cyanamid-Methode), geeignete Arylester, insbesondere durch Elektronen-anziehende Substituenten geeignet substituierte Phenylester (die man z.B. durch Behandeln der entsprechenden Säure mit einem geeignet substituierten Phenol, z.B. 4-Nitrophenol, 4-Methylsulfonyl-phenol, 2,4,5-Trichlorphenol,

2,3,4,5,6-Pentachlor-phenol oder 4-Phenyldiazophenol, in Gegenwart eines Kondensationsmittels, wie N,N'-Dicyclohexyl-carbodiimid, erhalten kann; Methode der aktivierten Arylester), Cyanmethylester (die man z.B. durch Behandeln der entsprechenden Säure mit Chloracetonitril in Gegenwart einer Base erhalten kann; Cyanmethylester-Methode), Thioester, insbesondere gegebenenfalls, z.B. duch Nitro, substituierte Phenyl-thioester (die man z.B. durch Behandeln der entsprechenden Säure mit gegebenenfalls, z. B. durch Nitro, substituierten Thiophenolen, u.a. mit Hilfe der Anhydrid-oder Carbodiimid-Methode, erhalten kann; Methode der aktivierten Thiolester), Amino-oder Amidoester (die man z.B. durch Behandeln der entsprechenden Säure mit einer N-Hydroxy-amino- bzw. N-Hydroxy-amido-Verbindung, z.B. N-Hydroxy-succinimid, N-Hydroxy-piperidin, N-Hydroxyphthalimid oder 1-Hydroxy-benztriazol, z.B. nach der Anhydrid- oder Carbodiimid-Methode, erhalten kann; Methode der aktivierten N-Hydroxyester) oder Silylester (die man z.B. durch Behandeln der entsprechenden Säure mit einem Silylierungsmittel, z.B. Hexamethyldisilazan, erhalten kann und die leicht mit Hydroxy-, nicht aber mit Aminogruppen reagieren).

Anhydride von Säuren können symmetrische oder vorzugsweise gemischte Anhydride dieser Säuren sein, so z.B. Anhydride mit anorganischen Säuren, wie Säurehalogenide, insbesondere Säurechloride (die man z.B. durch Behandeln der entsprechenden Säure mit Thionylchlorid, Phosphorpentachlorid oder Oxalylchlorid erhalten kann; Säurechloridmethode), Azide (die man z.B. aus einem entsprechenden Säureester über das entsprechende Hydrazid und dessen Behandlung mit salpetriger Säure erhalten kann; Azidmethode), Anhydride mit Kohlensäurehalbderivaten, wie mit entsprechenden Estern, z.B. Kohlensäureniederalkylhalbestern (die man z.B. durch Behandeln der entsprechenden Säure mit Halogen-, wie Chlorameisensäure-niederalkylestern oder mit einem 1-Niederalkoxycarbonyl-2-niederalkoxy-1,2-dihydro-chinolin, z.B. 1-Niederalkoxycarbonyl-2-ethoxy-1,2-dihydrochinolin, erhalten kann; Methode der gemischten O-Alkyl-kohlensäureanhydride), oder Anhydride mit dihalogenierter, insbesondere dichlorierter Phosphorsäure (die man z.B. durch Behandeln

der entsprechenden Säure mit Phosphoroxychlorid erhalten kann; Phosphoroxychloridmethode), oder Anhydride mit organischen Säuren, wie gemischte Anhydride mit organischen Carbonsäuren (die man z.B. durch Behandeln der entsprechenden Säure mit einem gegebenenfalls substituierten Niederalkan- oder Phenylalkancarbonsäurehalogenid, z.B. Phenylessigsäure-, Pivalinsäure- oder Trifluoressigsäurechlorid erhalten kann; Methode der gemischten Carbonsäureanhydride) oder mit organischen Sulfonsäuren (die man z.B. durch Behandeln eines Salzes, wie eines Alkalimetallsalzes, der entsprechenden Säure, mit einem geeigneten organischen Sulfonsäurehalogenid, wie Niederalkan- oder Aryl-, z.B. Methan- oder p-Toluolsulfonsäurechlorid, erhalten kann; Methode der gemischten Sulfonsäureanhydride), sowie symmetrische Anhydride (die man z.B. durch Kondensation der entsprechenden Säure in Gegenwart eines Carbodiimids oder von 1-Diethylaminopropin erhalten kann; Methode der symmetrischen Anhydride).

Geeignete cyclische Amide sind insbesondere Amide mit fünfgliedrigen Diazacyclen aromatischen Charakters, wie Amide mit Imidazolen, z.B. Imidazol (die man z.B. durch Behandeln der entsprechenden Säure mit N,N'-Carbonyldiimidazol erhalten kann; Imidazolid-Methode), oder Pyrazolen, z.B. 3,5-Dimethyl-pyrazol (die man z.B. über das Säurehydrazid durch Behandeln mit Acetylaceton erhalten kann; Pyrazolid-Methode).

Wie erwähnt können Derivate von Säuren, die als Acylierungsmittel verwendet werden, auch in situ gebildet werden. So kann man z.B. N,N'-disubstituierte Amidinoester in situ bilden, indem man das Gemisch des Ausgangsmaterials der Formel II und der als Acylierungsmittel verwendeten Säure in Gegenwart eines geeigneten N,N-disubstituierten Carbodiimids, z.B. N,N'-Dicyclohexylcarbodiimid, zur Reaktion bringt. Ferner kann man Amino- oder Amidoester der als Acylierungsmittel verwendeten Säuren in Gegenwart des zu acylierenden Ausgangsmaterials der Formel II bilden, indem man das Gemisch der entsprechenden Säure- und Amino-Ausgangsstoffe in Gegenwart eines N,N'-disubstituierten Carbodiimids, z.B.

N,N'-Dicyclohexyl-carbodimid, und eines N-Hydroxy-amins oder N-Hydroxy-amids, z.B. N-Hydroxysuccinimid, gegebenenfalls in Anwesenheit einer geeigneten Base, z.B. 4-Dimethylamino-pyridin, umsetzt.

Die Reaktion kann in an sich bekannter Weise durchgeführt werden, wobei die Reaktionsbedingungen in erster Linie davon abhängen, ob und wie die Carboxylgruppe des Acylierungsmittels aktiviert ist, üblicherweise in Gegenwart eines geeigneten Lösungs- oder Verdünnungsmittels oder eines Gemisches von solchen, und, falls notwendig, in Gegenwart eines Kondensationsmittels, das z.B., wenn die an der Reaktion teilnehmende Carboxylgruppe als Anhydrid vorliegt, auch ein Säure-bindendes Mittel sein kann, unter Kühlen oder Erwärmen, z.B. in einem Temperaturbereich von etwa -30°C bis etwa +150°C, insbesondere von etwa 0°C bis +100°C, vorzugsweise von Raumtemperatur (ca. +20°C) bis +70°C, in einem geschlossenen Reaktionsgefäss und/oder in der Atmosphäre eines Inertgases, z.B. Stickstoff. Uebliche Kondensationsmittel sind z.B. Carbodiimide, beispielsweise N,N'-Diethyl-, N,N'-Dipropyl-, N,N'-Dicyclohexyl- oder N-Ethyl-N'-(3-dimethylaminopropyl)-carbodiimid, geeignete Carbonylverbindungen, beispielsweise Carbonyldiimidazol, oder 1,2-Oxazoliumverbindungen, z.B. 2-Ethyl-5-phenyl-1,2-oxazolium-3'-sulfonat und 2-tert.-Butyl-5-methyl-isoxazolium-perchlorat, oder eine geeignete Acylaminoverbindung, z.B. 2-Ethoxy-1-ethoxycarbonyl-1,2-dihydrochinolin. Uebliche säurebindende Kondensationsmittel sind z.B. Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin.

Die Abspaltung der Schutzgruppen, z.B. der Carboxyl-, Amino-, Hydroxy- oder Mercaptoschutzgruppen, die nicht Bestandteil des gewünschten Endprodukts der Formel I sind, erfolgt in an sich bekannter Weise, z.B. mittels Solvolyse, insbesondere Hydrolyse, Alkoholyse oder Acidolyse, oder mittels Reduktion, insbesondere

Hydrogenolyse oder chemische Reduktion, gegebenenfalls stufenweise oder gleichzeitig, wobei auch enzymatische Methoden verwendet werden können.

So kann man tert.-Niederalkoxycarbonyl oder in 2-Stellung durch eine organische Silylgruppe oder in 1-Stellung durch Niederalkoxy oder Niederalkylthio substituiertes Niederalkoxycarbonyl oder gegebenenfalls substituiertes Diphenylmethoxycarbonyl z.B. durch Behandeln mit einer geeigneten Säure, wie Ameisensäure oder Trifluoressigsäure, gegebenenfalls unter Zugabe einer nucleophilen Verbindung, wie Phenol oder Anisol, in freies Carboxyl überführen. Gegebenenfalls substituiertes Benzyloxycarbonyl kann z.B. mittels Hydrogenolyse, d.h. durch Behandeln mit Wasserstoff in Gegenwart eines metallischen Hydrierkatalysators, wie eines Palladiumkatalysators, freigesetzt werden. Ferner kann man geeignet substituiertes Benzyloxycarbonyl, wie 4-Nitrobenzyloxycarbonyl, auch mittels chemischer Reduktion, z.B. durch Behandeln mit einem Alkalimetall-, z.B. Natriumdithionit, oder mit einem reduzierenden Metall, z.B. Zink, oder Metallsalz, wie einem Chrom-II-salz, z.B. Chrom-II-chlorid, üblicherweise in Gegenwart eines Wasserstoff-abgebenden Mittels, das zusammen mit dem Metall nascierenden Wasserstoff zu erzeugen vermag, wie einer Säure, in erster Linie einer geeigneten Carbonsäure, wie einer gegebenenfalls, z.B. durch Hydroxy, substituierten Niederalkancarbonsäure, z.B. Essigsäure, Ameisensäure, Glycolsäure, Diphenylglycolsäure, Milchsäure, Mandelsäure, 4-Chlor-mandelsäure oder Weinsäure, oder eines Alkohols oder Thiols, wobei man vorzugsweise Wasser zugibt, in freies Carboxyl überführen. Durch Behandeln mit einem reduzierenden Metall oder Metallsalz, wie oben beschrieben, kann man auch 2-Halogen-niederalkoxycarbonyl (gegebenenfalls nach Umwandlung einer 2-Brom-niederalkoxycarbonylgruppe in eine entsprechende 2-Iodniederalkoxycarbonylgruppe) oder Aroylmethoxycarbonyl in freies Carboxyl umwandeln, wobei Aroylmethoxycarbonyl ebenfalls durch Behandeln mit einem nucleophilen, vorzugsweise salzbildenden Reagens, wie Natriumthiophenolat oder Natriumiodid, gespalten werden kann. Substituiertes 2-Silylethoxycarbonyl kann auch durch Behandeln mit einem, das Fluoridanion liefernden Salz der Fluorwaserstoffsäure, wie einem Alkalimetallfluorid, z.B. Natrium-oder Kaliumfluorid, in Anwesenheit eines macrocyclischen Polyethers ("Kronenether"), oder mit einem Fluorid einer organischen quaternären Base, wie Tetra-niederalkylammoniumfluorid oder Triniederalkyl-aryl-ammoniumfluorid, z.B. Tetraethylammoniumfluorid oder Tetrabutylammoniumfluorid, in Gegenwart eines aprotischen polaren Lösungsmittels, wie Dimethylsulfoxid oder N,N-Dimethylacetamid, in freies Carboxyl übergeführt werden.

Verestertes Carboxyl kann auch enzymatisch gespalten werden, z.B. verestertes Lysin, z.B. Lysinmethylester, mittels Trypsin.

Eine geschützte Aminogruppe setzt man in an sich bekannter und je nach Art der Schutzgruppen in verschiedenartiger Weise, vorzugsweise mittels Solvolyse oder Reduktion, frei. 2-Halogen-niederalkoxycarbonylamino (gegebenenfals nach Umwandlung einer 2-Brom-niederalkoxycarbonylaminogruppe in eine 2-Iod-niederalkoxycarbonylaminogruppe), Aroylmethoxycarbonylamino oder 4-Nitrobenzyloxycarbonylamino kann z.B. durch Behandeln mit einem geeigneten chemischen Reduktionsmittel, wie Zink in Gegenwart einer geeigneten Carbonsäure, wie wässriger Essigsäure, gespalten werden. Aroylmethoxycarbonylamino kann auch durch Behandeln mit einem nucleophilen, vorzugsweise salzbildenden Reagens, wie Natriumthiophenolat, und 4-Nitro-benzyloxycarbonylamino auch durch Behandeln mit einem Alkalimetall-, z.B. Natriumdithionit, gespalten werden. Gegebenenfalls substituiertes Diphenylmethoxycarbonylamino, tert.-Niederalkoxycarbonylamino oder 2-trisubstituiertes Silylethoxycarbonylamino kann durch Behandeln mit einer geeigneten Säure, z.B. Ameisen- oder Trifluoressigsäure, gegebenenfalls substituiertes Benzyloxycarbonylamino z.B. mittels Hydrogenolyse, d.h. durch Behandeln mit Wasserstoff in Gegenwart eines geeigneten Hydrierkatalysators, wie eines Palladiumkatalysators, gegebenenfalls substituiertes Triarylmethylamino oder Formylamino z.B. durch Behandeln mit einer Säure, wie Mineralsäure, z.B. Chlorwasserstoffsäure, oder einer organischen Säure, z.B. Ameisen-, Essig- oder Trifluoressigsäure, gegebenenfalls in Gegenwart von Wasser, gespalten werden, und eine mit einer

organischen Silylgruppe geschützte Aminogruppe kann z.B. mittels Hydrolyse oder Alkoholyse freigesetzt werden. Eine durch 2-Halogenacetyl, z.B. 2-Chloracetyl, geschützte Aminogruppe kann durch Behandeln mit Thioharnstoff in Gegenwart einer Base, oder mit einem Thiolatsalz, wie einem Alkalimetallthiolat, des Thioharnstoffs und anschliessende Solvolyse, wie Alkoholyse oder Hydrolyse, des entstandenen Kondensationsprodukts freigesetzt werden. Eine durch 2-substituiertes Silylethoxycarbonyl geschützte Aminogruppe kann auch durch Behandeln mit einem Fluoridanionen liefernden Salz der Fluorwaserstoffsäure, wie oben im Zusammenhang mit der Freisetzung einer entsprechend geschützten Carboxylgruppe angegeben, in die freie Aminogruppe übergeführt werden.

In Form einer Azidogruppe geschütztes Amino wird z.B. durch Reduktion in freies Amino übergeführt, beispielsweise durch katalytische Hydrierung mit Wasserstoff in Gegenwart eines Hydrierkatalysators, wie Platinoxid, Palladium oder Raney-Nickel, oder auch durch Behandeln mit Zink in Gegenwart einer Säure, wie Essigsäure. Die katalytische Hydrierung wird vorzugsweise in einem inerten Lösungsmittel, wie einem halogenierten Kohlenwasserstoff, z.B. Methylenchlorid, oder auch in Wasser oder einem Gemisch von Wasser und einem organischen Lösungsmittel, wie einem Alkohol oder Dioxan, bei etwa 20°C bis 25°C, oder auch unter Kühlen oder Erwärmen, durchgeführt.

Eine durch eine geeignete Acylgruppe, eine organische Silylgruppe oder durch gegebenenfalls substituiertes 1-Phenylniederalkyl geschützte Hydroxy- oder Mercaptogruppe wird analog einer entsprechend geschützten Aminogruppe freigesetzt. Durch gegebenenfalls substituiertes 1-Phenylniederalkyl, z.B. Benzyl, geschütztes Hydroxy wird vorzugsweise durch katalytische Hydrierung, z.B. in Gegenwart eine Palladium-auf-Kohle-Katalysators freigesetzt. Eine durch 2,2-Dichloracetyl geschützte Hydroxy- bzw. Mercaptogruppe wird z.B. durch basische Hydrolyse, eine durch tert.-Niederalkyl oder durch einen 2-oxa- oder 2-thia-aliphatischen oder -cycloaliphatischen Kohlenwasserstoffrest veretherte Hydroxy- bzw. Mercaptogruppe durch

Acidolyse, z.B. durch Behandeln mit einer Mineralsäure oder einer starken Carbonsäure, z.B. Trifluoressigsäure, freigesetzt. Zwei Hydroxygruppen, die zusammen mittels einer vorzugsweise substituierten Methylengruppe, wie durch Niederalkyliden, z.B. Isopropyliden, Cycloalkyliden, z.B. Cyclohexyliden, oder Benzyliden, geschützt sind, können durch saure Solvolyse, besonders in Gegenwart einer Mineralsäure oder einer starken organischen Säure, freigesetzt werden. Mit einem organischen Silylrest, z.B. Trimethylsilyl, verethertes Hydroxy kann auch mit einem Fluoridanionen liefernden Salz der Fluorwasserstoffsäure, z.B. Tetrabutylammoniumfluorid, freigesetzt werden.

Bevorzugt werden beim Vorhandensein von mehreren geschützten funktionellen Gruppen die Schutzgruppen so gewählt, dass gleichzeitig mehr als eine solche Gruppe abgespalten werden kann, beispielsweise acidolytisch, wie durch Behandeln mit Trifluoressigsäure oder Ameisensäure, oder reduktiv, wie durch Behandeln mit Zink und Essigsäure, oder mit Wasserstoff und einem Hydrierkatalysator, wie einem Palladium-Kohle-Katalysator. Wenn die gewünschten Endstoffe Schutzgruppen enthalten, z.B. wenn $R^{12}$ Arylmethoxy, wie Benzyloxy, bedeutet, werden diejenigen Schutzgruppen, die nach erfolgter Reaktion abgespalten werden sollen, so gewählt, dass sie regioselektiv wieder abgespalten werden können, z.B. kann mit einem organischen Silylrest verethertes Hydroxy im Rest $R^8$ oder $R^{11}$ mit Fluorid freigesetzt werden, wobei eine Arylmethoxy-Schutzgruppe im Rest $R^9$ oder $R^{12}$ erhalten bleibt.

Die Ausgangsstoffe der Formel II, worin $R^{2a}$ Wasserstoff bedeutet, werden z.B. aus entsprechenden Verbindungen hergestellt, worin $R^{2a}$ eine Aminoschutzgruppe, z.B. Benzyloxycarbonyl, bedeutet.

Verfahren b:
Ein reaktionsfähiges Derivat einer Verbindung der Formel III ist z.B. eine Metalloxyverbindung, wie sie z.B. durch Umsetzung einer Verbindung der Formel III mit einer geeigneten Base, wie einem Alkalimetallhydrid oder -amid, erhalten werden kann.

Reaktionsfähiges verestertes Hydroxy X ist z.B. mit einer starken anorganischen oder organischen Säure verestertes Hydroxy, wie mit einer Mineralsäure, z.B. Halogenwasserstoffsäure, wie Chlorwasserstoff-, Bromwasserstoff- oder Iodwasserstoffsäure, ferner Schwefelsäure, oder Halogenschwefelsäure, z.B. Fluorschwefelsäure, oder mit einer starken organischen Sulfonsäure, wie einer gegebenenfalls, z.B. durch Halogen, wie Fluor, substituierten Niederalkansulfonsäure oder einer aromatischen Sulfonsäure, z.B. einer gegebenenfalls durch Niederalkyl, wie Methyl, Halogen, wie Brom, und/oder Nitro substituierten Benzolsulfonsäure, z.B. einer Methansulfon-, Trifluormethansulfon- oder p-Toluolsulfonsäure, verestertes Hydroxy, bevorzugterweise ein Chlorid, Bromid oder Iodid.

Freie funktionelle Gruppen in einer Verbindung der Formel IV, die vorteilhafterweise durch leicht abspaltbare Schutzgruppen geschützt sind, sind insbesondere Hydroxy-, Mercapto- oder Carboxygruppen.

Die Reaktion wird vorzugsweise in einem inerten organischen Lösungsmittel bei einer Temperatur zwischen -30°C und +150°C, insbesondere 0°C und +100°C, z.B. +20°C und +70°C, erforderlichenfalls in Gegenwart eines säurebindenden Mittels durchgeführt.

Die Abspaltung von Schutzgruppen, die nicht Bestandteil des gewünschten Endstoffs der Formel I sind, erfolgt wie bei Verfahren a) beschrieben.

Verfahren c:
Ein reaktionsfähiges Carbonsäurederivat einer Verbindung der Formel V, ist in erster Linie ein reaktionsfähiger Ester, ein reaktionsfähiges Anhydrid oder ein reaktionsfähiges cyclisches Amid, worin die Carboxylgruppe in analoger Weise wie bei den bei Verfahren a) beschriebenen reaktionsfähigen Acylierungsmitteln aktiviert ist, und wobei die Aktivierung auch in situ erfolgen kann.

In Verbindungen der Formeln V oder VI gegebenenfalls vorhandene funktionelle Gruppen, die vorzugsweise durch leicht abspaltbare Schutzgruppen geschützt werden, sind insbesondere Amino und Guanidino im Rest $R^{11}$, Carboxy als Rest $R^{10}$ und Hydroxy als Rest $R^9$ oder $R^{12}$, daneben aber auch Hydroxy oder Mercapto im Rest $R^8$ oder Hydroxy im Rest $R^{11}$.

In einem reaktionsfähigen Derivat einer Verbindung der Formel VI ist die Aminogruppe z.B. durch Umsetzung mit einem Phosphit, z.B. Diethylchlorphosphit, 1,1-Phenylen-chlorphosphit, Ethyl-dichlorphosphit, Ethylen-chlor-phosphit oder Tetraethylpyrophosphit, oder durch Bindung an Halogencarbonyl, z.B. Chlorcarbonyl, oder in Form einer Isocyanatgruppe aktiviert. Vorzugsweise wird die Reaktion so durchgeführt, dass man ein reaktionsfähiges Carbonsäurederivat einer Verbindung der Formel V mit einer Verbindung der Formel VI umsetzt, worin die an der Reaktion teilnehmende Amino- oder Hydroxygruppe in freier Form vorliegt.

Die Reaktion und die nachfolgende Abspaltung der Schutzgruppen, die nicht Bestandteil des gewünschten Endstoffes sind, wird analog wie bei Verfahren a) beschrieben durchgeführt.

Die Veresterung einer Verbindung der Formel V, worin q, r und t für 1 stehen, kann auch mit Hilfe der bei Verfahren d) beschriebenen Veresterungsmittel durchgeführt werden.

Verfahren d:
In einer Verbindung der Formel VII gegebenenfalls vorhandene freie funktionelle Gruppen, die durch leicht abspaltbare Schutzgruppen geschützt sind, sind insbesondere Carboxylgruppen, die nicht verestert werden sollen, und ferner Hydroxy-, Mercapto-, Guanidino und Aminogruppen.
Geeignete Schutzgruppen und ihre Abspaltung sind bei Verfahren a) beschrieben.

Ein reaktionsfähiges Carbonsäurederivat einer Verbindung der Formel VII ist insbesondere ein reaktionsfähiger Ester, ein reaktionsfähiges Anhydrid oder ein reaktionsfähiges cyclisches Amid, worin die Carboxylgruppe in analoger Weise wie bei den bei Verfahren a) beschriebenen reaktionsfähigen Acylierungsmitteln aktiviert ist, und wobei die Aktivierung auch in situ erfolgen kann. Vorzugsweise wird die Reaktion so durchgeführt, dass man ein reaktionsfähiges Carbonsäurederivat einer Verbindung der Formel VII zur Veresterung von Carboxy $R^{13}$ mit einer Verbindung $H-R^{9a}$, worin $R^{9a}$ die obengenannten Bedeutungen von $R^9$ mit Ausnahme von Hydroxy und Amino hat, oder, zur Veresterung von Carboxy $R^{10a}$, mit einem Niederalkanol oder Arylniederalkanol umsetzt.

Alternativ kann man eine Verbindung der Formel VII mit freier Carboxylgruppe durch Umsetzung mit einem reaktionsfähigen Derivat des als Veresterungskomponente gewünschten Alkohols verestern.

Geeignete Mittel zur Veresterung sind beispielsweise entsprechende Diazoverbindungen, wie gegebenenfalls substituierte Diazoniederalkane, z.B. Diazomethan, Diazoethan, Diazo-n-butan oder Diphenyldiazomethan. Diese Reagenzien werden in Gegenwart eines geeigneten inerten Lösungsmittels, wie eines aliphatischen, cycloaliphatischen oder aromatischen Kohlenwasserstoffs, wie Hexan, Cyclohexan, Benzol oder Toluol, eines halogenierten aliphatischen Kohlenwasserstoffs, z.B. Methylenchlorid, oder eines Ethers, wie eines Diniederalkylethers, z.B. Diethylether, oder eines cyclischen Ethers, z.B. Tetrahydrofuran oder Dioxan, oder eines Lösungsmittelgemisches, und, je nach Diazoreagens, unter Kühlen, bei Raumtemperatur oder unter leichtem Erwärmen, ferner, wenn notwendig, in einem geschlossenen Gefäss und/oder unter einer Inertgas-, z.B. Stickstoffatmosphäre, zur Anwendung gebracht.

Weitere geeignete Mittel zur Veresterung sind Ester entsprechender Alkohole, in erster Linie solche mit starken anorganischen oder organischen Säuren, wie Mineralsäuren, z.B. Halogenwasserstoffsäuren, wie Chlorwasserstoff-, Bromwasserstoff- oder Iodwasserstoffsäure, ferner Schwefelsäure, oder Halogen-schwefelsäure, z.B. Fluorschwefelsäure, oder starken organischen Sulfonsäuren, wie gegebenenfalls, z.B. durch Halogen, wie Fluor, substituierten Niederalkansulfonsäuren, oder aromatischen Sulfonsäuren, wie z.B. gegebenenfalls durch Niederalkyl, wie Methyl, Halogen, wie Brom, und/oder Nitro substituierten Benzolsulfonsäuren, z.B. Methansulfon-, Trifluormethansulfon- oder p-Toluolsulfonsäure. Solche Ester sind u.a. Niederalkylhalogenide, Diniederalkylsulfate, wie Dimethylsulfat, ferner Fluorsulfonsäureester, wie -niederalkylester, z.B. Fluorsulfonsäuremethylester, oder gegebenenfalls Halogensubstituierte Methansulfonsäure-niederalkylester, z.B. Trifluormethansulfonsäuremethylester. Sie werden üblicherweise in Gegenwart eines inerten Lösungsmittels, wie eines gegebenenfalls halogenierten, wie chlorierten, aliphatischen, cycloaliphatischen oder aromatischen Kohlenwasserstoffs, z.B. Methylenchlorid, eines Ethers, wie Dioxan oder Tetrahydrofuran, oder eines Gemisches davon verwendet. Dabei wendet man vorzugsweise geeignete Kondensationsmittel, wie Alkalimetallcarbonate oder -hydrogencarbonate, z.B. Natrium- oder Kaliumcarbonat oder -hydrogencarbonat (üblicherweise zusammen mit einem Sulfat), oder organische Basen, wie üblicherweise sterisch gehinderte Triniederalkylamine, z.B. N,N-Diisopropyl-N-ethyl-amin (vorzugsweise zusammen mit Halogensulfonsäure-niederalkylestern oder gegebenenfalls halogensubstituierten Methansulfonsäureniederalkylestern) an, wobei unter Kühlen, bei Raumtemperatur oder unter Erwärmen, z.B. bei Temperaturen von etwa -20°C bis etwa 50°C, und, wenn notwendig, in einem geschlossenen Gefäss und/oder in einer Inertgas-, z.B. Stickstoffatmosphäre, gearbeitet wird.

Weitere Mittel zur Veresterung sind entsprechende trisubstituierte Oxoniumsalze (sogenannte Meerweinsalze), oder disubstituierte Carbenium- oder Haloniumsalze, worin die Substituenten die verethernden Reste sind, beispielsweise Triniederalkyloxoniumsalze, sowie Diniederalkoxycarbenium- oder Diniederalkylhaloniumsalze, insbesondere die entsprechenden Salze mit komplexen, fluorhaltigen Säuren, wie die entsprechenden Tetrafluorborate, Hexafluorphosphate, Hexafluorantimonate, oder Hexachlorantimonate. Solche

Reagentien sind z.B. Trimethyloxonium- oder Triethyloxoniumhexafluorantimonat, -hexachlorantimonat, -hexafluorphosphat oder -tetrafluorborat, Dimethoxycarbeniumhexafluorphosphat oder Dimethylbromoniumhexafluorantimonat. Man verwendet diese Mittel vorzugsweise in einem inerten Lösungsmittel, wie einem Ether oder einem halogenierten Kohlenwasserstoff, z.B. Diethylether, Tetrahydrofuran oder Methylenchlorid, oder in einem Gemisch davon, wenn notwendig, in Gegenwart einer Base, wie einer organischen Base, z.B. eines, vorzugsweise sterisch gehinderten, Triniederalkylamins, z.B. N,N-Diisopropyl-N-ethyl-amin, und unter Kühlen, bei Raumtemperatur oder unter leichtem Erwärmen, z.B. bei etwa -20°C bis etwa 50°C, wenn notwendig, in einem geschlossenen Gefäss und/oder in einer Inertgas-, z.B. Stickstoffatmosphäre.

Eine bevorzugte Ausführungsform des Verfahrens d) ist die Umsetzung eines Cäsiumsalzes einer Verbindung der Formel VII mit dem als Veresterungskomponente gewünschten Alkohol, worin die Hydroxylgruppe in reaktionsfähiger veresterter Form vorliegt.

Verfahren e:
Eine Verbindung der Formel I, worin mindestens einer der Reste $R^8$, $R^9$, $R^{10}$, $R^{11}$ und $R^{12}$ in einer geschützten Form vorliegt, die nicht der Definition des gewünschten Endstoffes entspricht, ist insbesondere eine Verbindung der Formel I, worin eine Hydroxy- oder Mercaptogruppe im Rest $R^8$, eine Amino- oder Hydroxygruppe im Rest $R^{11}$, freies Hydroxy $R^9$ oder $R^{12}$, und/oder Carboxy $R^{10}$ durch eine leicht abspaltbare Schutzgruppe geschützt ist, die in dem gewünschten Endstoff nicht enthalten ist.

Leicht abspaltbare Schutzgruppen sind insbesondere die bei Verfahren a) genannten. Die Abspaltung der Schutzgruppen wird analog, wie bei Verfahren a) beschrieben, durchgeführt.

Verfahren f:
In einer Verbindung der Formel VIII gegebenenfalls vorhandene freie funktionelle Gruppen, die durch leicht abspaltbare Schutzgruppen geschützt sind, sind insbesondere Carboxylgruppen, die nicht amidiert werden sollen, und ferner Hydroxy-, Mercapto-, Guanidino und Aminogruppen.

Geeignete Schutzgruppen und ihre Abspaltung sind bei Verfahren a) beschrieben.

Ein reaktionsfähiges Carbonsäurederivat einer Verbindung der Formel VIII ist insbesondere ein reaktionsfähiger Ester, ein reaktionsfähiges Anhydrid oder ein reaktionsfähiges cyclisches Amid, worin die Carboxylgruppe in analoger Weise wie bei den bei Verfahren a) beschriebenen reaktionsfähigen Acylierungsmitteln aktiviert ist, und wobei die Aktivierung auch in situ erfolgen kann. Vorzugsweise wird die Reaktion so durchgeführt, dass man ein reaktionsfähiges Carbonsäurederivat einer Verbindung der Formel VIII mit Ammoniak umsetzt. Alternativ kann man auch eine Verbindung der Formel VIII mit freier Carboxylgruppe mit einem reaktionsfähigen Ammoniakderivat umsetzen, wobei die Aktivierung der Aminogruppe z.B. analog wie bei Verfahren c) beschrieben erfolgt.

Die Reaktion und die nachfolgende Abspaltung der Schutzgruppen, die nicht Bestandteil des gewünschten Endstoffes sind, wird analog wie bei Verfahren a) beschrieben durchgeführt.

Zusatzoperationen: Salze von Verbindungen der Formel I mit salzbildenden Gruppen können in an sich bekannter Weise hergestellt werden. So kann man Salze von Verbindungen der Formel I mit sauren Gruppen durch Umsetzung mit einer geeigneten Base, z.B. durch Behandeln mit geeigneten Metallverbindungen, wie Alkalimetallsalzen von geeigneten organischen Carbonsäuren, z.B. dem Natriumsalz der α-Ethyl-capronsäure, oder mit geeigneten anorganischen Alkali- oder Erdalkalimetallsalzen, insbesondere solchen, die sich von einer schwachen und vorzugsweise flüchtigen Säure ableiten, z.B. Natriumhydrogencarbonat, oder mit Ammoniak oder einem geeigneten organischen Amin bilden, wobei man vorzugsweise stöchiometrische Mengen oder nur einen kleinen Ueberschuss des salzbildenden Mittels verwendet. Säureadditionssalze von Verbindungen der Formel I erhält man in üblicher Weise, z.B. durch Behandeln mit einer Säure oder einem geeigneten Anionenaustauschreagens. Innere Salze von Verbindungen der Formel I, welche z.B. eine freie Carboxylgruppe und eine freie Aminogruppe enthalten, können z.B. durch Neutralisieren von Salzen, wie Säureadditionssalzen, auf den isoelektrischen Punkt, z.B. mit schwachen Basen, oder durch Behandeln mit flüssigen Ionenaustauschern gebildet werden.

Salze können in üblicher Weise in die freien Verbindungen übergeführt werden, Metall- und Ammoniumsalze z.B. durch Behandeln mit geeigneten Säuren, und Säureadditionssalze z.B. durch Behandeln mit einem geeigneten basischen Mittel.

Gemische von Isomeren können in an sich bekannter Weise, z.B. durch fraktionierte Kristallisation, Chromatographie etc. in die einzelnen Isomeren aufgetrennt werden.

Die oben beschriebenen Verfahren, inklusive die Verfahren zur Abspaltung von Schutzgruppen und die zusätzlichen Verfahrensmassnahmen, werden, wenn nicht anders angegeben, in an sich bekannter Weise, z.B. in An- oder Abwesenheit von vorzugsweise inerten Lösungs-und Verdünnungsmitteln, wenn notwendig, in Anwesenheit von Kondensationsmitteln oder Katalysatoren, bei erniedrigter oder erhöhter Temperatur, z.B. in einem Temperaturbereich von etwa -20°C bis etwa +150°C, insbesondere von etwa 0°C bis etwa +70°C, vorzugsweise von etwa +10°C bis etwa +40°C, hauptsächlich bei Raumtemperatur, in einem geschlossenen Gefäss und/oder in einer Inertgas-, z.B. Stickstoffatmosphäre, durchgeführt.

Dabei sind unter Berücksichtigung aller im Molekül befindlichen Substituenten, wenn erforderlich, z.B. bei Anwesenheit leicht hydrolysierbarer Reste, besonders schonende Reaktionsbedingungen,

wie kurze Reaktionszeiten, Verwendung von milden sauren oder basischen Mitteln in niedriger Konzentration, stöchiometrische Mengenverhältnisse, Wahl geeigneter Katalysatoren, Lösungsmittel, Temperatur- und/oder Druckbedingungen, anzuwenden.

Die Erfindung betrifft auch diejenigen Ausführungsformen des Verfahrens, bei denen man von einer auf irgendeiner Stufe des Verfahrens als Zwischenprodukt erhältlichen Verbindung ausgeht und die fehlenden Verfahrensschritte durchführt oder das Verfahren auf irgendeiner Stufe abbricht oder einen Ausgangsstoff unter den Reaktionsbedingungen bildet oder in Form eines reaktionsfähigen Derivats oder Salzes verwendet. Dabei geht man vorzugsweise von solchen Ausgangsstoffen aus, die verfahrensgemäss zu den oben als besonders wertvoll beschriebenen Verbindungen führen.

Neue Ausgangsstoffe und/oder Zwischenprodukte sowie Verfahren zu ihrer Herstellung sind ebenfalls Gegenstand der vorliegenden Erfindung. Vorzugsweise werden solche Ausgangsstoffe verwendet und die Reaktionsbedingungen so gewählt, dass man zu den in dieser Anmeldung als besonders bevorzugt aufgeführten Verbindungen gelangt.

Die Erfindung betrifft auch pharmazeutische Präparate, die eine wirksame Menge, insbesondere eine zur Prophylaxe oder Therapie von Virusinfektionen oder eine zur Therapie von Tumorerkrankungen wirksame Menge, der Aktivsubstanz zusammen mit pharmazeutisch verwendbaren Trägerstoffen enthalten, die sich zur topischen, z.B. intranasalen, enteralen, z.B. oralen oder rektalen, oder parenteralen Verabreichung eignen, und anorganisch oder organisch, fest oder flüssig sein können. So verwendet man Tabletten oder Gelatinekapseln, welche den Wirkstoff zusammen mit Verdünnungsmitteln, z.B. Lactose, Dextrose, Sukrose, Mannitol, Sorbitol, Cellulose und/oder Glycerin, und/oder Schmiermitteln, z.B. Kieselerde, Talk, Stearinsäure oder Salze davon, wie Magnesium- oder Calciumstearat, und/oder Polyethylenglykol, enthalten. Tabletten können ebenfalls Bindemittel, z.B. Magnesiumaluminiumsilikat, Stärken, wie Mais-, Weizen- oder Reisstärke, Gelatine, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat, und/oder Brausemischungen, oder Adsorptionsmittel, Farbstoffe, Geschmackstoffe und Süssmittel enthalten. Ferner kann man die pharmakologisch wirksamen Verbindungen der vorliegenden Erfindung in Form von parenteral verabreichbaren Präparaten oder von Infusionslösungen verwenden. Solche Lösungen sind vorzugsweise isotonische wässrige Lösungen oder Suspensionen, wobei diese z.B. bei lyophilisierten Präparaten, welche die Wirksubstanz allein oder zusammen mit einem Trägermaterial, z.B. Mannit, enthalten, vor Gebrauch hergestellt werden können. Die pharmazeutischen Präparate können sterilisiert sein und/oder Hilfsstoffe, z.B. Konservier-, Stabilisier-, Netz-und/oder Emulgiermittel, Löslichkeitsvermittler, Salze zur Regulierung des osmotischen Drucks und/oder Puffer enthalten. Die vorliegenden pharmazeutischen Präparate, die, wenn erwünscht, weitere pharmakologisch wirksame Stoffe, wie Antibiotika, enthalten können, werden in an sich bekannter Weise, z.B. mittels konventioneller Misch-, Granulier-, Dragier-, Lösungs- oder Lyophilisierungsverfahren, hergestellt und enthalten von etwa 0,001 % bis 20 %, insbesondere von etwa 0,01 % bis etwa 10 %, in erster Linie zwischen 0,1 % und 5 %, des bzw. der Aktivstoffe, wobei eine Wirkstoffkonzentration unterhalb von 1% insbesondere für topisch zu applizierende Präparate geeignet ist.

Als topisch zu applizierende Darreichungsformen sind die folgenden bevorzugt: Crèmes, Salben oder Pasten mit einem Wirkstoffgehalt von 0,001 % bis 1 %, insbesondere von 0,01 % bis 0,1 %, z.B. 0,05 %, z.B. Salben zur intranasalen Applikation oder Lippenstifte, oder wässrige Lösungen mit einem Wirkstoffgehalt von 0,001 % bis 1 %, insbesondere 0,05 % bis 0,5 %, z.B. 0,1 %, vorzugsweise isotonische, sterile und physiologisch verträgliche Lösungen, z.B. Augentropfen, vorzugsweise in Mikrobehältern zur einmaligen Verwendung, oder Sprays zur Anwendung im Mund- und Rachenraum.

Besonders geeignet sind die in den Beispielen beschriebenen pharmazeutischen Präparate.

Crèmes sind Oel-in-Wasser-Emulsionen, die mehr als 50 % Wasser aufweisen. Als ölige Grundlage verwendet man in erster Linie Fettalkohole, z.B. Lauryl-, Cetyl- oder Stearylalkohole, Fettsäuren, z.B. Palmitin- oder Stearinsäure, flüssige bis feste Wachse, z.B. Isopropylmyristat, Wollwachs oder Bienenwachs, und/oder Kohlenwasserstoffe, z.B. Vaseline (Petrolatum) oder Paraffinöl. Als Emulgatoren kommen oberflächenaktive Substanzen mit vorwiegend hydrophilen Eigenschaften in Frage, wie entsprechende nichtionische Emulgatoren, z.B. Fettsäureester von Polyalkoholen oder Ethylenoxidaddukte davon, wie Polyglycerinfettsäureester oder Polyoxyethylensorbitan-fettsäureester (Tweens), ferner Polyoxyethylen-fettalkoholether oder -fettsäureester, oder entsprechende ionische Emulgatoren, wie Alkalimetallsalze von Fettalkoholsulfaten, z.B. Natriumlaurylsulfat, Natriumcetylsulfat oder Natriumstearylsulfat, die man üblicherweise in Gegenwart von Fettalkoholen, z.B. Cetylalkohol oder Stearylalkohol, verwendet. Zusätze zur Wasserphase sind u.a. Mittel, welche die Austrocknung der Crémes vermindern, z.B. Polyalkohol, wie Glycerin, Sorbit, Propylenglykol und/oder Polyethylenglykole, ferner Konservierungsmittel, Riechstoffe, etc.

Salben sind Wasser-in-Oel-Emulsionen, die bis zu 70 %, vorzugsweise jedoch von etwa 20 % bis etwa 50 % Wasser oder wässrige Phase enthalten. Als Fettphase kommen in erster Linie Kohlenwasserstoffe, z.B. Vaseline, Paraffinöl und/oder Hartparaffine in Frage, die zur Verbesserung des Wasserbindungsvermögens vorzugsweise geeignete Hydroxyverbindungen, wie Fettalkohole oder Ester davon, z.B. Cetylalkohol oder Wollwachsalkohole, bzw. Wollwachs, enthalten. Emulgatoren sind entsprechende lipophile Substanzen, wie Sorbitanfettsäureester (Spans), z.B. Sorbitanoleat und/oder Sorbitanisostearat. Zusätze zur Wasserphase sind z.B. Feuchthaltungsmittel, wie Polyalkohole, z.B. Glycerin, Propylenglykol, Sorbit und/oder Polyethylenglykol, sowie Konservierungsmittel, Riechstoffe, etc.

Fettsalben sind wasserfrei und enthalten als Grundlage insbesondere Kohlenwasserstoffe, z.B. Paraffin, Vaseline und/oder flüssige Paraffine, ferner natürliche oder partialsynthetische Fette, z.B. Kokosfettsäuretriglycerid, oder vorzugsweise gehärtete Oele, z.B. hydriertes Erdnuss- oder Rizinusöl, ferner Fettsäurepartialester des Glycerins, z.B. Glycerinmono- und -distearat, sowie z.B. die im Zusammenhang mit den Salben erwähnten, die Wasserstoffaufnahmefähigkeit steigernden Fettalkohole, Emulgatoren und/oder Zusätze.

Pasten sind Crémes und Salben mit sekretabsorbierenden Puderbestandteilen, wie Metalloxiden, z.B. Titanoxid oder Zinkoxid, ferner Talk und/oder Aluminiumsilikate, welche die Aufgabe haben, vorhandene Feuchtigkeit oder Sekrete zu binden.

Schäume werden aus Druckbehältern verabreicht und sind in Aerosolform vorliegende flüssige Oel-in-Wasser-Emulsionen, wobei halogenierte Kohlenwasserstoffe, wie Chlorfluorniederalkane, z.B. Dichlordifluormethan und Dichlortetrafluorethan, als Treibmittel verwendet werden. Als Oelphase verwendet man u.a. Kohlenwasserstoffe, z.B. Paraffinöl, Fettalkohole, z.B. Cetylalkohol, Fettsäureester, z.B. Isopropylmyristat, und/oder andere Wachse. Als Emulgatoren verwendet man u.a. Gemische von solchen mit vorwiegend hydrophilen Eigenschaften, wie Polyoxyethylen-sorbitan-fettsäureester (Tweens), und solchen mit vorwiegend lipophilen Eigenschaften, wie Sorbitanfettsäureester (Spans). Dazu kommen die üblichen Zusätze, wie Konservierungsmittel, etc.

Tinkturen und Lösungen weisen meistens eine wässrig-ethanolische Grundlage auf, der u.a. Polyalkohole, z.B. Glycerin, Glykole, und/oder Polyethylenglykol, als Feuchthaltemittel zur Herabsetzung der Verdunstung, und rückfettende Substanzen, wie Fettsäureester mit niedrigen Polyethylenglykolen, d.h. im wässrigen Gemisch lösliche, lipophile Substanzen als Ersatz für die der Haut mit dem Ethanol entzogenen Fettsubstanzen, und, falls notwendig, andere Hilfs- und Zusatzmittel beigegeben sind.

Die Herstellung der topisch verwendbaren pharmazeutischen Präparate erfolgt in an sich bekannter Weise, z.B. durch Lösen oder Suspendieren des Wirkstoffs in der Grundlage oder in einem Teil davon, falls notwendig. Bei Verarbeitung des Wirkstoffs als Lösung wird dieser in der Regel vor der Emulgierung in einer der beiden Phasen gelöst; bei Verarbeitung als Suspension wird er nach der Emulgierung mit einem Teil der Grundlage vermischt und dann dem Rest der Formulierung beigegeben.

Die nachfolgenden Beispiele illustrieren die Erfindung, ohne sie in irgendeiner Form einzuschränken. Die $R_f$-Werte werden auf Kieselgeldünnschichtplatten (Firma Merck, Darmstadt, Deutschland) ermittelt. Das Verhältnis der Laufmittel in den verwendeten Laufmittelgemischen zueinander ist in Volumenanteilen (V/V), Temperaturen sind in Grad Celsius angegeben. Die Konzentration, c, der Substanz im Lösungsmittel(gemisch) ist im Falle der optischen Drehung in Prozent (Gewicht/Volumen) angegeben.

<u>Abkürzungen</u>:

abs. = absolut
Boc = tert.Butyloxycarbonyl
HV = Hochvakuum
i.Vak.= im Vakuum
Me = Methyl
MeOH = Methanol
PTFE = Polytetrafluorethylen, Teflon®
RT = Raumtemperatur
Smp. = Schmelzpunkt
Zers. = Zersetzung

<u>Beispiel 1:</u> 11,4 g (16,77 mMol) N-Propionyl-desmethylmuramyl-L-alanyl-D-isoglutamin-benzhydrylester (α,β-Gemisch), der 1,17 Mol Wasser enthält, werden in 120 ml absolutem Pyridin gelöst. Die so erhaltene Lösung wird mit 7,6 ml (80,3 mMol) Essigsäureanhydrid versetzt und 92 Stunden bei Raumtemperatur gerührt. Danach wird die gelbliche Lösung im Hochvakuum bei 40° eingedampft. Das so erhaltene

Rohprodukt wird mit 100 ml Diethylether verrieben und die so entstehende Suspension 2 Stunden bei Raumtemperatur gerührt. Dann werden die gebildeten Kristalle abgesaugt und mit Diethylether nachgewaschen.

Nach zweimaliger Umkristallisation aus Essigsäureethylester-Isopropanol-Diethylether (100:5:40) erhält man eine 1. Fraktion chromatographisch reinen 1,4,6-Tri-O-acetyl-N-propionyl-desmethylmuramyl-L-alanyl-D-isoglutamin-benzhydrylester (α,β-Gemisch) in Form farbloser Kristalle vom Smp. 165-166°.

Die Mutterlaugen der Umkristallisationen werden vereinigt und im Vakuum eingedampft. Den so erhaltenen Rückstand reinigt man durch Säulenchromatographie an 600 g Kieselgel (Typ 60, reinst, Merck; 0,063-0,2 mm) im System Chloroform-Ethanol (9:1) (10 ml Fraktionen).

Die Fraktionen 217-310 werden vereinigt und im Vakuum eingedampft. Man erhält eine 2. Fraktion chromatographisch reinen 1,4,6-Tri-O-acetyl-N-propionyl-desmethylmuramyl-L-alanyl-D-isoglutamin-benzhydrylester (α,β-Gemisch), die zusammen mit der 1. Fraktion in 200 ml absolutem Methanol gelöst wird. Die so erhaltene, leicht trübe Lösung wird dann durch ein Milliporefilter (Fluoropore, PTFE, 0,2 µm) filtriert und das klare Filtrat im Vakuum bei 40° eingedampft. Der Rückstand wird anschliessend in 50 ml absolutem Essigsäureethylester gelöst, der zuvor durch ein Milliporefilter (Fluoropore, PTFE, 0,2 µm) filtriert worden ist, und durch Zugabe von 20 ml absolutem Diethylether, der ebenfalls durch ein Milliporefilter filtriert worden ist, kristallisiert und mit filtriertem, absolutem Diethylether nachgewaschen. Man erhält 1,4,6-Tri-O-acetyl-N-propionyl-desmethylmuramyl-L-alanyl-D-isoglutamin-benzhydrylester (α,β-Gemisch) in Form farbloser Kristalle vom Smp. 165-166°, die noch 0,27 Mol Wasser enthalten.

$C_{38}H_{48}N_4O_{14} \cdot 0,27\ H_2O$ (789,68)

| | | | | | |
|---|---|---|---|---|---|
| Ber. | C 57,80 | H 6,22 | N 7,10 | O 28,91 | $H_2O$ 0,62 |
| Gef. | C 57,91 | H 6,20 | N 7,11 | O 28,77 | $H_2O$ 0,62 |

$[\alpha]_D^{20}$ = + 32,7 ± 2,1° (c = 0,486; Methanol), $R_f$ = 0,28 (Chloroform:Methanol = 9:1), $R_f$ = 0,70 (Chloroform:Methanol = 4:1).

Ein weiteres Produkt erhält man aus den Fraktionen 105-140 der oben beschriebenen Säulenchromatographie.

Der nach dem Eindampfen im Vakuum erhaltene gelbe Rückstand (0,55 g, Schaum) wird in Cyclohexan-Diethylether-Methylenchlorid (10:30:5) heiss gelöst und die so erhaltene Lösung auf Raumtemperatur abgekühlt. Dabei scheidet sich ein gelbes Oel ab, das nach Abdekantieren des Lösungsmittels 1/2 Stunde mit 50 ml absolutem Diethylether verrührt wird. Die so erhaltenen Kristalle werden abgenutscht und mit absolutem Diethylether nachgewaschen.

Die Kristalle werden dann in 100 ml tert.Butanol-Wasser (1:1) gelöst. Die so erhaltene Lösung wird durch ein Milliporefilter (Fluoropore, PTFE, 0,2 µm) filtriert und lyophilisiert.

Man erhält 1α,4,6-Tri-O-acetyl-2-desoxy-2-propionylamino-D-mannos-3-O-yl-acetyl-L-alanyl-D-isoglutamin-benzhydrylester als farbloses Pulver, das 1,11 Mol Wasser enthält.

$C_{38}H_{48}N_4O_{14} \cdot 1,11\ H_2O$ (804,81)

| | | | | | |
|---|---|---|---|---|---|
| Ber. | C 56,71 | H 6,31 | N 6,96 | O 30,03 | $H_2O$ 2,48 |
| Gef. | C 56,94 | H 6,36 | N 7,22 | O 30,15 | $H_2O$ 2,48 |

$[\alpha]_D^{20}$ = + 5,5 ± 2,7° (c = 0,365; Methanol), $R_f$ = 0,49 (Chloroform:Methanol = 9:1), $R_f$ = 0,79 (Chloroform:Methanol = 4:1).

Das Ausgangsmaterial erhält man wie folgt:

Stufe 1.1: 16,0 g (31,63 mMol) N-Propionyl-desmethylmuramyl-L-alanyl-D-isoglutamin, das 0,74 Mol Wasser enthält, werden in 300 ml Methanol-Dimethoxyethan (1:1) gelöst und anschliessend mit 9,2 g (47,4 mMol) Diphenyldiazomethan versetzt. Das Ganze wird 20 Stunden bei Raumtemperatur gerührt. Nach 20, 44 und 68 Stunden werden noch jeweils 4,6 g (23,7 mMol) Diphenyldiazomethan zugegeben. Nach beendeter Reaktion (90 Stunden) wird die rote Lösung im Vakuum bei 40° zur Trockne eingedampft, das so erhaltene rote Harz mit 300 ml absolutem Diethylether versetzt und 1/2 Stunde bei Raumtemperatur gerührt.

Man erhält farblose Kristalle, die abgenutscht und mit Diethylether nachgewaschen werden.

Nach Umkristallisation aus 400 ml Aceton erhält man N-Propionyl-desmethylmuramyl-L-alanin-D-isoglutamin-benzhydrylester in Form farbloser Kristalle vom Smp. 188-190° (Zers.), die 1,17 Mol Wasser enthalten.

$C_{32}H_{42}N_4O_{11}$ • 1,17 $H_2O$ (679,78)

| | | | | | |
|---|---|---|---|---|---|
| Ber. | C 56,55 | H 6,60 | N 8,24 | O 28,63 | $H_2O$ 3,09 |
| Gef. | C 56,67 | H 6,67 | N 8,43 | O 28,65 | $H_2O$ 3,09 |

$[\alpha]_D^{20}$ = + 7,5 ± 0,1° (c = 0,898; Methanol), $R_f$ = 0,62 (Chloroform:Methanol:Wasser = 70:30:5), $R_f$ = 0,62 (Chloroform:Methanol = 7:3).

Beispiel 2: 2,33 g (3,8 mMol) N-Propionyl-desmethylmuramyl-L-alanyl-D-isoglutamin-benzylester, der noch 1,59 Mol Wasser enthält, werden in 24 ml absolutem Pyridin gelöst, dann 1,4 ml (14,8 mMol) Essigsäureanhydrid zugegeben und die so erhaltene Lösung wird 16 Stunden bei Raumtemperatur gerührt. Anschliessend wird die farblose Lösung im Hochvakuum bei 30° eingedampft. Man erhält einen leicht gelben Schaum, der durch Säulenchromatographie an 400 g Kieselgel (Typ 60, reinst, Merck; 0,063-0,2 mm) im System Chloroform-Ethanol (9:1) gereinigt wird (10 ml Fraktionen), wobei man nacheinander das β-Anomere, das α,β-Anomerengemisch und das α-Anomere des 1,4,6-Tri-O-acetyl-N-propionyl-desmethylmuramyl-L-alanyl-D-isoglutamin-benzylesters eluiert.

Die Fraktionen 112-126 (β-Anomeres), 127-190 (α,β-Gemisch) und 191-260 (α-Anomeres) werden jeweils vereinigt und im Hochvakuum bei 30° eingedampft.

Der Rückstand der Fraktionen 112-126 wird aus 60 ml Chloroform:Diethylether (1:5) kristallisiert. Man erhält 1β,4,6-Tri-O-acetyl-N-propionyl-desmethylmuramyl-L-alanyl-D-isoglutamin-benzylester in Form farbloser Kristalle vom Smp. 189-190°. Die Kristalle werden anschliessend in 100 ml doppelt destilliertem Wasser-tert.Butanol (1:1) gelöst. Die so erhaltene Lösung wird durch ein Milliporefilter (Fluoropore, PTFE, 0,2 µm) filtriert und im Hochvakuum lyophilisiert. Man erhält 1β,4,6-Tri-O-acetyl-N-propionyl-desmethylmuramyl-L-alanyl-D-isoglutamin-benzylester als farbloses Pulver, das 1,21 Mol Wasser enthält.

$C_{32}H_{44}N_4O_{14}$ • 1,21 $H_2O$ (730,52)

| | | | | | |
|---|---|---|---|---|---|
| Ber. | C 52,62 | H 6,43 | N 7,67 | O 33,30 | $H_2O$ 2,98 |
| Gef. | C 52,97 | H 6,27 | N 7,86 | O 33,24 | $H_2O$ 2,98 |

$[\alpha]_D^{20}$ = + 14,3 ± 4,2° (c = 0,237; Dimethylformamid), $R_f$ = 0,25 (Chloroform:Ethanol = 9:1), $R_f$ = 0,67 (Chloroform: Ethanol = 4:1), $R_f$ = 0,45 (Chloroform:Methanol = 9:1).

Der Rückstand der Fraktionen 127-190 kristallisiert aus 120 ml Chloroform-Diethylether (1:5). Man erhält 1,4,6-Tri-O-acetyl-N-propionyl-desmethylmuramyl-L-alanyl-D-isoglutamin-benzylester (α,β-Gemisch, nach $^1$H-NMR α:β ≈ 6:1) in Form farbloser Kristalle vom Smp. 156-157°.

Die Kristalle werden anschliessend in 150 ml doppelt destilliertem Wasser-tert.Butanol (1:1) gelöst. Die so erhaltene Lösung wird durch ein Milliporefilter (Fluoropore, PTFE, 0,2 µm) filtriert und im Hochvakuum lyophilisiert. Man erhält 1,4,6-Tri-O-acetyl-N-propionyl-desmethylmuramyl-L-alanyl-D-isoglutamin-benzylester (α,β-Gemisch) als farbloses Pulver, das 1,00 Mol Wasser enthält.

$C_{32}H_{44}N_4O_{14}$ • 1,00 $H_2O$ (726,73)
Ber. C 52,89 H 6,41 N 7,71 O 33,02 $H_2O$ 2,48
Gef. C 53,18 H 6,42 N 7,49 O 32,81 $H_2O$ 2,48
$[\alpha]_D^{20}$ = + 56,0 ± 1,1° (c = 0,948; Dimethylformamid),
$R_f$ = 0,17 + 0,24 (Chloroform:Ethanol = 9:1; Doppelfleck), $R_f$ = 0,45 (Chloroform:Methanol = 9:1), $R_f$ = 0,67 (Chloroform:Methanol = 4:1).

Der Rückstand der Fraktionen 191-260 kristallisiert aus 90 ml Chloroform-Diethylether (1:5). Man erhält 1α,4,6-Tri-O-acetyl-N-propionyl-desmethylmuramyl-L-alanyl-D-isoglutamin-benzylester in Form farbloser Kristalle vom Smp. 147-149°.

Die Kristalle werden anschliessend in 100 ml doppelt destilliertem Wasser-tert.Butanol (1:1) gelöst. Die so erhaltene Lösung wird durch ein Milliporefilter (Fluoropore, PTFE, 0,2 µm) filtriert und im Hochvakuum lyophilisiert. Man erhält 1α,4,6-Tri-O-acetyl-desmethylmuramyl-N-propionyl-L-alanyl-D-isoglutamin-benzylester als farbloses Pulver, das noch 1,12 Mol Wasser enthält.

$C_{32}H_{44}N_4O_{14}$ • 1,12 $H_2O$ (728,89)
Ber. C 52,74 H 6,43 N 7,69 O 33,18 $H_2O$ 2,76
Gef. C 52,96 H 6,54 N 7,62 O 33,15 $H_2O$ 2,76
$[\alpha]_D^{20}$ = + 36,9 ± 1,7° (c = 0,593; Methylenchlorid:Methanol = 1:1),
$R_f$ = 0,17 (Chloroform:Ethanol = 9:1), $R_f$ = 0,45 (Chloroform:Methanol = 9:1), $R_f$ = 0,67 (Chloroform:Methanol = 4:1).

Beispiel 3: Analog Beispiel 1 erhält man aus 1,19 g (1,54 mMol) N-Propionyl-desmethylmuramyl-L-alanyl-D-isoglutaminyl-L-alanin-benzhydrylester (α,β-Gemisch) der 2,24 Mol Wasser enthält, mit 0,7 ml (7,4 mMol) Essigsäureanhydrid in 12 ml Pyridin (20 Stunden, Raumtemperatur) 1,4,6-Tri-O-acetyl-N-propionyl-desmethylmuramyl-L-alanyl-D-isoglutaminyl-L-alanin-benzhydrylester (α,β-Gemisch) als farbloses Pulver, das 1,33 Mol Wasser enthält.

$C_{41}H_{53}N_5O_{15}$ • 1,33 $H_2O$ (879,85)

| | | | | | |
|---|---|---|---|---|---|
| Ber. | C 55,97 | H 6,40 | N 7,96 | O 29,69 | $H_2O$ 2,72 |
| Gef. | C 56,14 | H 6,36 | N 7,98 | O 29,56 | $H_2O$ 2,72 |

$[\alpha]_D^{20}$ = + 23,8 ± 2,1° (c = 0,478; Dimethylformamid), $R_f$ = 0,36 (Chloroform:Methanol = 9:1), $R_f$ = 0,69 (Chloroform:Methanol = 4:1), $R_f$ = 0,87 (Chloroform:Methanol = 7:3).

Das Ausgangsmaterial erhält man wie folgt:

Stufe 3.1: Analog Beispiel 1 erhält man aus 2,11 g (4,07 mMol) N-Propionyl-desmethylmuramyl-L-alanyl-D-isoglutaminyl-L-alanin, das 1,56 Mol Wasser enthält, mit insgesamt 2,14 g (11,06 mMol) Diphenyldiazomethan in 42 ml Methanol-Dimethoxyethan (1:1) (18 Stunden, Raumtemperatur) N-Propionyl-desmethylmuramyl-L-alanyl-D-isoglutaminyl-L-alanin-benzhydrylester als farbloses Pulver, das 2,24 Mol Wasser enthält.

$C_{35}H_{47}N_5O_{12}$ • 2,24 $H_2O$ (770,14)

| | | | | | |
|---|---|---|---|---|---|
| Ber. | C 54,58 | H 6,76 | N 9,09 | O 29,59 | $H_2O$ 5,25 |
| Gef. | C 54,85 | H 6,86 | N 9,11 | O 29,21 | $H_2O$ 5,25 |

$[\alpha]_D^{20}$ = + 2,9 ± 2° (c = 0,478; Dimethylformamid), $R_f$ = 0,57 (Chloroform:Methanol = 7:3), $R_f$ = 0,65 (Chloroform:Methanol:Wasser = 70:30:5).

Beispiel 4: Analog Beispiel 1 erhält man aus 0,767 g (1,09 mMol) N-Propionyl-desmethylmuramyl-L-alanyl-D-glutaminsäure-($C_\alpha$)-methyl ester-($C_\gamma$)-benzhydrylester, der noch 1,42 Mol Wasser enthält, mit 0,49 ml (5,2 mMol) Essigsäureanhydrid in 8,7 ml Pyridin (44 Stunden, Raumtemperatur) 1,4,6-Tri-O-acetyl-N-propionyl-desmethylmuramyl-L-alanyl-D-glutaminsäure-($C_\alpha$)-methylester-($C_\gamma$)-benzhydrylester ($\alpha$,$\beta$-Gemisch) als farbloses Pulver, das 0,71 Mol Wasser enthält.

$C_{39}H_{49}N_3O_{15}$ • 0,71 $H_2O$ (812,62)

| | | | | | |
|---|---|---|---|---|---|
| Ber. | C 57,65 | H 6,28 | N 5,17 | O 30,92 | $H_2O$ 1,57 |
| Gef. | C 58,09 | H 6,23 | N 5,28 | O 30,71 | $H_2O$ 1,57 |

$[\alpha]_D^{20}$ = + 53,9 ± 1,9° (c = 0,519; Dimethylformamid), $R_f$ = 0,55 (Chloroform:Methanol = 9:1), $R_f$ = 0,87 (Chloroform:Methanol = 4:1), $R_f$ = 0,95 (Chloroform:Methanol = 7:3).

Das Ausgangsmaterial erhält man wie folgt:

Stufe 4.1: Analog Beispiel 1 erhält man aus 1,32 g (2,5 mMol) N-Propionyl-desmethylmuramyl-L-alanyl-D-glutaminsäure-($C_\alpha$)-methylester, der noch 0,68 Mol Wasser enthält, mit insgesamt 1,4 g (7,2 mMol) Diphenyldiazomethan in 27 ml Methanol-Dimethoxyethan (1:1) (18 Stunden, Raumtemperatur) N-Propionyl-desmethylmuramyl-L-alanyl-D-glutaminsäure-($C_\alpha$)-methylester-($C_\gamma$)-benzhydrylester als farbloses Pulver, das 1,42 Mol Wasser enthält.

$C_{33}H_{43}N_3O_{12}$ • 1,42 $H_2O$ (699,30)

| | | | | | |
|---|---|---|---|---|---|
| Ber. | C 56,69 | H 6,63 | N 6,01 | O 30,70 | $H_2O$ 3,65 |
| Gef. | C 56,75 | H 6,73 | N 6,21 | O 30,74 | $H_2O$ 3,65 |

$[\alpha]_D^{20}$ = + 10,3 ± 2,3° (c = 0,435; Wasser), $R_f$ = 0,73 (Chloroform:Methanol:Wasser = 70:30:5), $R_f$ = 0,91 (Chloroform:Methanol = 7:3).

Beispiel 5: Analog Beispiel 1 erhält man aus 1,15 g (1,57 mMol) N-Propionyl-desmethylmuramyl-L-alanyl-D-glutaminsäure-($C_\alpha$)-n-butylester-($C_\gamma$)-benzhydrylester, der noch 0,99 Mol Wasser enthält, mit 0,70 ml (7,4 mMol) Essigsäureanhydrid in 12 ml Pyridin (16 Stunden,

Raumtemperatur) 1,4,6-Tri-O-acetyl-N-propionyl-desmethylmuramyl-L-alanyl-D-glutaminsäure-($C_\alpha$)-n-butylester-($C_\gamma$)-benzhydrylester (α,β-Gemisch) als farbloses Pulver, das 0,73 Mol Wasser enthält.

$C_{42}H_{55}N_3O_{15}$ • 0,73 $H_2O$ (855,08)

| | | | | | |
|---|---|---|---|---|---|
| Ber. | C 58,99 | H 6,60 | N 4,91 | O 29,50 | $H_2O$ 1,54 |
| Gef. | C 58,96 | H 6,72 | N 4,76 | O 29,49 | $H_2O$ 1,54 |

$[\alpha]_D^{20}$ = + 12,6 ± 2,3° (c = 0,443; Chloroform), $R_f$ = 0,72 (Chloroform:Methanol = 9:1), $R_f$ = 0,96 (Chloroform:Methanol = 4:1).

Das Ausgangsmaterial erhält man wie folgt:

Stufe 5.1: Analog Beispiel 1 erhält man aus 1,9 g (3,3 mMol) N-Propionyl-desmethylmuramyl-L-alanyl-D-glutaminsäure-($C_\alpha$)-n-butylester, der 1,03 Mol Wasser enthält, mit insgesamt 2,5 g (12,85 mMol) Diphenyldiazomethan in 40 ml Methanol-Dimethoxyethan (1:1; 70 Stunden, Raumtemperatur) N-Propionyl-desmethylmuramyl-L-alanyl-D glutaminsäure-($C_\alpha$)-n-butylester-($C_\gamma$)-benzhydrylester als farbloses Pulver, das 0,99 Mol Wasser enthält.

$C_{36}H_{49}N_3O_{12}$ • 0,99 $H_2O$ (733,62)

| | | | | | |
|---|---|---|---|---|---|
| Ber. | C 58,94 | H 7,03 | N 5,73 | O 28,32 | $H_2O$ 2,43 |
| Gef. | C 59,24 | H 7,06 | N 5,59 | O 28,08 | $H_2O$ 2,43 |

$[\alpha]_D^{20}$ = + 12,5 ± 1,9° (c = 0,522; Methanol), $R_f$ = 0,46 (Chloroform:Methanol = 9:1), $R_f$ = 0,63 (Chloroform:Methanol = 4:1), $R_f$ = 0,88 (Chloroform:Methanol = 7:3).

Beispiel 6: Analog Beispiel 1 erhält man aus 1,0 g (1,43 mMol) rohem N-Acetyl-muramyl-L-alanyl-D-glutaminsäure-($C_\alpha$)-pivaloyloxymethylester-($C_\gamma$)-benzylester und 0,57 ml (6,0 mMol) Essigsäureanhydrid in 10 ml absolutem Pyridin (20 Stunden, Raumtemperatur) N-Acetyl-1,4,6-tri-O-acetyl-muramyl-L-alanyl-D-glutaminsäure-($C_\alpha$)-pivaloyloxymethylester-($C_\gamma$)-benzylester (α,β-Gemisch) als farbloses Pulver, das 0,79 Mol Wasser enthält.

$C_{38}H_{53}N_3O_{17}$ • 0,79 $H_2O$ (838,08)

| | | | | | |
|---|---|---|---|---|---|
| Ber. | C 54,47 | H 6,60 | N 5,02 | O 33,96 | $H_2O$ 1,69 |
| Gef. | C 54,52 | H 6,36 | N 5,12 | O 33,71 | $H_2O$ 1,69 |

$[\alpha]_D^{20}$ = + 68,5 ± 4,1° (c = 0,241; Dimethylformamid), $R_f$ = 0,52 (Chloroform:Methanol = 9:1), $R_f$ = 0,55 (Chloroform:Ethanol = 9:1).

Das Ausgangsmaterial erhält man folgendermassen:

Stufe 6.1: 20,2 g (60 mMol) N-tert.-Butyloxycarbonyl-D-glutaminsäure-($C_\gamma$)-benzylester werden in einem Gemisch aus 300 ml Tetrahydrofuran und 50 ml Wasser gelöst. Zu dieser Lösung gibt man 19,55 g (60 mMol) Cäsiumcarbonat (purum, Fluka), gelöst in 100 ml Wasser, und lässt das Ganze 1/2 Stunde bei Raumtemperatur stehen. Anschliessend wird im Hochvakuum bei 30° eingedampft und der so erhaltene Rückstand nacheinander mit 200 ml Methanol und zweimal mit je 200 ml Dimethylformamid im Hochvakuum bei 40° abgedampft.

Das so erhaltene Cäsiumsalz des N-tert.-Butyloxycarbonyl-D-glutaminsäure-($C_\gamma$)-benzylesters (28,1 g; 60 mMol) wird in 500 ml Dimethylformamid suspendiert. Zu dieser Suspension werden bei Raumtemperatur 18,0 g (17,4 ml; 120 mMol) Pivalinsäurechlormethylester (purum, Fluka) und 18,0 g (120 mMol) Natriumiodid gegeben. Das so erhaltene Gemisch wird 17 Stunden bei Raumtemperatur gerührt. Danach wird filtriert und das Filtrat im Hochvakuum bei 40° eingedampft. Der so erhaltene Rückstand wird in 500 ml Essigsäureethylester aufgenommen und nacheinander je dreimal mit 150 ml 0,1 N Natriumthiosulfatlösung und Wasser gewaschen. Nach Trocknen der Essigesterphase über Natriumsulfat wird erneut im Vakuum eingedampft. Der noch schwach gelbe Rückstand wird aus 450 ml Methanol-Wasser (1:2) umkristallisiert. Man erhält N-tert.-Butyloxycarbonyl-D-glutaminsäure-($C_\alpha$)-pivaloyloxymethylester-($C_\gamma$)-benzylester in Form farbloser Kristalle mit Smp. 76-77°.

$C_{23}H_{33}NO_8$ (451,52)

| | | | | |
|---|---|---|---|---|
| Ber. | C 61,18 | H 7,37 | N 3,10 | O 28,35 |
| Gef. | C 61,08 | H 7,25 | N 3,33 | O 28,58 |

$[\alpha]_D^{20}$ = + 18,4 ± 0,1° (c = 1,034; Essigsäureethylester), $R_f$ = 0,85 (Methylenchlorid:Methanol = 9:1).

Stufe 6.2: 20,0 g (44,3 mMol) N-tert.-Butyloxycarbonyl-D-glutaminsäure-($C_\alpha$)-pivaloyloxymethylester-($C_\gamma$)-benzylester werden bei 0° in einem Gemisch von 400 ml Trifluoressigsäure-Methylenchlorid (1:1) 1,5 Stunden gerührt. Dann wird die Reaktionslösung im Hochvakuum bei 30° eingedampft und der so erhaltene Rückstand mehrmals in Methylenchlorid aufgenommen und erneut eingedampft. Man erhält ein gelbes Oel, das in einem Gemisch von 100 ml Wasser und 300 ml Diethylether aufgenommen und auf 0° gekühlt wird. Zu der Lösung gibt man unter starkem Rühren 80 ml 1N Natronlauge (pH 7,5) und trennt die Etherphase ab. Die etherische Lösung wird einmal mit 100 ml Wasser, die Wasserphase einmal mit 200 ml Diethylether extrahiert. Die Etherphasen werden vereinigt, über Natriumsulfat getrocknet und filtriert. Das rötliche Filtrat wird dann mit 8,43 g (44,3 mMol) p-Toluolsulfonsäure-monohydrat (puriss., Fluka), gelöst in 100 ml Diethylether, versetzt und 1 Stunde bei Raumtemperatur, dann 1/2 Stunde bei 0° gerührt. Die so erhaltenen Kristalle werden abgesaugt und mit Diethylether gewaschen. Das erhaltene Rohprodukt wird aus 400 ml Essigsäureethylester umkristallisiert. Man erhält D-Glutaminsäure-($C_\alpha$)-pivaloyloxymethylester-($C_\gamma$)-benzylester-tosylat in Form farbloser Kristalle vom Smp. 135-137°.

$C_{25}H_{33}NO_9S$ (523,60)

| | | | | | |
|---|---|---|---|---|---|
| Ber. | C 57,35 | H 6,35 | N 2,68 | O 27,50 | S 6,12 |
| Gef. | C 57,39 | H 6,52 | N 2,72 | O 27,53 | S 6,12 |

$[\alpha]_D^{20}$ = + 8,8 ± 0,1° (c = 0,924; Methylenchlorid),

$R_f$ = 0,78 (Methylenchlorid:Methanol = 9:1; Doppelfleck),

$R_f$ = 0,84 (Methylenchlorid:Methanol = 5:1; Doppelfleck).

Stufe 6.3: 2,6 g (6,5 mMol) N-Acetyl-muramyl-L-alanin-natriumsalz, das 0,63 Mol Wasser enhält, werden in 50 ml Dimethylformamid gelöst. Zu dieser Lösung gibt man bei Raumtemperatur 1,47 g (7,14 mMol) N,N-Dicyclohexylcarbodiimid, 0,74 g (7,14 mMol) N-Hydroxy-succinimid und 3,41 g (6,5 mMol) D-Glutaminsäure-($C_\alpha$)-pivaloyloxymethylester-($C_\gamma$)-benzylester-tosylat und rührt die so erhaltene klare, farblose Lösung 14 Stunden bei Raumtemperatur.

Die während der Reaktion ausgefallenen Kristalle (N,N-Dicyclohexylharnstoff) werden dann abgesaugt und das Filtrat wird im Hochvakuum bei 40° eingedampft. Der Rückstand wird in 300 ml Essigsäureethylester aufgenommen, filtriert, und die so erhaltene Lösung nacheinander viermal mit je 100 ml Wasser, dreimal mit je 100 ml gesättigter wässeriger Natriumsulfatlösung und zweimal mit je 100 ml Wasser gewaschen.

Die Essigesterphasen werden vereinigt, über Natriumsulfat getrocknet, filtriert und erneut eingedampft.

Man erhält einen leicht gelben Schaum, der durch Säulenchromatographie an 440 g Kieselgel (Typ 60, reinst, Merck; 0,063-0,200 mm) im System Aceton-Essigsäureethylester (7:3) gereinigt wird (10 ml Fraktionen). Die Fraktionen 21-70 werden vereinigt und im Vakuum bei 40° eingedampft. Man erhält schwach verunreinigten N-Acetyl-muramyl-L-alanyl-D-glutaminsäure-($C_\alpha$)-pivaloyloxymethylester-($C_\gamma$)-benzylester als farblosen Schaum, der ohne weitere Reinigung verarbeitet wird.
$R_f$ = 0,74 (Chloroform:Methanol:Wasser = 70:30:5), $R_f$ = 0,85 (Chloroform:Methanol = 7:3).

Beispiel 7: 10 g (15,86 mMol) N-Benzoyl-desmethylmuramyl-L-alanyl-D-isoglutamin-benzylester löst man in 100 ml Pyridin und versetzt mit 9 ml Acetanhydrid. Nach 24 Stunden bei Raumtemperatur dampft man am Hochvakuum ein, nimmt den Rückstand in Methylenchlorid auf und schüttelt die organische Phase nacheinander mit je 300 ml 1N Salzsäure, Wasser, 5%iger $NaHCO_3$-Lösung und Wasser aus. Die wässrigen Phasen extrahiert man zweimal mit je 150 ml Methylenchlorid. Man trocknet die vereinigten organischen Phasen mit Natriumsulfat und dampft ein.

Zwecks Kristallisation löst man in 20 ml Ethanol und gibt 30 ml Essigsäureethylester und dann Diethylether bis zur Trübung (~ 60 ml) zu. Man erhält farblose Kristalle des α,β-1,4,6-Tri-O-acetyl-N-benzoyl-desmethylmuramyl-L-alanyl-D-isoglutamin-benzylesters mit Smp. 168-169°. Aus der Mutterlauge lassen sich weitere Kristalle gewinnen.
$[\alpha]_D^{20}$ = + 44,9° ± 1,2° (c = 0,813, $CHCl_3$),
$R_f$ = 0,54 ($CHCl_3$:Methanol = 9:1).

Beispiel 8: Man versetzt 4,5 g (6,4 mMol) 1,4,6-Tri-O-acetyl-N-benzoyl-desmethylmuramyl-L-alanyl-D-isoglutamin, gelöst in 60 ml Acetonitril, mit 3,7 g (19,1 mMol) Diphenyldiazomethan in 30 ml Acetonitril. Nach 5 Stunden bei Raumtemperatur ist die Reaktion beendet; man dampft im Vakuum zur Trockne ein, extrahiert den Rückstand viermal mit je 20 ml Diethylether und reinigt ihn chromatographisch über 250 g Kieselgel (Merck, 0,04-0,6 mm). Man eluiert nacheinander mit 1 Liter Methylenchlorid-Aceton (9:1, Fraktion 1), pro Fraktion je 500 ml $CH_2Cl_2$-Aceton (7:3, Fraktionen 2 und 3) und pro Fraktion je 1 Liter $CH_2Cl_2$-Aceton (1:1, Fraktionen 4, 5 und 6). Man erhält so aus Fraktion 1 unreines α-Anomeres, aus Fraktion 3 reines α-Anomeres und aus den Fraktionen 4 und 5 α,β-Anomerengemisch des 1,4,6-Tri-O-acetyl-N-benzoyl-desmethylmuramyl-L-alanyl-D-isoglutamin-benzhydrylesters.
α-Anomer: Smp. 108-110°, $[\alpha]_D^{20}$ = + 36,3 ± 1° (c = 1,01, $CHCl_3$),
$R_f$ = 0,27 ($CHCl_3$:Aceton = 1:1).

Das Ausgangsmaterial, 1,4,6-Tri-O-acetyl-N-benzoyl-desmethylmuramyl-L-alanyl-D-isoglutamin, erhält man durch katalytische Hydrierung des entsprechenden Benzylesters (Beispiel 7) mit 5%iger Palladium-Kohle in Dimethoxyethan als Anomerengemisch. Smp. 114-120°,
$[\alpha]_D^{20}$ = + 51,9° ± 1° (c = 0,963, $H_2O$), $R_f$ = 0,16 ($CHCl_3$:Methanol:$H_2O$ = 85:15:2).

Beispiel 9: 1,5 g (2 mMol) N-Benzoyl-(1α,β),4,6-tri-O-butyryl-desmethylmuramyl-L-alanyl-D-isoglutamin setzt man analog Beispiel 8 mit Diphenyldiazomethan um. Nach analoger Aufarbeitung erhält man ein Rohprodukt, das an 50 g Kieselgel (Merck, 0,04-0,6 mm) chromatographiert wird. Man eluiert nacheinander mit 1 Liter $CH_2Cl_2$-Aceton

(9:1, Fraktion 1), pro Fraktion je 1 Liter $CH_2Cl_2$-Aceton (8:2, Fraktionen 2 und 3) und pro Fraktion je 500 ml $CH_2Cl_2$-Aceton (7:3, Fraktionen 4 und 5).

Aus Fraktion 2 erhält man α-Anomeres, aus Fraktion 4 unreines β-Anomeres des N-Benzoyl-1,4,6-tri-O-butyryl-desmethylmuramyl-L-alanyl-D-isoglutamin-benzhydrylesters. Das α-Anomere erhält man kristallin durch Lösen in Aceton (0,7 ml/g) und Zugabe von Diethylether (22 ml/g). Smp. 109-113°, $[\alpha]_D^{20}$ = + 45,1° ± 1,1° (c = 0,92, $CHCl_3$), $R_f$ = 0,62 ($CHCl_3$:Aceton = 1:1).

Das β-Anomere erhält man kristallin durch Lösen in 8 ml Aceton bei 50° und Zugabe von 20 ml Diethylether bei Raumtemperatur.

Smp. 169-171°, $[\alpha]_D^{20}$ = + 13,8° ± 1,3° (c = 0,741, $CHCl_3$:Methanol = 1:1), $R_f$ = 0,52 ($CHCl_3$:Aceton = 1:1).

Das Ausgangsmaterial erhält man folgendermassen:

<u>Stufe 9.1:</u> N-Benzoyl-(1α,β),4,6-tri-O-butyryl-desmethylmuramyl-L-alanyl-D-isoglutamin-benzylester erhält man in bekannter Weise aus N-Benzoyl-desmethylmuramyl-L-alanyl-D-isoglutamin-benzylester in Pyridin mit 6 Aequivalenten Buttersäureanhydrid unter Zugabe katalytischer Mengen 4-Dimethylaminopyridin. Nach üblicher Aufarbeitung erhält man beim Verreiben mit Diethylether Kristalle vom Smp. 166-171°, $[\alpha]_D^{20}$ = + 43,2° ± 1° (c = 0,954, $CHCl_3$), α-Anomeres: $R_f$ = 0,42 ($CHCl_3$:Aceton = 1:1), β-Anomeres: $R_f$ = 0,28 ($CHCl_3$:Aceton = 1:1).

<u>Stufe 9.2:</u> Durch katalytische Hydrierung von N-Benzoyl-(1α,β),4,6-tri-O-butyryl-desmethylmuramyl-L-alanyl-D-isoglutamin-benzylester mit 5%iger Palladium-Kohle in Tetrahydrofuran erhält man N-Benzoyl-(1α,β),4,6-tri-O-butyryl-desmethylmuramyl-L-alanyl-D-isoglutamin als Hemihydrat mit Smp. 100-105°,

$[\alpha]_D^{20}$ = + 54,2° ± 1° (c = 0,96, Methanol), $R_f$ = 0,45 ($CHCl_3$: Methanol = 8:2).

<u>Beispiel 10:</u> Analog Beispiel 1 setzt man 3,69 g (5,6 mMol) N-Acetyl-desmethylmuramyl-L-alanyl-D-isoglutamin-benzhydrylester, gelöst in 40 ml abs. Pyridin, mit 2,04 g (20 mMol) Essigsäureanhydrid (1 Stunde, Raumtemperatur) um. Die klare Lösung wird bei 30° stark eingeengt, der Rückstand in 150 ml Essigsäureethylester aufgenommen und die Lösung zweimal mit je 50 ml Wasser extrahiert. Nach Trocknen und Verdampfen des Lösungsmittels verbleibt N-Acetyl-(1α,β),4,6-tri-O-acetyl-desmethylmuramyl-L-alanyl-D-isoglutamin-benzhydrylester.

$[\alpha]_D^{20}$ = + 33° ± 1° (c = 1,159, Methanol), $R_f$ = 0,70 (Chloroform: Methanol:Wasser = 70:30:5), $R_f$ = 0,48 (n-Butanol:Essigsäure:Wasser = 75:7,5:21) und $R_f$ = 0,81 (Essigsäureethylester:n-Butanol:Pyridin: Essigsäure:Wasser = 42:21:21:6:10).

Der N-Acetyl-desmethylmuramyl-L-alanyl-D-isoglutamin-benzhydrylester kann wie folgt hergestellt werden:

<u>Stufe 10.1:</u> Eine Lösung von 22,8 g (40 mMol) N-Acetyl-desmethylmuramyl-L-alanyl-D-isoglutamin in 500 ml eines 1:1-Gemisches von 1,2-Dimethoxy-ethan und Methanol wird mit 11,6 g (60 mMol) Diphenyldiazomethan versetzt, und die Lösung während 16 Stunden bei Raumtemperatur gerührt. Die rote Suspension wird bei 20° und unter vermindertem Druck eingedampft, und der Rückstand mehrfach mit Diethylether verrieben, bis ein fast farbloses Produkt erhalten wird. Dieses wird in 100 ml Methanol gelöst und durch portionenweise Zugabe eines 2:1-Gemisches von Diethylether:Petrolether zur Kristallisation gebracht. Nach mehrstündigem Rühren, zunächst bei Raumtemperatur, dann im Eisbad, wird die Kristallmasse abfiltriert und unter vermindertem Druck getrocknet. Man erhält so den N-Acetyl-desmethylmuramyl-L-alanyl-D-isoglutamin-benzhydrylester in Form von würfelförmigen Kristallen. Smp. 170° (mit Zersetzung),

$[\alpha]_D^{20}$ = + 14 ± 1° (c = 1,5, Methanol), $R_f$ = 0,40 (Chloroform: Methanol:Wasser = 70:30:5) und $R_f$ = 0,66 (Essigsäureethylester: n-Butanol:Pyridin:Eisessig:Wasser = 42:21:21:6:10).

Beispiel 11: Analog Beispiel 10 erhält man aus 0,55 g (0,85 mMol) N-Acetyl-desmethylmuramyl-L-α-aminobutyryl-D-isoglutamin-benzhydrylester, gelöst in 5 ml abs. Pyridin, und 0,281 g (2,76 mMol) Essigsäureanhydrid (30 Minuten, Raumtemperatur) N-Acetyl-(1α,β),4,6-tri-O-acetyl-desmethylmuramyl-L-α-aminobutyryl-D-isoglutamin-benzhydrylester; $R_f$ = 0,63 (Chloroform:Methanol:Wasser = 70:30:5) und $R_f$ = 0,82 (Essigsäureethylester:n-Butanol:Pyridin: Essigsäure:Wasser = 42:21:21:6:10).

Beispiel 12: Aus 2,10 g (2,54 mMol) N-Acetyl-desmethylmuramyl-L-α-aminobutyryl-D-glutaminsäure-dibenzhydrylester und 1,053 ml (11,43 mMol) Essigsäureanhydrid in 6 ml absolutem Pyridin erhält man analog Beispiel 1 nach 12stündigem Stehen bei Raumtemperatur die Peracetylverbindung. Die Reinigung erfolgt an 450 g Kieselgel (Typ 60, Merck; Korngrösse 0,063-0,200 mm; 5 ml Fraktionen) erst mit Chloroform, dann ab Fraktion 20 mit Chloroform:Isopropanol (3:1). Das in den Fraktionen 43-106 enthaltene Material wird gesammelt, in 5 ml Chloroform gelöst, nach Zugabe von 40 ml abs. Dioxan durch ein Millipore-Filter (Fluoropore, PTFE, 0,2 µm) filtriert und dann lyophilisiert. Man erhält N-Acetyl-(1α,β),4,6-tri-O-acetyl-desmethylmuramyl-L-α-aminobutyryl-D-glutaminsäure-dibenzhydrylester als farbloses, lockeres Pulver, 0,37 Mol Wasser enthaltend.

$C_{51}H_{57}N_3O_{15}$ • 0,37 $H_2O$ (958,69)

| | | | | |
|---|---|---|---|---|
| Ber. | C 63,90 | H 6,09 | N 4,38 | $H_2O$ 0,69 |
| Gef. | C 63,6 | H 6,1 | N 4,6 | $H_2O$ 0,70 |

$[\alpha]_D^{20}$ = + 29,6 ± 1,7° (c = 0,577; Methanol), $R_f$ = 0,69 (n-Butanol: Essigsäure:Wasser = 75:7,5:21) und $R_f$ = 0,93 (Essigsäureethylester: n-Butanol:Pyridin:Essigsäure:Wasser = 42:21:21:6:10).

Das Ausgangsmaterial erhält man analog Beispiel 1 wie folgt: 3,00 g (6,1 mMol) N-Acetyl-desmethylmuramyl-L-α-aminobutyryl-D-glutaminsäure, gelöst in 30 ml eines 1:1 Gemisches aus Dimethylformamid und Methanol, werden bei Raumtemperatur und Rühren mit überschüssigem Diphenyldiazomethan versetzt. Nach 3 Tagen wird die rote Suspension im Hochvakuum bei 30° eingedampft, der rote Rückstand mehrfach mit Diethylether-Petrolether (1:3) verrieben und das Ueberstehende abdekantiert. Der feste Rückstand wird erst durch Chromatographie an 600 g Kieselgel (Typ 60, Merck) im System Chloroform-Methanol-Wasser (70:30:5; u.a. zwecks Entfernung von Halbester), dann auf einer zweiten Säule (100 g) erst mit Essigsäureethylester, dann Essigsäureethylester-Methanol (3:1; 5 ml Fraktionen) gereinigt.

Das gesammelte Material wird in 4,5 ml Methanol gelöst und nach Zugabe von 70 ml abs. Dioxan und übliche Filtration durch ein Millipore-Filter lyophilisiert. Man erhält N-Acetyl-desmethylmuramyl-L-α-aminobutyryl-D-glutaminsäure-dibenzhydrylester als farbloses Pulver, 0,93 Mol Wasser enthaltend.

$C_{45}H_{51}N_3O_{12}$ • 0,93 $H_2O$ (842,67)

| | | | | |
|---|---|---|---|---|
| Ber. | C 64,14 | H 6,34 | N 4,99 | $H_2O$ 1,99 |
| Gef. | C 64,1 | H 6,5 | N 4,9 | $H_2O$ 2,0 |

$[\alpha]_D^{20}$ = + 11,6 ± 2,9° (c = 0,344; Methanol), $R_f$ = 0,55 (Essigsäureethylester:Methanol = 4:1) und $R_f$ = 0,77 (Chloroform:Isopropanol = 1:1).

Beispiel 13: Analog Beispiel 1 erhält man aus 0,67 g (1 mMol) N-Acetyl-desmethylmuramyl-L-valyl-D-isoglutamin-benzhydrylester und 0,367 g (3,6 mMol) Essigsäureanhydrid in 5 ml abs. Pyridin (30 Minuten, Raumtemperatur) N-Acetyl-(1α,β),4,6-tri-O-acetyl-desmethylmuramyl-L-valyl-D-isoglutamin-benzhydrylester.

$[\alpha]_D^{20}$ = + 35° ± 1° (c = 0,594; Methanol), $R_f$ = 0,92 (Chloroform: Methanol:Wasser = 70:30:5) und $R_f$ = 0,80 (Essigsäureethylester: n-Butanol:Pyridin:Essigsäure:Wasser = 42:21:21:6:10).

Das Ausgangsmaterial erhält man analog Beispiel 1 wie folgt:

Stufe 13.1: 1,60 g (3,15 mMol) N-Acetyl-desmethylmuramyl-L-valyl-D-isoglutamin, gelöst in 30 ml Methanol, werden mit überschüssigem Diphenyldiazomethan (2 Stunden, Raumtemperatur) verestert. Das Produkt wird durch mehrfaches Umfällen aus Methanol-Diethylether (1:8) gereinigt. Man erhält N-Acetyl-desmethylmuramyl-L-valyl-D-isoglutamin-benzhydrylester.
$[\alpha]_D^{20}$ = + 13° ± 1° (c = 1,067; Methanol), $R_f$ = 0,56 (Chloroform: Methanol:Wasser = 70:30:5), $R_f$ = 0,42 (n-Butanol:Essigsäure:Wasser = 75:7,5:21) und $R_f$ = 0,72 (Essigsäureethylester:n-Butanol:Pyridin: Essigsäure:Wasser = 42:21:21:6:10).

Beispiel 14: Eine Lösung von 1,2 g N-Acetyl-muramyl-L-valyl-D-isoglutamin-benzhydrylester in 12 ml absolutem Pyridin wird unter Rühren mit 0,75 ml Essigsäureanhydrid versetzt und 24 Stunden bei Raumtemperatur stehen gelassen. Das Reaktionsgemisch wird dann auf 40 ml Eiswasser gegossen, wobei das Produkt auskristallisiert. Die Kristalle werden abgesaugt, mit Wasser gewaschen und getrocknet. Nach Umkristallisation aus Essigsäureethylester und Diethylether erhält man N-Acetyl-1,4,6-tri-O-acetyl-muramyl-L-valyl-D-isoglutamin-benzhydrylester, $R_f$ = 0,3 (Methylenchlorid:Methanol = 5:1).

Das verwendete Ausgangsmaterial wird wie folgt hergestellt:

Stufe 14.1: Eine Lösung von 2,0 g N-Acetyl-muramyl-L-valyl-D-isoglutamin in 25 ml Methanol und 25 ml 1,2-Dimethoxyethan wird mit 1,1 g Diphenyldiazomethan versetzt und 20 Stunden bei Raumtemperatur gerührt. Das Reaktionsgemisch wird zur Trockne eingedampft und mit Diethylether extrahiert. Der Rückstand wird in Wasser suspendiert, gerührt, abfiltriert und über NaOH-Pillen getrocknet. Der erhaltene N-Acetyl-muramyl-L-valyl-D-isoglutamin-benzhydrylester schmilzt bei 185° (Zers.), $R_f$ = 0,5 (Chloroform:Methanol:Wasser = 14:6:1), $[\alpha]_D^{20}$ = + 38° (c = 0,912; Methanol).

Beispiel 15: Eine Lösung von 2,0 g N-Acetyl-muramyl-L-alanyl-D-isoglutamin-benzhydrylester in 20 ml absolutem Pyridin wird unter Rühren mit 1,7 ml Essigsäureanhydrid versetzt und 24 Stunden bei Raumtemperatur stehen gelassen. Das Reaktionsgemisch wird dann auf 50 ml Eiswasser gegossen. Das ausgeschiedene Material wird in 100 ml Essigsäureethylester aufgenommen, mit verdünnter Salzsäure und halbgesättigter Kochsalzlösung gewaschen und über Magnesiumsulfat getrocknet. Nach Eindampfen des Lösungsmittels erhält man N-Acetyl-1,4,6-tri-O-acetyl-muramyl-L-alanyl-D-isoglutamin-benzhydrylester. $R_f$ = 0,7 (Methylenchlorid:Methanol = 5:1).

Das verwendete Ausgangsmaterial wird wie folgt hergestellt:

Stufe 15.1: Eine Lösung von 1,1 g N-Acetyl-muramyl-L-alanyl-D-isoglutamin in 12,5 ml Methanol und 12,5 ml 1,2-Dimethoxyethan wird mit 0,42 g Diphenyldiazomethan versetzt und 20 Stunden bei 40°C gerührt. Nach Eindampfen des Lösungsmittels wird der Rückstand gründlich mit Diethylether und Wasser extrahiert und über NaOH-Pillen getrocknet. Man erhält N-Acetyl-muramyl-L-alanyl-D-isoglutamin-benzhydrylester in Form weisser Kristalle. $R_f$ = 0,6 (Methylenchlorid:Methanol:Wasser = 14:6:1).

Beispiel 16: 1,45 g (1,99 mMol) N-Acetyl-muramyl-L-alanyl-D-isoglutaminyl-L-alanin-benzhydrylester, gelöst in 10 ml abs. Pyridin, werden mit 0,73 g (7,15 mMol) Essigsäureanhydrid versetzt und während 35 Stunden bei Raumtemperatur stehen gelassen. Die gelbliche Lösung wird im Hochvakuum bei 30° zur Trockne eingedampft, der Rückstand mit abs. Dioxan versetzt und lyophilisiert. Das gefriergetrocknete Material wird in 10 ml Chloroform-Methanol (1:1) aufgenommen und mit 100 ml abs. Diethylether gefällt (3mal). Das so erhaltene Pulver wird in 100 ml abs. Dioxan-Wasser (1:1) gelöst, wie üblich durch ein Millipore-Filter (PTFE; 0,2 µm) filtriert und lyophilisiert. Man erhält N-Acetyl-(1α,β),4,6-tri-O-acetyl-muramyl-L-alanyl-D-isoglutaminyl-L-alanin-benzhydrylester als farbloses Pulver, 1,42 Mol Wasser und 0,7 Mol Dioxan enthaltend.

$C_{41}H_{51}N_5O_{15}$ • 0,7 $C_4H_8O_2$ • 1,42 $H_2O$

Ber. C 55,90 H 6,37 N 7,44 $H_2O$ 2,72

Gef. C 55,98 H 6,42 N 7,76 $H_2O$ 2,71

$[\alpha]_D^{20}$ = + 25,5 ± 2,1° (c = 0,475; Dimethylsulfoxid), $R_f$ = 0,39 (n-Butanol:Essigsäure:Wasser = 75:7,5:21), $R_f$ = 0,83 (Chloroform:Methanol:Wasser = 70:30:5) und $R_f$ = 0,85 (Chloroform:Methanol:Wasser:Essigsäure = 70:40:9:1).

Das Ausgangsmaterial erhält man wie folgt:

Stufe 16.1: 3,25 g (5,6 mMol) N-Acetyl-muramyl-L-alanyl-D-isoglutminyl-L-alanin werden in 50 ml Methanol gelöst und unter Rühren mit überschüssigem Diphenyldiazomethan verestert. Nach 6 Stunden wird die violette Lösung eingedampft und der gelbliche Rückstand durch Chromatographie an 400 g Kieselgel (Typ 60, Merck) im System Chloroform:Methanol:Wasser (70:30:5) gereinigt (13 ml Fraktionen). Das nach einem Vorlauf von 600 ml in den Fraktionen 42 bis 60 enthaltene Material wird gesammelt, in 100 ml Chloroform aufgenommen, durch ein Millipore-Filter (PTFE; 0,2 µm) filtriert und eingedampft. Der Rückstand wird in 150 ml abs. Dioxan aufgenommen und lyophilisiert. Man erhält N-Acetyl-muramyl-L-alanyl-D-isoglutaminyl-L-alanin-benzhydrylester als farbloses, lockeres Pulver.

$C_{35}H_{47}N_5O_{12}$ • 1,51 $H_2O$ (756,99)

Ber. C 55,54 H 6,69 N 9,25 $H_2O$ 3,59

Gef. C 55,68 H 6,39 N 9,04 $H_2O$ 3,59

$[\alpha]_D^{20}$ = + 16,3 ± 1° (c = 0,979; Dimethylsulfoxid), $R_f$ = 0,31 (n-Butanol:Essigsäure:Wasser = 70:7,5:21), $R_f$ = 0,57 (Chloroform:Methanol:Wasser = 70:30:5) und $R_f$ = 0,33 (Chloroform:Methanol:Wasser:Essigsäure = 75:27:5:0,5).

Beispiel 17: Analog Beispiel 16 erhält man aus 1,00 g (1,53 mMol) N-Acetyl-muramyl-L-alanyl-D-isoglutaminyl-L-alanin-benzylester, gelöst in 18 ml abs. Pyridin, und 0,66 g (5,97 mMol) Essigsäureanhydrid die Peracetylverbindung (6 Stunden, Raumtemperatur). Die Reinigung erfolgt durch Chromatographie an 150 g Kieselgel (Typ 60,

Merck) im System Chloroform-Methanol-Wasser (70:30:5; 5 ml Fraktionen). Man erhält N-Acetyl-(1α,β),4,6-tri-O-acetyl-muramyl-L-alanyl-D-isoglutaminyl-L-alanin-benzylester als farbloses Lyophilisat, 1,84 Mol Wasser enthaltend.

$C_{35}H_{49}N_5O_{15} \cdot 1,84\ H_2O$ (812,94)

Ber. C 51,72   H 6,56   N 8,62   $H_2O$ 4,07

Gef. C 51,5   H 6,9   N 8,3   $H_2O$ 4,1

$[\alpha]_D^{20}$ = + 38,5 ± 3,5° (c = 0,282; Dimethylformamid), $R_f$ = 0,63 (Acetonitril:Wasser = 3:1), $R_f$ = 0,39 (n-Butanol:Essigsäure:Wasser = 75:7,5:21), $R_f$ = 0,83 (Essigsäureethylester:n-Butanol:Pyridin:Essigsäure:Wasser = 42:21:21:6:10).

Beispiel 18: 1,45 g (1,5 mMol) N-Acetyl-muramyl-L-alanyl-D-isoglutaminyl-L-alanin-cholesteryl-3-ester und 0,72 g (7,1 mMol) Essigsäureanhydrid werden in 16 ml einer Mischung aus abs. Pyridin und Dimethylformamid (6:1) gelöst und während 22 Stunden bei Raumtemperatur stehen gelassen. Die klare Lösung wird im Hochvakuum bei 30° auf ca. 4 ml eingeengt, der entstehende gallertige Rückstand mit 50 ml Essigsäureethylester verrieben und der Ueberstand abdekantiert (4mal). Der feste Rückstand wird in 5 ml Dimethylformamid aufgenommen, 50 ml abs. Dioxan zugefügt, das Ganze durch ein Millipore-Filter (PTFE; 0,2 µm) filtriert und lyophilisiert. Man erhält N-Acetyl-(1α,β),4,6-tri-O-acetyl-muramyl-L-alanyl-D-isoglutaminyl-L-alanin-cholesteryl-3-ester als farbloses Pulver, 0,62 Mol Wasser enthaltend.

$C_{55}H_{87}N_5O_{15} \cdot 0,62\ H_2O$ (1069,49)

Ber. C 61,77   H 8,33   N 6,55   $H_2O$ 1,04

Gef. C 61,8   H 8,4   N 6,8   $H_2O$ 1,0

$[\alpha]_D^{20}$ = + 7,7 ± 1° (c = 0,977; Dimethylsulfoxid), $R_f$ = 0,41 (n-Butanol:Essigsäure:Wasser = 75:7,5:21), $R_f$ = 0,87 (Essigsäureethylester:n-Butanol:Pyridin:Essigsäure:Wasser = 42:21:21:6:10).

Beispiel 19: Analog Beispiel 1 erhält man aus N-Acetyl-muramyl-L-alanyl-D-glutamin-pivaloyloxymethylester und Essigsäureanhydrid in absolutem Pyridin N-Acetyl-1,4,6-tri-O-acetyl-muramyl-L-alanyl-D-glutamin-pivaloyloxymethylester.

Das Ausgangsmaterial erhält man folgendermassen:

Stufe 19.1: 3,0 g (8,54 mMol) N-Benzyloxycarbonyl-L-alanyl-D-glutamin werden in einem Gemisch aus 300 ml Tetrahydrofuran und 20 ml Wasser gelöst. Zu dieser Lösung gibt man 1,39 g (4,27 mMol) Cäsiumcarbonat (purum, Fluka), gelöst in 7 ml Wasser, und lässt das Ganze 1/2 Stunde bei Raumtemperatur stehen. Anschliessend wird im Hochvakuum bei 30° eingedampft und der so erhaltene Rückstand nacheinander mit 250 ml Methanol und zweimal je 250 ml Dimethylformamid im Hochvakuum bei 40° abgedampft.

Das so erhaltene Cäsiumsalz des N-Benzyloxycarbonyl-L-alanyl-D-glutamins wird in 300 ml Dimethylformamid suspendiert. Zu dieser Suspension werden bei Raumtemperatur 2,57 g (2,48 ml; 17,08 mMol) Pivalinsäurechlormethylester (purum, Fluka) und 2,56 g (17,08 mMol) Natriumiodid gegeben. Das so erhaltene Gemisch wird 42 Stunden bei Raumtemperatur gerührt. Anschliessend wird im Hochvakuum bei 40° eingedampft. Der so erhaltene gelbe, kristalline Rückstand wird in 500 ml Essigsäureethylester aufgenommen (Suspension) und dreimal mit je 150 ml Wasser gewaschen. Die klaren Essigesterphasen werden vereinigt, über Natriumsulfat getrocknet und im Vakuum bei 30° eingedampft. Der Rückstand kristallisiert aus Essigsäureethylester:n-Pentan = 1:15 (20:300 ml). Man erhält nach zweimaliger Umkristallisation N-Benzyloxycarbonyl-L-alanyl-D-glutamin-pivaloyloxymethylester in Form farbloser Kristalle vom Smp. 109-110°.

$C_{22}H_{31}N_3O_8$ (465,50)

| | | | |
|---|---|---|---|
| Ber. C 56,76 | H 6,71 | N 9,03 | O 27,49 |
| Gef. C 56,42 | H 6,69 | N 8,82 | O 27,73 |

$[\alpha]_D^{20}$ = + 5,5 ± 0,1° (c = 0,973; Methanol), $R_f$ = 0,62 (Methylenchlorid:Methanol = 5:1), $R_f$ = 0,86 (Methylenchlorid:Methanol: Wasser = 70:30:5).

Stufe 19.2:
Eine Lösung von 6,7 g (14,4 mMol) N-Benzyloxycarbonyl-L-alanyl-D-glutamin-pivaloyloxymethylester in 200 ml Dimethoxyethan wird bei konstantem pH-Wert von 5,5 (Titration mit 1N Salzsäure) mit 1,0 g 10%iger Palladiumkohle als Katalysator 40 Minuten bei Raumtemperatur und Normaldruck hydriert.

Dann wird vom Katalysator abfiltriert und das Filtrat im Hochvakuum bei 30° eingedampft. Der so erhaltene Rückstand wird in 30 ml Diethylether suspendiert. Die so erhaltenen farblosen Kristalle werden abgesaugt und mit Diethylether nachgewaschen. Man erhält L-Alanyl-D-glutamin-pivaloyloxymethylester-hydrochlorid in Form farbloser Kristalle vom Smp. 110-111° (Zers.).
$C_{14}H_{26}ClN_3O_6$ (367,83)

| | | | | | |
|---|---|---|---|---|---|
| Ber. | C 45,72 | H 7,13 | Cl 9,64 | N 11,42 | O 26,10 |
| Gef. | C 45,51 | H 7,36 | Cl 9,34 | N 11,47 | O 26,40 |

$[\alpha]_D^{20}$ = + 25,5 ± 0,1° (c = 1,128; Methanol), $R_f$ = 0,07 (Methylenchlorid:Methanol = 5:1), $R_f$ = 0,37 (Methylenchlorid:Methanol: Wasser = 70:30:5), $R_f$ = 0,59 (Methylenchlorid:Methanol: Wasser = 5:5:1).

Stufe 19.3: 5,18 g (13,0 mMol; 2,52 mMol/g) N-Acetyl-4-O,6-O-isopropyliden-muraminsäure-natriumsalz werden in 150 ml Dimethylformamid suspendiert. Dann werden bei Raumtemperatur 4,8 g (13,0 mMol) L-Alanyl-D-glutamin-pivaloyloxymethylester-hydrochlorid, 2,95 g (14,3 mMol) Dicyclohexylcarbodiimid und 1,64 g (14,3 mMol) N-Hydroxy-succinimid zugegeben. Die so erhaltene, leicht gelbe Suspension wird 22 Stunden bei Raumtemperatur gerührt. Danach wird von der Hauptmenge des entstandenen Dicyclohexylharnstoffes abgesaugt und das so erhaltene Filtrat im Hochvakuum bei 40° eingedampft. Das zurückbleibende gelbe Oel wird in 20 ml Methanol aufgenommen, eine weitere Fraktion Dicyclohexylharnstoff abgesaugt und erneut im Hochvakuum eingedampft.

Das so erhaltene Rohprodukt, das auch noch wenig Dicyclohexylharnstoff enthält, wird durch Säulenchromatographie an 1000 g Kieselgel (Typ 60, reinst, Merck; 0,063-0,2 mm) im System Methylenchlorid: Methanol = 9:1 gereinigt (10 ml Fraktionen).

Die Fraktionen 380-1210 werden vereinigt, wobei ab Fraktion 850 mit dem System Methylenchlorid:Methanol:Wasser = 70:30:5 eluiert wird.

Nach Eindampfen der Eluate im Hochvakuum bei 30° erhält man ein Gemisch das hauptsächlich aus N-Acetyl-4-O,6-O-isopropyliden-muramyl-L-alanyl-D-glutamin-pivaloyloxymethylester und etwas N-Acetyl-muramyl-L-alanyl-D-glutamin-pivaloyloxymethylester besteht, in Form eines schwach gelben Schaumes, der ohne weitere Reinigung verarbeitet wird.
$R_f$ = 0,33 [N-Acetyl-muramyl-L-alanyl-D-glutamin-pivaloyloxymethylester] und 0,66 [N-Acetyl-4-O,6-O-isopropyliden-muramyl-L-alanyl-D-glutamin-pivaloyloxymethylester] (Methylenchlorid:Methanol: Wasser = 70:30:5).

<u>Stufe 19.4:</u> Eine Lösung von 5,5 g (ca. 8,5 mMol) rohem N-Acetyl-4-O,6-O-isopropyliden-muramyl-L-alanyl-D-glutamin-pivaloyloxymethylester in 100 ml Eisessig-Wasser (3:2) wird 4,5 Stunden bei Raumtemperatur gerührt. Danach wird die leicht gelbe Lösung im Hochvakuum bei 40° eingedampft und der so erhaltene Rückstand durch Säulenchromatographie an 500 g Kieselgel (Typ 60, reinst, Merck; 0,063-0,2 mm) im System Methylenchlorid:Methanol:Wasser (70:30:5) gereinigt (10 ml Fraktionen). Die Fraktionen 52-65 werden vereinigt und im Hochvakuum bei 30° eingedampft. Man erhält N-Acetyl-muramyl-L-alanyl-D-glutamin-pivaloyloxymethylester, der in 60 ml bidestilliertem Wasser gelöst wird. Die Lösung wird durch ein Millipore-filter (Nalgene® S; 0,2 µm) filtriert und anschliessend im Hochvakuum lyophilisiert. Man erhält N-Acetyl-muramyl-L-alanyl-D-glutamin-pivaloyloxymethylester als farbloses Pulver, das noch 1,21 Mol Wasser enthält.

$C_{25}H_{42}N_4O_{13} \cdot 1,21\ H_2O$ (628,50)

| | | | | | |
|---|---|---|---|---|---|
| Ber. | C 47,78 | H 7,16 | N 8,91 | O 36,18 | $H_2O$ 3,48 |
| Gef. | C 48,09 | H 7,15 | N 9,02 | O 36,01 | $H_2O$ 3,48 |

$[\alpha]_D^{20} = + 39,5 \pm 0,1°$ (c = 0,443; Wasser),

$R_f$ = 0,61 (Methylenchlorid:Methanol:Wasser = 70:30:5).

Beispiel 20: Nach den in dieser Anmeldung beschriebenen Verfahren erhält man die folgenden Verbindungen:

a) N-Acetyl-1,4,6-tri-O-acetyl-muramyl-L-N-methyl-alanyl-D-isoglu-amin-benzhydrylester,

b) N-Acetyl-1,4,6-tri-O-acetyl-muramyl-α-amino-isobutyryl-D-isoglutamin-benzhydrylester und

c) N-Acetyl-1,4,6-tri-O-acetyl-desmethylmuramyl-L-alanyl-D-γ-methoxycarbonyl-isoglutamin-benzhydrylester.

Beispiel 21: Weibliche MF-2f SPF-Mäuse mit einem Körpergewicht von 14-16 g werden unter leichter Narkose mit einem Gemisch aus gleichen Teilen Diethylether, Ethanol und Chloroform mit letalen Dosen (ungefähr eine $LD_{80-90}$; 1-4 plaque forming units [PFU]) in Form von je 0,05 ml einer Suspension von Influenza A/Texas/1/77 (Virus A) oder von Influenza B/Hong-Kong 15/72 (Virus B) Viren (Maus-adaptierte Stämme) intranasal infiziert.

Jeder Maus aus Gruppen von jeweils 10 dieser Mäuse wird zum unten angegebenen Zeitpunkt [Tage] bezogen auf den Tag der Infektion einmal (Einzeldosis) die in Tabelle 1 genannte Menge der jeweiligen Wirksubstanz in 0,05 bzw. in 0,2 ml einer 0,005 Gew.%igen Lösung von Carboxymethylcellulose-natriumsalz in doppelt destilliertem, pyrogenfreiem Wasser im Falle von intranasaler bzw. oraler Applikation auf die in Tabelle 1 genannte Art appliziert.

Jeweils 20 der obengenannten infizierten Mäuse dienen zur Kontrolle, d.h. sie erhalten ein Placebo (0,005 Gew.%ige Lösung von Carboxymethylcellulose-natriumsalz).

Die intranasale Applikation der Wirksubstanz wird unter leichter Narkose mit einem Gemisch aus gleichen Teilen Diethylether, Ethanol und Chloroform durchgeführt.

Verbindung I = 1,4,6-Tri-O-acetyl-N-propionyl-desmethylmuramyl-L-alanyl-D-isoglutamin-benzhydrylester.

0192611

Tabelle 1:

| Virus | Wirksubstanz | Applikationsart | Applikationszeit [Tage] | Prozentsatz der 23 Tage nach der Infektion noch lebenden Mäuse in Abhängigkeit von der Wirksubstanzmenge [mg/kg], statistische Signifikanz * P ≤ 0,05, **P ≤ 0,01 (Vierfelder-Test), n.t. = nicht geprüft | | | | | | |
|---|---|---|---|---|---|---|---|---|---|---|
| | | | | 10 | 1 | 0,1 | 0,01 | 0,001 | 0,0001 | 0=Kontrolle |
| A | I | oral | +7 | n.t. | 100** | 70(*) | 100** | n.t. | n.t. | 30 |
| | | oral | +7 | 80** | 70** | 90** | n.t. | n.t. | n.t. | 20 |
| | | intranasal | -7 | n.t. | 77* | 100** | 80** | n.t. | n.t. | 20 |
| B | I | oral | +7 | n.t. | n.t. | n.t. | 50** | 0 | 10 | 0 |
| | | intranasal | -7 | n.t. | n.t. | n.t. | 100** | 100** | 90* | 40 |

0192611

Beispiel 22: Nicht-wässrige Einzeldosis zur Nasenapplikation

Zusammensetzung:

| | |
|---|---|
| 1,4,6-Tri-O-acetyl-N-propionyl-desmethylmuramyl-L-alanyl-D-isoglutamin-benzhydrylester | 0,03 mg |
| Miglyol 812 | 30,00 mg |

Herstellung:

0,03 mg des Wirkstoffs werden unter aseptischen Bedingungen in 29,97 mg Miglyol gelöst.

Diese Lösung wird in einen handelsüblichen Einmalnasenapplikator abgefüllt, der vor der Anwendung auf eine Treibstoffdose aufgesetzt wird.

Beispiel 23: Nasentropfen

Zusammensetzung:

| | | |
|---|---|---|
| 1,4,6-Tri-O-acetyl-N-propionyl-desmethylmuramyl-L-alanyl-D-isoglutamin-benzhydrylester | 0,15 mg | 0,10 mg |
| Thiomersal | 0,02 mg | -- |
| Natriummonohydrogenphosphat•$2H_2O$ | 0,30 mg | 0,30 mg |
| Natriumdihydrogenphosphat•$12H_2O$ | 10,10 mg | 10,10 mg |
| Benzalkoniumchlorid | -- | 0,10 mg |
| Ethylendiamintetraessigsäure-dinatriumsalz (EDTA) | 0,50 mg | 0,50 mg |
| Natriumchlorid | 3,70 mg | 4,50 mg |
| Entmineralisiertes Wasser | 988,30 mg | 987,60 mg |

Herstellung:

In einem Teil der obengenannten Menge von entmineralisiertem Wasser werden unter Rühren Natriumdihydrogenphosphat, Dinatriumhydrogen phosphat, Natriumchlorid, Thiomersal und EDTA-Dinatriumsalz bei Raumtemperatur gelöst.

In dieser Lösung löst man anschliessend den Wirkstoff auf und ergänzt mit dem restlichen entmineralisierten Wasser.

Die Lösung oder ein Teil oder ein Vielfaches davon wird durch einen Membranfilter filtriert und in gereinigte Behälter abgefüllt. Geeignete Behälter sind z.B.

a) Glas- oder Kunststoffbehälter (à 5 ml oder 10 ml), welche eine Pipette aus Glas oder Kunststoff mit einem elastomeren Pipettensauger besitzen,
b) Knautschflaschen aus Kunststoff mit einem Steigrohr und einem Sprühkopf aus Kunststoff,
c) Einzeldosisbehälter aus Kunststoff (Inhalt 2-3 Tropfen) oder
d) Glas- oder Kunststoffflaschen, die mit einem normierten Pumpdosierspray aus Kunststoff versehen sind (ohne Treibgas).

Beispiel 24: Nasensalbe

Zusammensetzung:

| | |
|---|---|
| 1,4,6-Tri-O-acetyl-N-propionyl-desmethylmuramyl-L-alanyl-D-isoglutaminbenzhydrylester | 0,03 g |
| Paraffinöl dickflüssig | 20,00 g |
| weisses Vaselin | 30,00 g |
| Wollfett, wasserfrei | 40,00 g |
| entmineralisiertes Wasser | 19,97 g |

Herstellung:

Die Fettphase, bestehend aus Paraffinöl, Vaselin und Wollfett, wird zusammengeschmolzen. Die wässrige Lösung des Wirkstoffs wird bei ca. 50°C in die Fettphase eingearbeitet.

Beispiel 25: Herstellung von 1000 Tabletten, enthaltend 0,5 % Wirkstoff

Zusammensetzung pro 1000 Tabletten:

| | |
|---|---|
| 1,4,6-Tri-O-acetyl-N-propionyl-desmethylmuramyl-L-alanyl-D-isoglutamin-benzhydrylester | 0,5 g |
| Lactose gemahlen | 43,0 g |
| Maisstärke | 52,0 g |
| Pharmacoat 603® (Hydroxypropylmethyl-cellulose, enthaltend 28-30 % Methoxyl-gruppen, geliefert von Shinetsu Chemical Company, Toio, Japan) | 3,0 g |
| Aerosil® (kolloidales Siliziumdioxid, geliefert von Degussa, Frankfurt, Bunderepublik Deutschland) | 1,0 g |
| Magnesiumstearat | 0,5 g |

Herstellung:

Der Wirkstoff und 15 g Lactose werden vorgemischt. Die so erhaltene Vormischung wird mit 28 g Lactose und 47 g Maisstärke zusammengemischt. Mit der so erhaltenen Mischung und einer wässerigen Lösung des Pharmacoat wird eine granulierfertige Masse hergestellt, die granuliert, getrocknet und gemahlen wird. Hierzu mischt man 5 g Maisstärke, Aerosil und Magnesiumstearat und komprimiert zu 1000 Tabletten mit einem Gewicht von je 100 mg.

Die Presslinge können auf an sich bekannte Weise magensaftresistent lackiert werden.

Beispiel 26: Nach den in dieser Anmeldung beschriebenen Verfahren erhält man die folgenden Verbindungen:

N-Acetyl-1,4,6-tri-O-acetyl-muramyl-L-alanyl-D-isoglutaminyl-L-α-([2-benzyloxy-carbonylamino-ethyl]-sulfonyl-methyl)-glycin-benzylester,

N-Acetyl-1,4,6-tri-O-acetyl-muramyl-L-alanyl-D-isoglutaminyl-L-O-acetyl-serin-benzylester,
1,4,6-Tri-O-acetyl-N-benzoyl-desmethylmuramyl-L-alanyl-D-glutaminsäure-dicholinester,
N-Propionyl-1,4,6-tri-O-propionyl-desmethylmuramyl-L-alanyl-D-isoglutamin-benzhydrylester,
1,4,6-Tri-O-acetyl-N-propionyl-desmethylmuramyl-L-alanyl-D-glutaminsäure-α-n-butylester-γ-benzylester,
1,4,6-Tri-O-acetyl-N-propionyl-desmethylmuramyl-L-alanyl-D-glutaminsäure-dibenzylester,
N-Acetyl-1,4,6-tri-O-propionyl-muramyl-L-α-aminobutyryl-D-isoglutamin-benzhydrylester,
1,4,6-Tri-O-acetyl-N-benzoyl-desmethylmuramyl-L-alanyl-D-isoglutaminbenzylester und
1,4,6-Tri-O-acetyl-N-propionyl-desmethylmuramyl-L-alanyl-D-glutaminsäure-dipivaloyloxymethylester.

Beispiel 27: Analog Beispiel 1 erhält man aus 250 mg (circa 0,098 mMol) rohem 4,6-Di-O-acetyl-N-propionyl-desmethylmuramyl-L-alanyl-D-glutaminsäure-dipivaloyloxymethylester (α,β-Gemisch) und 2,0 ml (21,0 mMol) Essigsäureanhydrid in 10 ml absolutem Pyridin (18 Stunden, Raumtemperatur) 1,4,6-Tri-O-acetyl-N-propionyl-desmethylmuramyl-L-alanyl-D-glutaminsäure-dipivaloyloxymethylester (α,β-Gemisch);
$R_f$ = 0,43 (Chloroform:Ethanol = 95:5),
$R_f$ = 0,56 (Chloroform:Methanol = 9:1),
$R_f$ = 0,72 (Chloroform:Methanol = 4:1).

Das Ausgangsmaterial erhält man wie folgt:
Stufe 27.1: Zu einer Lösung von 9,78 g (34,1 mMol) N-tert.-Butyloxycarbonyl-L-alanin-N-hydroxy-succinimidester in 200 ml absolutem N,N-Dimethylformamid gibt man bei Raumtemperatur unter Rühren 15,9 g (34,1 mMol) D-Glutaminsäure-($C_\alpha$)-pivaloyloxymethylester-($C_\gamma$)-benzylester-tosylat und 3,75 ml (34,1 mMol) N-Methylmorpholin. Nach 18 Stunden Rühren bei Raumtemperatur wird im Vakuum bei 40° eingedampft. Das so erhaltene Rohprodukt (gelbes Oel) wird durch

Säulenchromatographie an 800 g Kieselgel (Typ 60, reinst, Merck; 0,063-0,2 mm) im System Methylenchlorid-Essigsäureethylester (85:15) gereinigt (10 ml Fraktionen). Die Fraktionen 126-230 werden vereinigt und im Vakuum bei 30° eingedampft. Man erhält N-tert.-Butyloxycarbonyl-L-alanyl-D-glutaminsäure-($C_\alpha$)-pivaloyloxymethylester-($C_\gamma$)-benzylester als gelbliches Oel;
$[\alpha]_D^{20}$ = -10,6±0,1° (c = 1,035; Methylenchlorid),
$R_f$ = 0,29 (Methylenchlorid:Essigsäureethylester = 85:15),
$R_f$ = 0,73 (Methylenchlorid:Methanol = 9:1),
$R_f$ = 0,78 (Methylenchlorid:Methanol = 5:1).

Stufe 27.2: Zu einer Lösung von 4,0 g (7,65 mMol) N-tert.-Butyloxycarbonyl-L-alanyl-D-glutaminsäure-($C_\alpha$)-pivaloyloxymethylester-($C_\gamma$)-benzylester in 50 ml absolutem Essigsäureethylester gibt man bei 0° 100 ml ca. 5 N Salzsäure in Essigsäureethylester und rührt 1 Stunde bei 0°. Danach wird im Hochvakuum bei 30° eingedampft und der so erhaltene Rückstand mehrmals in Essigsäureethylester aufgenommen und erneut eingedampft. Man erhält einen farblosen Schaum, der in 50 ml Essigsäureethylester gelöst wird. Nach Zugabe von 250 ml Pentan und Kühlung auf -10° erhält man L-Alanyl-D-glutaminsäure-($C_\alpha$)-pivaloyloxymethylester-($C_\gamma$)-benzylester-hydrochlorid als farblose Kristalle vom Smp. 73-74°, die noch 0,63 Mol Wasser enthalten;
$[\alpha]_D^{20}$ = +23,5±0,1° (c = 1,150; Methanol),
$R_f$ = 0,20 (Methylenchlorid:Methanol = 9:1),
$R_f$ = 0,49 (Methylenchlorid:Methanol = 5:1),
$R_f$ = 0,87 (Methylenchlorid:Methanol:Wasser = 70:30:5).

Stufe 27.3: Zu einer Suspension von 2,99 g (~ 5,0 mMol, enthält noch Natriumchlorid; 1,673 mMol/g) 1-α-O-Benzyl-4,6-O,O-isopropyliden-N-propionyl-desmethylmuraminsäure-natriumsalz in 50 ml absolutem N,N-Dimethylformamid werden bei Raumtemperatur unter Rühren nacheinander 1,34 g (6,5 mMol) N,N-Dicyclohexylcarbodiimid, 1,03 g (6,5 mMol) 1-Hydroxybenzotriazol und 2,35 g (5,0 mMol L-Alanyl-D-glutaminsäure-($C_\alpha$)-pivaloyloxymethylester-($C_\gamma$)-benzylester-hydrochlorid gegeben und dann 22 Stunden bei Raumtemperatur weiter gerührt. Danach wird die Suspension filtriert (N,N-Dicyclohexylharnstoff) und das Filtrat im Hochvakuum bei 40° eingedampft. Den Rückstand, einen gelblichen Schaum, nimmt man in 100 ml Essigsäureethylester auf und wäscht die so erhaltene Lösung nacheinander mit je 50 ml 2 N Zitronensäure, 10%iger Natriumhydrogencarbonatlösung und Wasser. Die Essigesterphasen werden vereinigt, über Natriumsulfat getrocknet und im Vakuum eingedampft. Das so erhaltene Rohprodukt wird durch Säulenchromatographie an 400 g Kieselgel (Typ 60, reinst, Merck; 0,063-0,2 mm) zunächst mit Chloroform (Vorlauf 2 Liter), dann im System Chloroform-Ethanol (95:5) gereinigt (10 ml Fraktionen). Die Fraktionen 93-122 werden vereinigt und im Vakuum eingedampft. Man erhält noch schwach verunreinigten 1-α-O-Benzyl-4,6-O,O-isopropyliden-N-propionyl-desmethylmuramyl-L-alanyl-D-glutaminsäure-($C_\alpha$)-pivaloyloxymethylester-($C_\gamma$)-benzylester als farblosen Schaum, der ohne weitere Reinigung verarbeitet wird;
$R_f$ = 0,55 (Chloroform:Ethanol = 9:1),
$R_f$ = 0,63 (Chloroform:Methanol = 9:1).

<u>Stufe 27.4:</u> Zu einer Lösung von 830 mg (1,0 mMol) 1-α-O-Benzyl-4,6-O,O-isopropyliden-N-propionyl-desmethylmuramyl-L-alanyl-D-glutaminsäure-($C_\alpha$)-pivaloyloxymethylester-($C_\gamma$)-benzylester in 28,5 ml absolutem Methylenchlorid gibt man bei 0° 1,5 ml Trifluoressigsäure und rührt das Ganze 1,5 Stunden bei 0°. Danach wird die so erhaltene farblose Lösung im Hochvakuum bei 40° zur Trockne eingedampft. Der Rückstand kristallisiert aus Chloroform-Methanol-Diethylether (5:1:70). Man erhält 1-α-O-Benzyl-N-propionyl-desmethylmuramyl-L-alanyl-D-glutaminsäure-($C_\alpha$)-pivaloyloxymethylester-($C_\gamma$)-benzylester als farblose Kristalle; Smp. 148-149°;
$R_f$ = 0,33 (Chloroform:Methanol = 9:1),
$R_f$ = 0,67 (Chloroform:Methanol = 4:1),
$R_f$ = 0,90 (Chloroform:Methanol:Wasser = 70:30:5).

<u>Stufe 27.5:</u> 400 mg (0,5 mMol) 1-α-O-Benzyl-N-propionyl-desmethylmuramyl-L-alanyl-D-glutaminsäure-($C_\alpha$)-pivaloyloxymethylester-($C_\gamma$)-benzylester werden in 80 ml Tetrahydrofuran-Methanol (4:1) mit 200 mg 10%iger Palladiumkohle als Katalysator bei Raumtemperatur unter Normaldruck hydriert. Nach beendeter Hydrierung (1 Stunde)

wird vom Katalysator abfiltriert und das Filtrat im Hochvakuum eingedampft. Der Rückstand, ein farbloser Schaum, wird in einem Gemisch aus 30 ml Tetrahydrofuran und 1 ml Wasser gelöst und die so erhaltene Lösung bei Raumtemperatur mit einer Lösung von 96 mg (0,25 mMol) Cäsiumcarbonat in 0,38 ml Wasser versetzt. Nach einer halben Stunde Stehen bei Raumtemperatur wird die farblose Lösung im Vakuum eingedampft. Der Rückstand wird anschliessend zweimal mit je 100 ml Methanol und einmal mit 100 ml N,N-Dimethylformamid versetzt und erneut eingedampft. Man erhält noch schwach verunreinigtes 1-α-O-Benzyl-N-propionyl-desmethylmuramyl-L-alanyl-D-glutaminsäure-($C_\alpha$)-pivaloyloxymethylester-cäsiumsalz als farblosen Schaum, der ohne weitere Reinigung verarbeitet wird;

$R_f$ = 0,15 (Chloroform:Methanol = 4:1),
$R_f$ = 0,35 (Chloroform:Methanol = 7:3),
$R_f$ = 0,45 (Chloroform:Methanol:Wasser = 70:30:5).

Stufe 27.6: Zu einer Lösung von 420 mg (0,5 mMol) 1-α-O-Benzyl-N-propionyl-desmethylmuramyl-L-alanyl-D-glutaminsäure-($C_\alpha$)-pivaloyloxymethylester-cäsiumsalz in 15 ml N,N-Dimethylformamid gibt man bei Raumtemperatur unter Rühren 150 mg (145 µl, 1,0 mMol) Pivalinsäurechlormethylester und 150 mg (1,0 mMol) Natriumiodid. Das so erhaltene Gemisch wird 18 Stunden bei Raumtemperatur gerührt, dann werden nochmals 145 µl (1,0 mMol) Pivalinsäurechlormethylester und 150 mg (1,0 mMol) Natriumiodid zugegeben und weitere 48 Stunden gerührt. Anschliessend wird das Gemisch im Hochvakuum bei 30° eingedampft. Das Rohprodukt reinigt man durch zweifache Säulenchromatographie an 200 bzw. 250 g Kieselgel (Typ 60, reinst, Merck; 0,063-0,2 mm) im System Chloroform-Methanol (9:1) bzw. Chloroform:Methanol (95:5). Die das gewünschte Produkt enthaltenden Fraktionen werden jeweils vereinigt und im Vakuum eingedampft. Man erhält 1-α-O-Benzyl-N-propionyl-desmethylmuramyl-L-alanyl-D-glutaminsäure-dipivaloyloxymethylester;

$R_f$ = 0,25 (Chloroform:Methanol = 9:1),
$R_f$ = 0,35 (Chloroform:Ethanol = 9:1),
$R_f$ = 0,70 (Chloroform:Methanol = 4:1).

Stufe 27.7: Analog Beispiel 1 erhält man aus 80 mg (0,098 mMol) 1-α-O-Benzyl-N-propionyl-desmethylmuramyl-L-alanyl-D-glutaminsäure-dipivaloyloxymethylester und 0,8 ml (8,4 mMol) Essigsäureanhydrid in 8 ml absolutem Pyridin (18 Stunden Raumtemperatur) 4,6-Di-O-acetyl-1-α-O-benzyl-N-propionyl-desmethylmuramyl-L-alanyl-D -glutaminsäure-dipivaloyloxymethylester;

$R_f$ = 0,50 (Chloroform:Ethanol = 95:5),
$R_f$ = 0,65 (Chloroform:Ethanol = 9:1),
$R_f$ = 0,77 (Chloroform:Methanol = 9:1).

Stufe 27.8: 140 mg (0,098 mMol 4,6-Di-O-acetyl-1-α-O-benzyl-N-propionyl-desmethylmuramyl-L-alanyl-D-glutaminsäure-dipivaloyloxymethylester werden in 80 ml Dimethoxyethan-Wasser (9:1) mit Palladiumkohle (Degussa, E 101 N) als Katalysator 20 Stunden bei Raumtemperatur unter Normaldruck hydriert. Danach wird vom Katalysator abfiltiert und das Filtrat im Hochvakuum bei 30° eingedampft. Man erhält rohen 4,6-Di-O-acetyl-N-propionyl-desmethylmuramyl-L-alanyl-D-glutaminsäure-dipivaloyloxymethylester (α,β-Gemisch), der ohne weitere Reinigung verarbeitet wird;

$R_f$ = 0,38 (Chloroform:Methanol = 9:1),
$R_f$ = 0,64 (Chloroform:Methanol:Wasser = 70:30:5),
$R_f$ = 0,68 (Chloroform:Methanol = 4:1).

Beispiel 28: Analog Beispiel 1 erhält man aus 174,0 mg (0,25 mMol) N-Propionyl-desmethylmuramyl-L-alanyl-D-isoglutaminyl-L-alanin-benzoyloxymethylester, der noch Spuren von N-Methyl-morpholin-hydrochlorid enthält, in 1,7 ml absolutem Pyridin mit 0,1 ml (1,04 mMol) Essigsäureanhydrid unter Zusatz von wenig 4-Dimethylamino-pyridin 1,4,6-Tri-O-acetyl-N-propionyl-desmethylmuramyl-L-alanyl-D-iso-glutaminyl-L-alanin-benzoyloxymethylester (α,β-Gemisch) als farblose Kristalle;

Smp. 177-178° (aus Methylenchlorid:Diethylether = 1:1),
$R_f$ = 0,45 (Chloroform:Methanol = 4:1),
$R_f$ = 0,64 (Chloroform:Methanol = 7:3).

Das Ausgangsmaterial erhält man wie folgt:

Stufe 28.1: Eine Lösung von 18,9 g (0,1 Mol) N-tert.-Butyloxycarbonyl-L-alanin in 50 ml N,N-Dimethylacetamid wird mit 14,9 ml (0,1 Mol) 1,8-Diazabicyclo[5.4.0]undec-7-en und 36,2 ml (0,45 Mol) Diiodmethan versetzt und 7 Stunden bei 22° gerührt. Man dampft die Lösung im Hochvakuum ein und gibt zum Rückstand eine Suspension von 36,6 g (0,3 Mol) Benzoesäure, 6,1 g (0,05 Mol) 4-Dimethylaminopyridin und 37,3 ml 1,8-Diazabicyclo[5.4.0]undec-7-en in 75 ml N,N-Dimethylacetamid. Die nach 40 Minuten entstandene klare Lösung wird 16 Stunden bei 22° stehengelassen. Man schüttelt den Ansatz mit einem Gemisch aus 0,3molarer Dikaliumhydrogenphosphat-Lösung und Essigsäureethylester, trennt die organische Schicht ab, wäscht sie mit weiterer Phosphat-Lösung, trocknet über Natriumsulfat und dampft ein. Digerieren des Rückstandes mit Pentan, dann Pentan-Diethylether (3:1) gibt N-tert.-Butyloxycarbonyl-L-alanin-benzoyloxymethylester; $R_f$ = 0,68 (Chloroform:Essigsäureethylester = 9:1).

Stufe 28.2: 3,72 g (11,5 mMol) N-tert.-Butyloxycarbonyl-L-alanin-benzoyloxy-methylester werden bei 0° in 100 ml einer 5molaren Chlorwasserstoff-Lösung in Essigsäureethylester klar gelöst und 2 Stunden bei 0° stehengelassen. Bei 0° wird die Lösung zu 200 ml Diethylether-Pentan (1:1) gegeben und nach 20 Minuten Rühren bei 0° das ausgefallene gelartige Rohprodukt abfiltiert. Es wird 2 Stunden bei 22° in 360 ml Essigsäureethylester-Diethylether-Pentan (1:1:1) verrührt, dann abfiltriert. Man erhält L-Alanin-benzoyloxymethylester-hydrochlorid; Smp. 99-100°.

Stufe 28.3: Zu einer Lösung von 506 mg (1,0 mMol) N-Propionyl-desmethylmuramyl-L-alanyl-D-isoglutamin, das 0,77 Mol Wasser enthält, in 10 ml absolutem N,N-Dimethylformamid gibt man bei Raumtemperatur unter Rühren 268 mg (1,3 mMol) N,N-Dicyclohexylcarbodiimid und 207 mg (1,3 mMol) 1-Hydroxy-benzotriazol. Nach 2 Stunden wird die so erhaltene farblose Suspension nacheinander mit 269 mg (1,0 mMol) L-Alanin-benzoyloxymethylester-hydrochlorid und 110 µl (1,0 mMol) N-Methylmorpholin versetzt und danach bei Raumtemperatur weitergerührt. Nach 22 Stunden wird von ausgefallenem N,N-Dicyclohexylharnstoff abfiltiert und das Filtrat im Hochvakuum bei 30° eingedampft. Der so erhaltene, schwach gelbgefärbte Rückstand (Schaum) wird in 25 ml Essigsäureethylester suspendiert und diese Suspension 1 Stunde bei Raumtemperatur gerührt. Das in Form farbloser Kristalle erhaltene Rohprodukt wird durch Säulenchromatographie an 150 g Kieselgel (Typ 60, reinst, Merck; 0,063-0,2 mm) im System Chloroform-Methanol-Wasser (70:30:5) weiter gereinigt (5 ml Fraktionen). Die Fraktionen 25-35 werden vereinigt und im Hochvakuum eingedampft. Man erhält fast reinen N-Propionyl-desmethylmuramyl-L-alanyl-D-isoglutaminyl-L-alanin-benzoyloxymethylester (α,β-Gemisch) als farblosen Schaum, der noch Spuren von N-Methyl-morpholin-hydrochlorid enthält und ohne weitere Reinigung verarbeitet wird;

$R_f$ = 0,36 (Chloroform:Methanol = 7:3),

$R_f$ = 0,41 (Chloroform:Methanol:Wasser = 70:30:5),

$R_f$ = 0,74 (Acetonitril:Wasser = 3:1).

Beispiel 29: 1,95 g (2,18 mMol) N-Acetyl-muramyl-L-alanyl-D-isoglutaminyl-L-($C^\alpha$)-([2-benzyloxycarbonylamino-ethyl]-sulfonyl-methyl)-glycin-benzylester werden in 40 ml abs. Pyridin gelöst und mit der 30fachen molaren Menge an Essigsäureanhydrid peracetyliert (30 Stunden, RT). Die gallertige Masse wird mit wenig Wasser in Lösung gebracht, das Ganze am Rotationsverdampfer im Hochvakuum bei 30° auf ein kleines Volumen eingeengt und nach Zugabe von abs. Dioxan lyophilisiert. Das Rohprodukt wird an Kieselgel (Korngrösse 0,04-0,063 mm) erst mit Chloroform, dann mit Chloroform-Methanol-Gemischen (98:2 bis 1:1) einer Flash-chromatographie (145:1; 25 ml Fraktionen; 0,5 bar) unterworfen. Die das Produkt enthaltenden Fraktionen werden gesammelt, in 90%igem Dioxan (80 ml) gelöst und nach Sterilfiltration (0,2 µm; PTFE) lyophilisiert. Man erhält N-Acetyl-1,4,6-tri-O-acetyl-muramyl-L-alanyl-D-isoglutaminyl-L-($C^\alpha$)-([2-benzyloxycarbonylamino-ethyl]-sulfonyl-methyl)-glycin-benzylester (α,β-Gemisch) als schwach gelbliches Pulver, 1,27 Mol Wasser enthaltend;

$[\alpha]_D^{20}$ = +36,6±1° (c = 1,030; Dimethylformamid),

$R_f$ = 0,15 (Chloroform:Methanol:Wasser = 70:30:5),

$R_f$ = 0,53 (n-Butanol:Essigsäure:Wasser = 75:7,5:21),
$R_f$ = 0,61 (Acetonitril:Wasser = 3:1).

Das Ausgangsmaterial erhält man wie folgt:

Stufe 29.1: Zu einer Lösung von 8,50 g (20 mMol) $N^{\varepsilon}$-Benzyloxycarbonyl-L-thialysin-benzylester-hydrochlorid werden innerhalb von 30 Minuten bei RT unter Rühren 21,5 g (100 mMol) einer 31,4%igen Wasserstoffperoxidlösung eingetropft. Nach 30stündigem Rühren werden zur Zersetzung von überschüssigem Peroxid 1,5 g Palladiumkohle (5%ig) eingetragen, wobei eine starke Gasentwicklung eintritt. Nach 3stündigem Rühren wird der Katalysator entfernt und die Reaktionslösung am Rotationsverdampfer bei 30° zur Trockne verdampft. Der ölige Rückstand wird in 11 ml Aceton gelöst und durch Zugabe von 20 ml Diethylether zur Kristallisation gebracht. Nach Rekristallisation aus dem gleichen Gemisch erhält man $N^{\varepsilon}$-Benzyloxycarbonyl-L-thialysin-S,S-dioxid-benzylester-hydrochlorid in Form farbloser Nadeln; Smp. 141-142° (Zers.); Smp. des p-Toluolsulfonats 130-134° (Zers.);
$R_f$ = 0,50 (Chloroform:Methanol:Wasser = 70:30:5),
$R_f$ = 0,89 (Methylethylketon:Pyridin:Wasser = 65:5:20),
$R_f$ = 0,71 (n-Butanol:Eisessig:Wasser = 3:1:1).

Stufe 29.2: 2,95 g (6 mMol) N-Acetyl-muramyl-L-alanyl-D-isoglutamin und 2,74 g (6 mMol) $N^{\varepsilon}$-Benzyloxycarbonyl-L-thialysin-S,S-dioxidbenzylester-hydrochlorid werden wie üblich (siehe Stufe 30.1) nach der Dicyclohexylcarbodiimid-1-Hydroxy-benztriazol-Methode miteinander verknüpft. Nach total 51 Stunden Rühren bei RT wird die rötliche Suspension mit 200 ml Essigsäureethylester versetzt, nach 1stündigem Rühren das Unlösliche abfiltriert und das Filtrat am Rotationsverdampfer im Hochvakuum bei 30° zur Trockene verdampft. Der Rückstand wird mit je 100 ml Essigsäureethylester und Wasser versetzt und während einer Stunde gerührt. Das gallertige Material wird abfiltriert, getrocknet und dann zwischen je 80 ml Ober- und Unterphase von gegeneinander gesättigtem n-Butanol-Wasser (1:1) verteilt. Nach einstündigem Rühren wird das unlösliche Material abgesaugt, gewaschen und getrocknet. Man erhält N-Acetyl-muramyl-L-alanyl-D-iso-

glutaminyl-L-($C^{\alpha}$)-([2-benzyloxycarbonylamino-ethyl]-sulfonyl-methyl)-glycin-benzylester (α,β-Gemisch) in Form eines weissen Pulvers, 1,63 Mol Wasser enthaltend;
$R_f$ = 0,60 (Chloroform:Methanol:Wasser = 70:30:5),
$R_f$ = 0,83 (Acetonitril:Wasser = 3:1).

Beispiel 30: 1,50 g (2,24 mMol) N-Acetyl-muramyl-L-alanyl-D-isoglutaminyl-L-serin-benzylester werden in 40 ml abs. Pyridin suspendiert und mit der 40fachen molaren Menge an Essigsäureanhydrid peracetyliert. Das nach 24stündigem Rühren bei RT erhaltene gallertige Material wird durch Zugabe von 5 ml Wasser in Lösung gebracht, das Ganze am Rotationsverdampfer im Hochvakuum (30°) auf etwa 5 ml eingeengt, mit doppelt-destilliertem Wasser versetzt und lyophilisiert. Das Rohprodukt wird in 50 ml warmem Essigsäureethylester aufgenommen und durch Zugabe von fünf Volumenteilen Diethylether gefällt (2x). Der Niederschlag wird in Chloroform-Methanol (1:1) gelöst, sterilfiltriert (0,2 µm, PTFE), nach Verdampfen des Lösungsmittels in abs. Dioxan aufgenommen und gefriergetrocknet. Man erhält N-Acetyl-1,4,6-O-triacetyl-muramyl-L-alanyl-D-isoglutaminyl-L-O-acetyl-serin-benzylester-hydrat (α,β-Gemisch) als farbloses Pulver;
$[\alpha]_D^{20}$ = +26,5±0,9° (c = 1,096; Chloroform:Methanol = 1:1),
$R_f$ = 0,10 (Chloroform:Isopropanol = 7:2),
$R_f$ = 0,69 (Chloroform:Methanol:Wasser = 70:30:5),
$R_f$ = 0,33 (n-Butanol:Essigsäure:Wasser = 75:7,5:21).

Das Ausgangsmaterial erhält man wie folgt:
Stufe 30.1: 5,00 g (10,15 mMol) N-Acetyl-muramyl-L-alanyl-D-isoglutamin, 2,00 g (13,2 mMol) 1-Hydroxybenztriazol (12 % Wasser enthaltend) und 2,35 g (10,15 mMol) L-Serin-benzylester-hydrochlorid werden in 50 ml Dimethylformamid gelöst. Dazu fügt man 1,17 ml (10,66 mMol) N-Methyl-morpholin und schliesslich 2,72 g (13,2 mMol) N,N'-Dicyclohexylcarbodiimid. Nach 24stündigem Rühren bei RT wird die rötliche Suspension mit 100 ml Essigsäureethylester versetzt und während einer Stunde gerührt. Der unlösliche Dicyclohexylharnstoff wird abfiltriert und das Filtrat im Hochvakuum am Rotationsverdampfer bei 30° eingedampft. Das erhaltene gelbe Oel

wird an Kieselgel (60:1; 15 ml Fraktionen) im System Chloroform:Methanol:Wasser = 70:30:5 gereinigt. Die das Produkt enthaltenden reinen Fraktionen werden gesammelt und nach Verdampfen des Lösungsmittels aus tert.-Butanol-Wasser (9:1) lyophilisiert. Man erhält N-Acetyl-muramyl-L-alanyl-D-isoglutaminyl-L-serin-benzylester (α,β-Gemisch) als farbloses Pulver, 1,87 Mol Wasser enthaltend;
$[\alpha]^{20}_{546\ nm}$ = +12,4±1° (c = 0,978; Wasser),
$R_f$ = 0,41 (Chloroform:Methanol:Wasser = 70:30:5),
$R_f$ = 0,59 (Essigsäureethylester:n-Butanol:Pyridin:Essigsäure:Wasser = 42:21:21:6:10).

Beispiel 31: 0,36 g (0,535 mMol) N-Acetyl-muramyl-L-N-methyl-alanyl-D-isoglutamin-benzhydrylester (α,β-Gemisch) werden in 4 ml abs. Pyridin gelöst und nach Zugabe von 0,15 ml (16 mMol) Acetanhydrid während drei Stunden bei RT stehengelassen. Die gelbliche Lösung wird bei 30° im Hochvakuum eingedampft, in 10 ml Wasser aufgenommen und lyophilisiert. Das Rohprodukt wird durch Chromatographie an Kieselgel (1:180) im System Chloroform-Isopropanol (9:1) (0,8 ml Fraktionen) gereinigt. Die einheitlichen Fraktionen werden gesammelt und aus abs. Dioxan lyophilisiert. Man erhält N-Acetyl-1,4,6-tri-O-acetyl-muramyl-L-N-methyl-alanyl-D-isoglutamin-benzhydrylester (α,β-Gemisch) als farbloses Pulver;
$R_f$ = 0,61 (n-Butanol:Essigsäure:Wasser = 75:7,5:21),
$R_f$ = 0,20 (Chloroform:Isopropanol:Essigsäure = 70:8:2).

Das Ausgangsmaterial erhält man wie folgt:
Stufe 31.1: 0,50 g (0,99 mMol) N-Acetyl-muramyl-L-N-methyl-alanyl-D-isoglutamin (α,β-Gemisch), gelöst in Methanol, werden mit überschüssigem Diphenyldiazomethan verestert. Nach zweistündigem Stehen wird zur Trockne verdampft und der Rückstand mehrfach mit Petrolether verrieben. Man saugt ab, nimmt den Rückstand in wenig Methanol auf und fällt das Material mit der zehnfachen Menge an Diethylether:Petrolether (1:1) aus. Man erhält N-Acetyl-muramyl-L-N-methyl-alanyl-D-isoglutamin-benzhydrylester als farbloses Pulver;
$R_f$ = 0,76 (Acetonitril:Wasser = 3:1),
$R_f$ = 0,63 (Chloroform:Methanol:Wasser = 70:30:5).

Beispiel 32: 0,50 g (0,99 mMol) N-Acetyl-muramyl-α-aminoisobutyryl-D-isoglutamin (α,β-Gemisch) werden analog Stufe 31.1 mit Diphenyldiazomethan verestert. Das Rohprodukt [$R_f$ = 0,48 (Chloroform:Methanol:Wasser = 70:30:5)] wird analog Beispiel 31 peracetyliert. Die Reaktionslösung wird im Vakuum zur Trockene verdampft, in Wasser aufgenommen und lyophilisiert. Die Reinigung erfolgt an Kieselgel (1:100) in Chloroform-Methanol (9:1; 1 ml Fraktionen). Die reinen Fraktionen werden vereinigt, in 5 ml Chloroform aufgenommen, filtriert (PTFE; 0,2 µm) und dann zur Trockene verdampft. Der Rückstand wird in abs. Dioxan aufgenommen und lyophilisiert. Man erhält N-Acetyl-1,4,6-tri-O-acetyl-muramyl-α-aminoisobutyryl-D-isoglutamin-benzhydrylester (α,β-Gemisch) als farbloses Pulver, 0,73 Mol Wasser enthaltend;
$[\alpha]_D^{20}$ = +45,7±1° (c = 0,993; Methanol),
$R_f$ = 0,80 (Chloroform:Methanol:Wasser = 70:30:5),
$R_f$ = 0,32 (Chloroform:Methanol = 9:1),
$R_f$ = 0,50 (n-Butanol:Essigsäure:Wasser = 75:7,5:21).

Beispiel 33: 33 mg N-Acetyl-desmethylmuramyl-L-alanyl-D-γ-methoxycarbonyl-isoglutamin-benzhydrylester (α,β-Gemisch) werden analog Beispiel 31 peracetyliert. Nach Lyophilisation aus abs. Dioxan verbleibt N-Acetyl-1,4,6-tri-O-acetyl-desmethylmuramyl-L-alanyl-D-γ-methoxycarbonyl-isoglutamin-benzhydrylester (α,β-Gemisch) als farbloses Pulver;
$R_f$ = 0,67 (Chloroform:Methanol:Wasser = 70:30:5),
$R_f$ = 0,35 (n-Butanol:Essigsäure:Wasser = 75:7,5:21).

Das Ausgangsmaterial erhält man wie folgt:
Stufe 33.1: 0,216 g (0,40 mMol) N-Acetyl-desmethylmuramyl-L-alanyl-D-γ-carboxy-isoglutamin (α,β-Gemisch) werden analog Stufe 31.1 mit Diphenyldiazomethan (3 Aequivalente) verestert. Die hellrötliche Suspension wird nach dreistündigem Rühren filtriert und das Filtrat zur Trockene verdampft. Das Gemisch, bestehend aus Edukt, Mono- und Dibenzhydrylester sowie Zersetzungsprodukten, die vom Diphenyldiazomethan herrühren, wird durch Chromatographie an Kieselgel (1:100) im

System Chloroform-Methanol-Wasser (70:30:5) aufgetrennt. Nach zweimaligem Chromatographieren erhält man N-Acetyl-desmethylmuramyl-L-alanyl-D-γ-benzhydryloxycarbonyl-isoglutamin-benzhydrylester (α,β-Gemisch) als farbloses Harz mit den $R_f$-Werten

$R_f$ = 0,67 (Chloroform:Methanol:Wasser = 70:30:5) und

$R_f$ = 0,46 (n-Butanol:Essigsäure:Wasser = 75:7,5:21)

und N-Acetyl-desmethylmuramyl-L-alanyl-D-isoglutamin-benzhydrylester mit dem $R_f$-Wert

$R_f$ = 0,13 (Chloroform:Methanol:Wasser = 70:30:5).

Stufe 33.2: N-Acetyl-desmethylmuramyl-L-alanyl-D-isoglutamin-benzhydrylester wird in methanolischer Lösung wie üblich mit einer Lösung von Diazomethan in Diethylether verestert. Nach dem Eindampfen verbleibt N-Acetyl-desmethylmuramyl-L-alanyl-D-γ-methoxycarbonyl-isoglutamin-benzhydrylester (α,β-Gemisch) als farbloses Harz;

$R_f$ = 0,56 (Chloroform:Methanol:Wasser = 70:30:5).

Beispiel 34: 1,70 g (2,53 mMol) N-Acetyl-muramyl-L-α-aminobutyryl-D-isoglutamin-benzhydrylester (α,β-Gemisch), gelöst in 25 ml abs. Pyridin, werden analog Beispiel 31 perpropionyliert (90 Aequivalente Anhydrid). Nach dreistündigem Stehen bei RT fügt man 30 ml Wasser zu und verdampft das Ganze im HV bei 30° zur Trockene. Der harzige Rückstand wird in 80 ml abs. Dioxan aufgenommen, filtriert (PTFE; 0,2 μm) und lyophilisiert. Man erhält N-Acetyl-1,4,6-tri-O-propionyl-muramyl-L-α-aminobutyryl-D-isoglutamin-benzhydrylester (α,β-Gemisch) als farbloses Pulver, 0,79 Mol Wasser enthaltend;

$[\alpha]_D^{20}$ = +52,2±3,7° (c = 0,270; Methanol),

$R_f$ = 0,67 (Acetonitril:Wasser = 3:1),

$R_f$ = 0,32 (Chloroform:Isopropanol:Essigsäure = 70:8:2),

$R_f$ = 0,87 (Essigsäureethylester:n-Butanol:Pyridin:Essigsäure:Wasser = 42:21:21:6:10).

Das Augangsmaterial erhält man wie folgt:

Stufe 34.1: 2,03 g (4 mMol) N-Acetyl-muramyl-L-α-aminobutyryl-D-isoglutamin (α,β-Gemisch) werden analog Stufe 31.1 in den Benzhydrylester übergeführt. Nach der üblichen Aufarbeitung verbleibt N-Acetyl-muramyl-L-α-aminobutyryl-D-isoglutamin-benzhydrylester (α,β-Gemisch) als farbloses Pulver;

$[\alpha]_D^{20}$ = +41,8±1,8° (c = 0,548; Methanol),

$R_f$ = 0,73 (Chloroform:Methanol:Wasser = 70:30:5),

$R_f$ = 0,78 (Acetonitril:Wasser = 3:1).

Beispiel 35: Analog Beispiel 1 erhält man aus 2,0 g (2,94 mMol) N-Propionyl-desmethylmuramyl-L-alanyl-D-isoglutamin-benzhydrylester (α,β-Gemisch), der 1,17 Mol Wasser enthält, in 20 ml absolutem Pyridin mit 1,68 ml (13,06 mMol) Propionsäureanhydrid (purissimum; d = 1,012) 1,4,6-Tri-O-propionyl-N-propionyl-desmethylmuramyl-L-alanyl-D-isoglutamin-benzhydrylester (α,β-Gemisch) als farbloses Lyophilisat, das noch 0,68 Mol Wasser enthält;

Smp. 156-157°,

$[\alpha]_D^{20}$ = +39,0±2,1° (c = 0,467; Methanol),

$R_f$ = 0,40 (Chloroform:Ethanol = 9:1),

$R_f$ = 0,48 (Chloroform:Methanol = 9:1),

$R_f$ = 0,88 (Chloroform:Methanol = 4:1).

Beispiel 36: Analog Beispiel 1 erhält man aus 2,0 g (3,1 mMol) N-Propionyl-desmethylmuramyl-L-alanyl-D-glutaminsäure-($C_\alpha$)-n-butylester-($C_\gamma$)-benzylester (hauptsächlich α-Anomeres), der noch 0,41 Mol Wasser enthält, in 20 ml absolutem Pyridin mit 1,14 ml (12,0 mMol) Essigsäureanhydrid 1,4,6-Tri-O-acetyl-N-propionyl-desmethylmuramyl-L-alanyl-D-glutaminsäure-($C_\alpha$)-n-butylester-($C_\gamma$)-benzylester als farbloses Lyophilisat, das noch 0,73 Mol Wasser enthält. Laut $^1$H-NMR-Spektrum (360 MHz) liegt die Verbindung fast ausschliesslich in Form des α-Anomeren vor;

"Smp." 62-64°,

$[\alpha]_D^{20}$ = +36,4±3,6° (c = 0,280; Methanol),

$R_f$ = 0,43 (Chloroform:Methanol = 9:1),

$R_f$ = 0,55 (Chloroform:Ethanol = 9:1),
$R_f$ = 0,85 (Chloroform:Methanol = 4:1).

Beispiel 37: Analog Beispiel 1 erhält man aus 0,57 g (0,83 mMol) N-Propionyl-desmethylmuramyl-L-alanyl-D-glutaminsäure-dibenzylester (α,β-Gemisch), der 0,89 Mol Wasser enthält, in 8 ml absolutem Pyridin mit 0,36 ml (3,807 mMol) Essigsäureanhydrid 1,4,6-Tri-O-acetyl-N-propionyl-desmethylmuramyl-L-alanyl-D-glutaminsäure-dibenzylester (α,β-Gemisch) in Form farbloser Kristalle;
Smp. 141-142° (aus Chloroform:Diethylether = 1:10),
$[\alpha]_D^{20}$ = +22,7±2,2° (c = 0,454; Methylenchlorid),
$R_f$ = 0,42 (Chloroform:Methanol = 9:1),
$R_f$ = 0,46 (Chloroform:Ethanol = 9:1),
$R_f$ = 0,70 (Chloroform:Methanol = 4:1).

Das Ausgangsmaterial erhält man wie folgt:
Stufe 37.1: 0,74 g (1,95 mMol) 4,6-O,O-Isopropyliden-N-propionyl-desmethylmuraminsäure-natriumsalz werden in 20 ml N,N-Dimethylformamid suspendiert, danach 0,342 g (2,145 mMol) 1-Hydroxy-benzotriazol, 0,442 g (2,145 mMol) N,N-Dicyclohexylcarbodiimid und 0,85 g (1,95 mMol) L-Alanyl-D-glutaminsäure-dibenzylester-hydrochlorid zugegeben und das Ganze über Nacht bei Raumtemperatur gerührt. Danach wird von ausgefallenem N,N-Dicyclohexylharnstoff abfiltriert und das so erhaltene Filtrat im Hochvakuum bei 30° zur Trockne eingedampft. Der Rückstand wird in 150 ml Essigsäureethylester aufgenommen und nacheinander mit je 50 ml gesättigter Natriumhydrogencarbonatlösung, 2 N Zitronensäure, gesättigter Natriumhydrogencarbonatlösung und Wasser gewaschen. Die organischen Phasen werden vereinigt, über Natriumsulfat getrocknet und im Hochvakuum bei 30° eingedampft. Man erhält 4,6-O,O-Isopropyliden-N-propionyl-desmethylmuramyl-L-alanyl-D-glutaminsäure-dibenzylester (α,β-Gemisch), der etwas N-Propionyl-desmethylmuramyl-L-alanyl-D-glutaminsäure-dibenzylester (α,β-Gemisch) sowie N,N-Dicyclohexylharnstoff enthält und ohne weitere Reinigung verarbeitet wird.

Stufe 37.2: 3,3 g roher 4,6-O,O-Isopropyliden-N-propionyl-desmethyl-muramyl-L-alanyl-D-glutaminsäure-dibenzylester (α,β-Gemisch) werden in 30 ml 50%iger Essigsäure über Nacht stehengelassen. Die so erhaltene Lösung wird danach filtriert und das Filtrat im Hochvakuum bei 30° eingedampft. Den Rückstand reinigt man durch Säulenchromatographie an 150 g Kieselgel (Typ 60, reinst, Merck; 0,063-0,2 mm) im System Chloroform-Ethanol (9:1; 10 ml Fraktionen). Die Fraktionen 56-95 werden vereinigt und im Hochvakuum eingedampft. Der Rückstand wird dann in 80 ml Methanol gelöst und die so erhaltene, leicht trübe Lösung durch ein Milliporefilter (Fluoropore, PTFE, 0,2 μm) filtriert. Das klare Filtrat wird im Vakuum bei 40° eingedampft. Der Rückstand wird anschliessend in 10 ml absolutem Methanol, das zuvor durch ein Milliporefilter (Fluoropore, PTFE, 0,2 μm) filtriert worden ist, gelöst und durch Zugabe von 100 ml absolutem Diethylether, der ebenfalls durch ein Milliporefilter filtriert worden ist, kristallisiert und mit filtriertem, absolutem Diethylether nachgewaschen. Man erhält N-Propionyl-desmethylmuramyl-L-alanyl-D-glutaminsäure-dibenzylester (α,β-Gemisch) in Form farbloser Kristalle, die 0,89 Mol Wasser enthalten;

Smp. 146-147°,

$[\alpha]_D^{20}$ = 14,6±2,1° (c = 0,478; Methanol),

$R_f$ = 0,20 (Chloroform:Methanol = 9:1),

$R_f$ = 0,65 (Chloroform:Methanol = 4:1),

$R_f$ = 0,81 (Chloroform:Methanol = 7:3).

Beispiel 38: Analog Beispiel 1 erhält man mit Pyridin und Acetanhydrid aus N-Benzoyl-desmethylmuramyl-L-alanyl-D-glutaminsäure-dicholinester-dichlorid 1,4,6-Tri-O-acetyl-N-benzoyl-desmethyl-muramyl-L-alanyl-D-glutaminsäure-dicholinester-dichlorid.

Das Ausgangsmaterial erhält man wie folgt:

Stufe 38.1: Analog wie beschrieben in Synthesis 1982, 138, erhält man aus D-Glutaminsäure-dimethylester-hydrochlorid und überschüssigem Cholinchlorid mit Titan-tetraethylester als Katalysator in Acetonitril bei 80° D-Glutaminsäure-dicholinester-dichlorid-hydrochlorid.

Stufe 38.2: Analog Stufe 6.3 erhält man aus N-Benzoyl-desmethylmuramyl-L-alanin und D-Glutaminsäure-dicholinester-dichlorid-hydrochlorid mit Dicyclohexylcarbodiimid N-Benzoyl-desmethylmuramyl-L-alanyl-D-glutaminsäure-dicholinester-dichlorid.

Beispiel 39: Analog Beispiel 1 erhält man aus N-Benzoyl-desmethylmuramyl-L-alanyl-D-isoglutamin-benzhydrylester mit n-Hexanoylchlorid, Pyridin und 4-Dimethylamino-pyridin N-Benzoyl-1,4,6-tri-O-n-hexanoyl-L-alanyl-D-isoglutamin-benzhydrylester, Smp. 95-98°, $[\alpha]_D^{20}$ = + 40,9° ± 1,8° (c = 0,552; Chloroform), $R_f$ = 0,38 (Chloroform:Aceton = 7:3).

<u>Patentansprüche</u> für die Vertragsstaaten BE, CH, DE, FR, GB, IT, LI, LU, NL und SE

1. Verbindungen der Formel I,

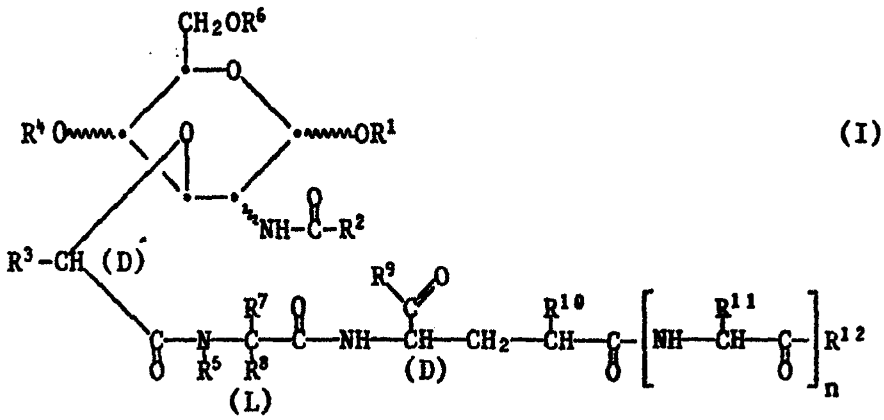

(I)

worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy, 3-Cholesteryloxy oder 2-Trimethylammonio-ethyloxy, $R^{10}$ Wasserstoff, Carboxy, Niederalkoxycarbonyl oder Arylniederalkoxycarbonyl und $R^{11}$ Wasserstoff oder unsubstituiertes oder durch Amino, Hydroxy, Niederalkanoylamino, Niederalkanoyloxy, 2-Benzyloxycarbonylamino-ethyl-sulfinyl, 2-Benzyloxycarbonylamino-ethyl-sulfonyl, 2-Niederalkoxycarbonylamino-ethyl-sulfinyl, 2-Niederalkoxycarbonylamino-ethyl-sulfonyl oder Guanidino substituiertes Niederalkyl bedeuten, mit der Massgabe, dass mindestens einer der

Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im Alkoxyteil verschieden ist, und Salze von solchen Verbindungen mit mindestens einer salzbildenden Gruppe.

2. Verbindungen der Formel I nach Anspruch 1, worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy oder 3-Cholesteryloxy, $R^{10}$ Wasserstoff, Carboxy, Niederalkoxycarbonyl oder Arylniederalkoxycarbonyl und $R^{11}$ Wasserstoff oder unsubstituiertes oder durch Amino oder Hydroxy substituiertes Niederalkyl bedeuten, mit der Massgabe, dass mindestens einer der Reste $R^9$ und $R^{12}$ von Hydroxy, Amino und Niederalkoxy oder $R^{10}$ von Wasserstoff, Carboxy oder Niederalkoxycarbonyl verschieden ist, und Salze von solchen Verbindungen mit mindestens einer salzbildenden Gruppe.

3. Verbindungen nach Anspruch 1 oder 2, worin $R^9$ und $R^{12}$ unabhängig voneinander Hydroxy, Amino, $C_{1-10}$-Alkoxy, Alkanoyloxyniederalkoxy mit bis zu 16 C-Atomen, 3-Cholesteryloxy oder im Phenylteil jeweils unsubstituiertes oder durch Niederalkyl, Phenyl, Halogen, Hydroxy, Niederalkoxy, Niederalkanoyloxy, Amino, Mono- oder Diniederalkylamino oder Niederalkanoylamino substituiertes Phenyl- oder Benzoyloxy-niederalkoxy, und $R^{10}$ Wasserstoff, Carboxy, Niederalkoxycarbonyl oder im Phenylteil unsubstituiertes oder durch Niederalkyl, Phenyl, Halogen, Hydroxy, Niederalkoxy, Niederalkanoyloxy, Amino, Mono- oder Diniederalkylamino oder Niederalkanoylamino substituiertes Phenylniederalkoxycarbonyl bedeuten, und die übrigen Substituenten die obengenannten Bedeutungen haben, mit der Massgabe, dass mindestens einer der Reste $R^9$ und $R^{12}$ von Hydroxy, Amino

und Niederalkoxy oder $R^{10}$ von Wasserstoff, Carboxy und Niederalkoxycarbonyl verschieden ist, und Salze von solchen Verbindungen mit mindestens einer salzbildenden Gruppe.

4. Verbindungen nach Anspruch 1, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-4}$-Alkyl oder Phenyl, $R^3$, $R^5$ und $R^7$ unabhängig voneinander Wasserstoff oder Methyl, oder $R^5$ mit $R^8$ zusammen Trimethylen und $R^7$ Wasserstoff, $R^8$ Wasserstoff, $C_{1-4}$-Alkyl oder durch Phenyl, Hydroxy, Mercapto oder Methylthio substituiertes $C_{1-2}$-Alkyl, $R^9$ und $R^{12}$ unabhängig voneinander Hydroxy, Amino, Niederalkoxy, Phenylniederalkoxy, Niederalkanoyloxyniederalkoxy, Benzoyloxyniederalkoxy oder 3-Cholesteryloxy, $R^{10}$ Wasserstoff, Carboxy, Niederalkoxycarbonyl oder Phenylniederalkoxycarbonyl und $R^{11}$ Wasserstoff oder unsubstituiertes oder durch Amino oder Hydroxy substituiertes $C_{1-4}$-Alkyl bedeuten, mit der Massgabe, dass mindestens einer der Reste $R^9$ und $R^{12}$ von Hydroxy, Amino und Niederalkoxy oder $R^{10}$ von Wasserstoff, Carboxy und Niederalkoxycarbonyl verschieden ist, und Salze von solchen Verbindungen mit mindestens einer salzbildenden Gruppe.

5. Verbindungen nach Anspruch 1, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-2}$-Alkyl oder Phenyl, $R^3$, $R^5$ und $R^7$ unabhängig voneinander Wasserstoff oder Methyl, $R^8$ $C_{1-4}$-Alkyl, $R^9$ und $R^{12}$ unabhängig voneinander Hydroxy, Amino, $C_{1-4}$-Alkoxy, Phenyl-methoxy, Niederalkanoyloxymethoxy, Benzoyloxymethoxy oder 3-Cholesteryloxy, $R^{10}$ Wasserstoff, Carboxy, Alkoxycarbonyl mit bis zu 5 C-Atomen oder Phenylmethoxycarbonyl und $R^{11}$ $C_{1-4}$-Alkyl bedeuten, mit der Massgabe, dass mindestens einer der Reste $R^9$ und $R^{12}$ von Hydroxy, Amino und $C_{1-4}$-Alkoxy oder $R^{10}$ von Wasserstoff, Carboxy und Alkoxycarbonyl mit bis zu 5 C-Atomen verschieden ist, und Salze von solchen Verbindungen mit mindestens einer salzbildenden Gruppe.

6. Verbindungen nach Anspruch 1, worin sich der Hexoseteil von D-Glucose ableitet, n für 0 oder 1 steht, $R^1$, $R^4$ und $R^6$ Acetyl oder Butyryl, $R^2$ $C_{1-2}$-Alkyl oder Phenyl, $R^3$ Wasserstoff oder Methyl, $R^5$ und $R^7$ Wasserstoff, $R^8$ $C_{1-3}$-Alkyl, $R^9$ Amino, $C_{1-4}$-Alkoxy, Pivaloyloxymethoxy oder Mono- oder Diphenylmethoxy, $R^{10}$ Wasserstoff, $R^{11}$ Methyl und $R^{12}$ Mono- oder Diphenylmethoxy oder 3-Cholesteryloxy bedeuten.

7. Verbindungen nach Anspruch 1, worin sich der Hexoseteil von D-Glucose ableitet, n für 0 oder 1 steht, $R^1$, $R^4$ und $R^6$ $C_{2-6}$-Alkanoyl, $R^2$ $C_{1-4}$-Alkyl oder Phenyl, $R^3$, $R^5$ und $R^7$ unabhängig voneinander Wasserstoff oder Methyl, $R^8$ $C_{1-4}$-Alkyl, $R^9$ Amino, Niederalkoxy, Pivaloyloxymethoxy, Diphenylmethoxy, Benzyloxy oder 2-Trimethylammonio-ethoxy, $R^{10}$ Wasserstoff oder Niederalkoxycarbonyl, $R^{11}$ $C_{1-4}$-Alkyl, Niederalkanoyloxymethyl, oder (2-Benzyloxycarbonylamino-ethyl)-sulfonyl-methyl und $R^{12}$ Amino, Niederalkoxy, Pivaloyloxymethoxy, Diphenylmethoxy, Benzyloxy, 2-Trimethylammonioethoxy, 3-Cholesteryloxy oder Benzoyloxymethoxy bedeuten, mit der Massgabe, dass mindestens einer der Reste $R^9$ und $R^{12}$ von Amino und Niederalkoxy verschieden ist, und Salze von solchen zur Salzbildung fähigen Verbindungen.

8. Verbindungen nach Anspruch 1 oder 7, worin mindestens einer der Reste $R^9$ und $R^{12}$ für Pivaloyloxymethoxy, Diphenylmethoxy, Benzyloxy, 2-Trimethylammonio-ethoxy, 3-Cholesteryloxy oder Benzoyloxymethoxy steht und der andere der Reste $R^9$ und $R^{12}$ die genannte Bedeutung hat, und Salze von solchen zur Salzbildung fähigen Verbindungen.

9. Eine Verbindung nach Anspruch 1, ausgewählt aus der Gruppe, bestehend aus 1,4,6-Tri-O-acetyl-N-propionyl-desmethylmuramyl-L-alanyl-D-isoglutamin-benzhydrylester,
1,4,6-Tri-O-acetyl-N-propionyl-desmethylmuramyl-L-alanyl-D-isoglutamin-benzylester,
1,4,6-Tri-O-acetyl-N-propionyl-desmethylmuramyl-L-alanyl-D-glutaminsäure-($C_\alpha$)-n-butylester-($C_\gamma$)-benzhydrylester,

N-Acetyl-1,4,6-tri-O-acetyl-muramyl-L-alanyl-D-glutaminsäure-($C_\alpha$)-pivaloyloxymethylester-($C_\gamma$)-benzylester,
1,4,6-Tri-O-acetyl-N-benzoyl-desmethylmuramyl-L-alanyl-D-isoglutamin-benzylester,
1,4,6-Tri-O-acetyl-N-benzoyl-desmethylmuramyl-L-alanyl-D-isoglutamin-benzhydrylester,
N-Benzoyl-1,4,6-tri-O-butyryl-desmethylmuramyl-L-alanyl-D-isoglutamin-benzhydrylester,
N-Acetyl-(1α,β),4,6-tri-O-acetyl-desmethylmuramyl-L-α-aminobutyryl-D-isoglutamin-benzhydrylester,
N-Acetyl-(1α,β),4,6-tri-O-acetyl-desmethylmuramyl-L-α-aminobutyryl-D-glutaminsäure-dibenzhydrylester,
N-Acetyl-(1α,β),4,6-tri-O-acetyl-desmethylmuramyl-L-valyl-D-isoglutamin-benzhydrylester,
N-Acetyl-(1α,β),4,6-tri-O-acetyl-muramyl-L-alanyl-D-isoglutaminyl-L-alanin-cholesteryl-3-ester,
N-Acetyl-1,4,6-tri-O-acetyl-muramyl-α-amino-isobutyryl-D-isoglutamin-benzhydrylester,
N-Acetyl-1,4,6-tri-O-acetyl-desmethylmuramyl-L-alanyl-D-γ-methoxy-carbonyl-isoglutamin-benzhydrylester,
N-Acetyl-1,4,6-tri-O-acetyl-muramyl-L-alanyl-D-isoglutaminyl-L-α-([2-benzyloxy-carbonylamino-ethyl]-sulfonyl-methyl)-glycin-benzylester,
1,4,6-Tri-O-acetyl-N-benzoyl-desmethylmuramyl-L-alanyl-D-glutaminsäure-dicholinester,
N-Propionyl-1,4,6-tri-O-propionyl-desmethylmuramyl-L-alanyl-D-isoglutamin-benzhydrylester,
1,4,6-Tri-O-acetyl-N-propionyl-desmethylmuramyl-L-alanyl-D-glutaminsäure-α-n-butylester-γ-benzylester,
1,4,6-Tri-O-acetyl-N-propionyl-desmethylmuramyl-L-alanyl-D-glutaminsäure-dibenzylester,
1,4,6-Tri-O-acetyl-N-benzoyl-desmethylmuramyl-L-alanyl-D-isoglutamin-benzylester,
1,4,6-Tri-O-acetyl-N-propionyl-desmethylmuramyl-L-alanyl-D-glutaminsäure-dipivaloyloxymethylester und

1,4,6-Tri-O-acetyl-N-propionyl-desmethylmuramyl-L-alanyl-D-isoglutaminyl-L-alanin-benzoyloxymethylester und den pharmazeutisch verwendbaren Salzen solcher zur Salzbildung fähiger Verbindungen.

10. Eine Verbindung nach einem der Ansprüche 1-9 oder ein pharmazeutisch verwendbares Salz einer solchen Verbindung mit mindestens einer salzbildenden Gruppe zur Anwendung in einem Verfahren zur therapeutischen Behandlung des menschlichen oder tierischen Körpers.

11. Pharmazeutische Präparate, die eine Verbindung nach einem der Ansprüche 1-9 oder ein pharmazeutisch verwendbares Salz einer solchen zur Salzbildung fähigen Verbindung zusammen mit einem pharmazeutischen Trägermaterial enthalten.

12. Verwendung einer Verbindung nach einem der Ansprüche 1-9 oder eines pharmazeutisch verwendbaren Salzes einer solchen zur Salzbildung fähigen Verbindung zur Herstellung von pharmazeutischen Präparaten, die für die Anwendung zur Prophylaxe und Therapie von Virusinfektionen bestimmt sind.

13. Verwendung nach Anspruch 12 einer Verbindung gemäss einem der Ansprüche 1-9 zur Herstellung von pharmazeutischen Präparaten, die für die Anwendung zur Prophylaxe und Therapie von Virusinfektionen, die durch Influenza-, Parainfluenza-, Herpes-, Encephalomyocarditis- oder Vaccinia-Viren hervorgerufen werden, bestimmt sind.

14. Verfahren zur Herstellung von Verbindungen der Formel I,

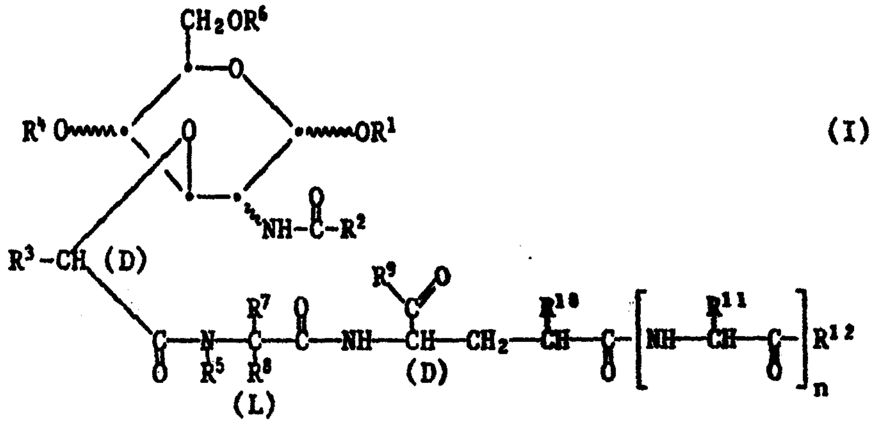

(I)

worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-18}$-Alkoxy, Arylniederalkoxy, Alkanoyloxyniederalkoxy mit bis zu 16 C-Atomen, Aroyloxyniederalkoxy, 3-Cholesteryloxy oder 2-Trimethylammonio-ethyloxy, $R^{10}$ Wasserstoff, Carboxy, Niederalkoxycarbonyl oder Arylniederalkoxycarbonyl und $R^{11}$ Wasserstoff oder unsubstituiertes oder durch Amino, Hydroxy, Niederalkanoylamino, Niederalkanoyloxy, 2-Benzyloxycarbonylamino-ethyl-sulfinyl, 2-Benzyloxycarbonylamino-ethyl-sulfonyl, 2-Niederalkoxycarbonylamino-ethyl-sulfinyl, 2-Niederalkoxycarbonylamino-ethyl-sulfonyl oder Guanidino substituiertes Niederalkyl bedeuten, mit der Massgabe, dass mindestens einer der Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im Alkoxyteil verschieden ist, und von Salzen von solchen Verbindungen mit mindestens einer salzbildenden Gruppe, dadurch gekennzeichnet, dass man

a) eine Verbindung der Formel II,

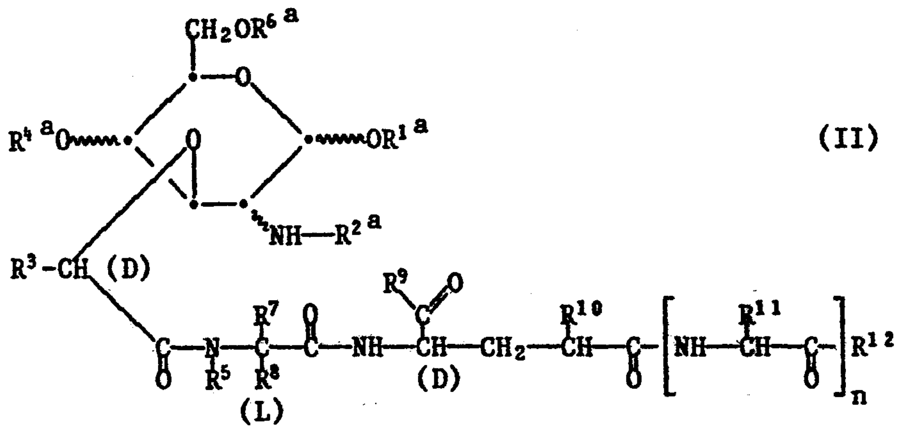

(II)

worin mindestens einer der Reste $R^{1a}$, $R^{2a}$, $R^{4a}$ und $R^{6a}$ für Wasserstoff steht und die übrigen dieser Reste die Bedeutungen von $R^1$, der Gruppe $R^2$-C(=O)-, $R^4$ bzw. $R^6$ haben, und die restlichen Substituenten die obengenannten Bedeutungen haben, wobei in einer Verbindung der Formel II vorhandene freie funktionelle Gruppen mit Ausnahme der Gruppen, die an der Reaktion teilnehmen sollen, erforderlichenfalls durch leicht abspaltbare Schutzgruppen geschützt sind, mit einem den einzuführenden Rest $R^1$, $R^2$-C(=O)-, $R^4$ oder $R^6$ übertragenden Acylierungsmittel umsetzt und vorhandene Schutzgruppen erforderlichenfalls abspaltet, oder

b) eine Verbindung der Formel III,

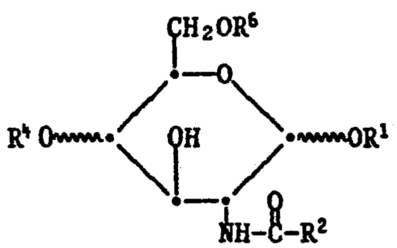

(III)

worin die Substituenten die obengenannten Bedeutungen haben, oder ein reaktionsfähiges Derivat davon mit einer Verbindung der Formel IV,

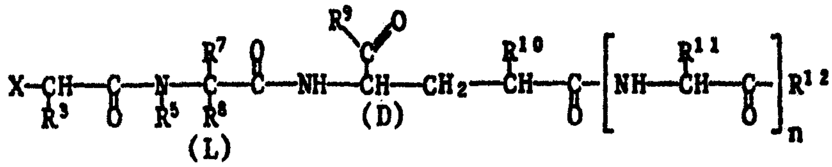
(IV)

worin X eine reaktionsfähige veresterte Hydroxygruppe bedeutet, und die restlichen Substituenten die obengenannten Bedeutungen haben, wobei in einer Verbindung der Formel IV vorhandene freie funktionelle Gruppen mit Ausnahme von X erforderlichenfalls durch leicht abspaltbare Schutzgruppen geschützt sind, umsetzt und vorhandene Schutzgruppen erforderlichenfalls abspaltet, oder

c) eine Verbindung der Formel V,

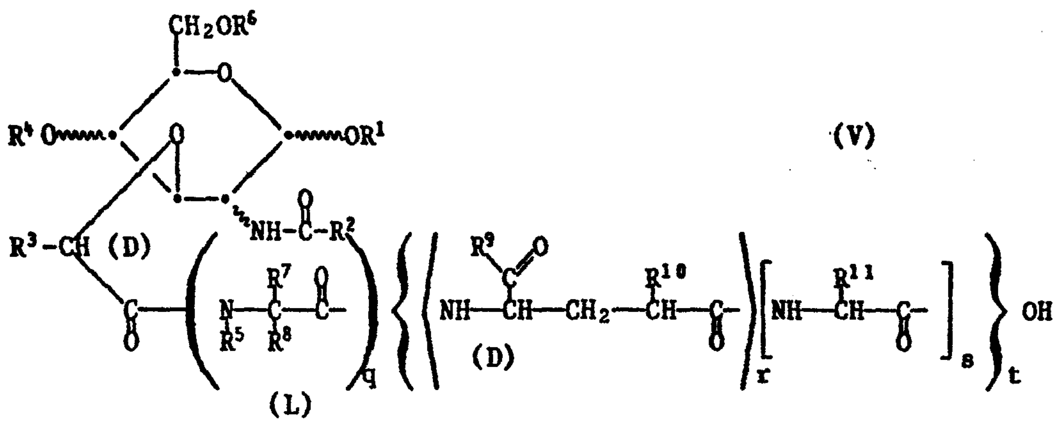
(V)

worin q, r, s und t unabhängig voneinander für 0 oder 1 stehen und worin die Substituenten die obengenannten Bedeutungen haben, wobei in einer Verbindung der Formel V vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit einer Verbindung der Formel VI,

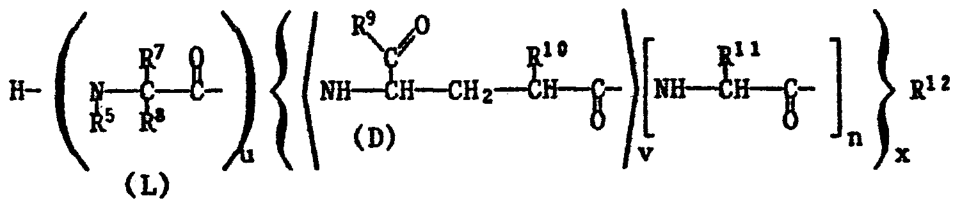 (VI)

worin u, v und x unabhängig voneinander für 0 oder 1 stehen und die übrigen Symbole und Substituenten die obengenannten Bedeutungen haben, wobei in einer Verbindung der Formel VI vorhandene freie funktionelle Gruppen mit Ausnahme der Gruppe, die an der Reaktion teilnehmen soll, erforderlichenfalls durch leicht abspaltbare Schutzgruppen geschützt sind, wobei u, v und x für 1 stehen, wenn im Reaktionspartner der Formel V q und t für 0 stehen, oder u für 0 und v und x für 1 stehen, wenn q für 1 und t für 0 stehen, oder u und v für 0 und x für 1 stehen, wenn q, r und t für 1 und s für 0 stehen oder (zur Herstellung von Verbindungen der Formel I, worin n für 1 steht) u und x für 0 stehen, wenn q, r, s und t für 1 stehen, oder mit einem reaktionsfähigen Derivat davon umsetzt und vorhandene Schutzgruppen erforderlichenfalls abspaltet, oder

d) zur Herstellung einer Verbindung der Formel I, worin $R^9$ eine der obengenannten Bedeutungen ausser Hydroxy und Amino hat und/oder $R^{10}$ für Niederalkoxycarbonyl oder Arylniederalkoxycarbonyl steht und die übrigen Substituenten die obengenannten Bedeutungen haben, eine Verbindung der Formel VII,

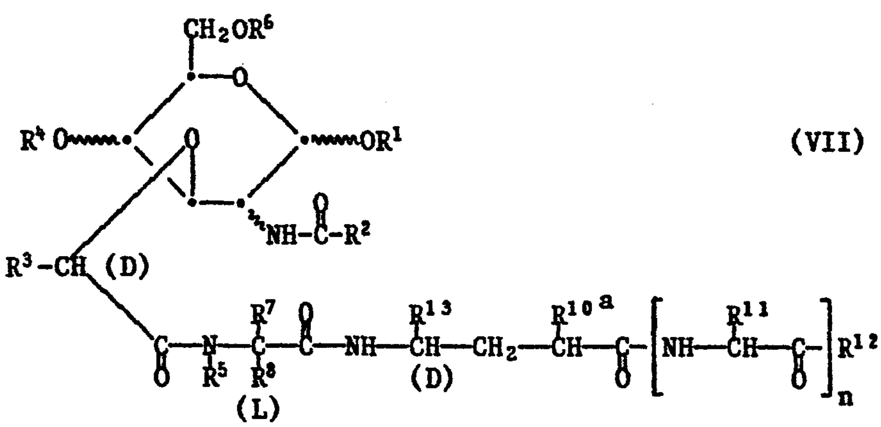 (VII)

worin mindestens einer der Reste $R^{10a}$ und $R^{13}$ für Carboxy steht und der andere der Reste $R^{10a}$ und $R^{13}$ die obengenannte Bedeutung von $R^{10}$ beziehungsweise von der Gruppe $R^9$-C(=O)- hat, und worin die übrigen Substituenten die obengenannten Bedeutungen haben, wobei in einer Verbindung der Formel VII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon verestert und vorhandene Schutzgruppen erforderlichenfalls abspaltet, oder

e) in einer Verbindung der Formel I, worin mindestens einer der Reste $R^8$, C(=O)-$R^9$, $R^{10}$, $R^{11}$ und C(=O)-$R^{12}$ in einer geschützten Form vorliegt, die nicht der Definition des gewünschten Endstoffes entspricht, die entsprechende(n) Schutzgruppe(n) abspaltet, oder

f) zur Herstellung einer Verbindung der Formel I, worin $R^9$ Amino bedeutet und die übrigen Substituenten die obengenannten Bedeutungen haben, eine Verbindung der Formel VIII,

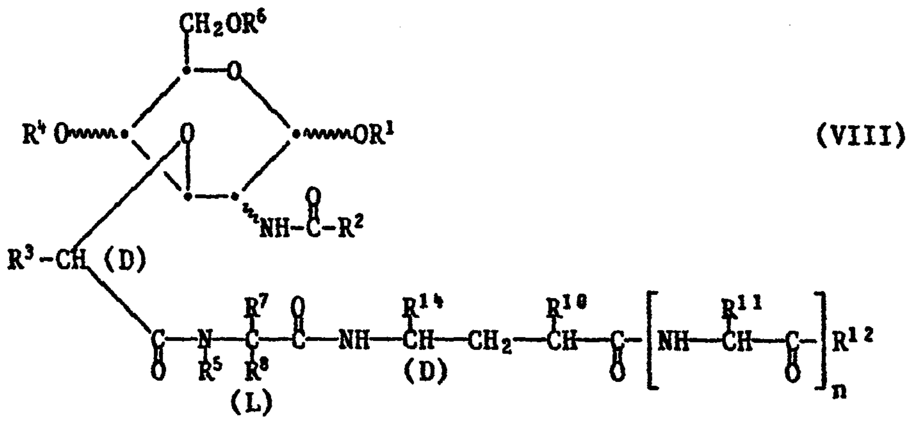

(VIII)

worin der Rest $R^{14}$ für Carboxy steht und die übrigen Substituenten die obengenannten Bedeutungen haben, wobei in einer Verbindung der Formel VIII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon amidiert und vorhandene Schutzgruppen erforderlichenfalls abspaltet,

und, wenn erwünscht, nach Durchführung eines der Verfahren a - f) eine erhaltene Verbindung der Formel I mit mindestens einer salzbildenden Gruppe in ihr Salz überführt oder ein erhaltenes Salz einer Verbindung der Formel I in die freie Verbindung umwandelt, und/oder ein erhaltenes Isomerengemisch auftrennt.

Patentansprüche für den Vertragsstaat AT

1. Verfahren zur Herstellung von Verbindungen der Formel I,

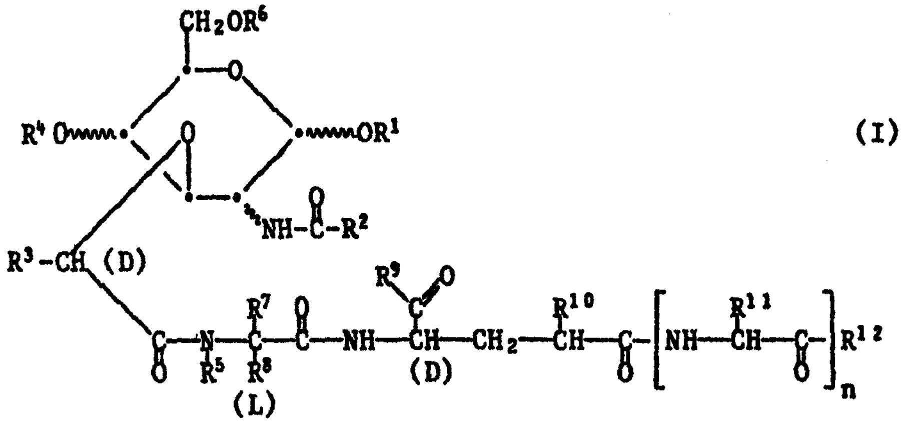

(I)

worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy, 3-Cholesteryloxy oder 2-Trimethylammonio-ethyloxy, $R^{10}$ Wasserstoff, Carboxy, Niederalkoxycarbonyl oder Arylniederalkoxycarbonyl und $R^{11}$ Wasserstoff oder unsubstituiertes oder durch Amino, Hydroxy, Niederalkanoylamino, Niederalkanoyloxy, 2-Benzyloxycarbonylamino-ethyl-sulfinyl, 2-Benzyloxycarbonylamino-ethyl-sulfonyl, 2-Niederalkoxycarbonylamino-ethyl-sulfinyl, 2-Niederalkoxycarbonylamino-ethyl-sulfonyl oder Guanidino substituiertes Niederalkyl bedeuten, mit der Massgabe, dass mindestens einer der Reste $R^9$ und $R^{12}$ von Hydroxy, Amino und $C_{1-7}$-Alkoxy oder $R^{10}$ von Wasserstoff, Carboxy und Alkoxycarbonyl mit bis zu 7 C-Atomen im

Alkoxyteil verschieden ist, und von Salzen von solchen Verbindungen mit mindestens einer salzbildenden Gruppe, dadurch gekennzeichnet, dass man

a) eine Verbindung der Formel II,

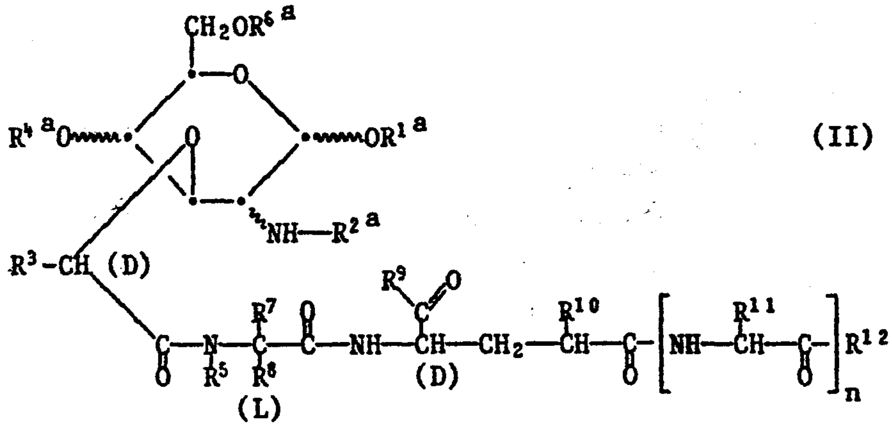

(II)

worin mindestens einer der Reste $R^{1a}$, $R^{2a}$, $R^{4a}$ und $R^{6a}$ für Wasserstoff steht und die übrigen dieser Reste die Bedeutungen von $R^1$, der Gruppe $R^2$-C(=O)-, $R^4$ bzw. $R^6$ haben, und die restlichen Substituenten die obengenannten Bedeutungen haben, wobei in einer Verbindung der Formel II vorhandene freie funktionelle Gruppen mit Ausnahme der Gruppen, die an der Reaktion teilnehmen sollen, erforderlichenfalls durch leicht abspaltbare Schutzgruppen geschützt sind, mit einem den einzuführenden Rest $R^1$, $R^2$-C(=O)-, $R^4$ oder $R^6$ übertragenden Acylierungsmittel umsetzt und vorhandene Schutzgruppen erforderlichenfalls abspaltet, oder

b) eine Verbindung der Formel III,

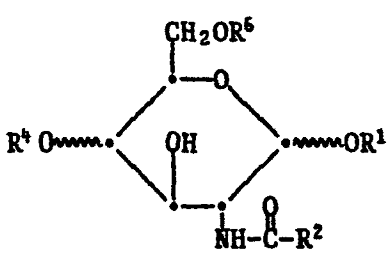

(III)

worin die Substituenten die obengenannten Bedeutungen haben, oder ein reaktionsfähiges Derivat davon mit einer Verbindung der Formel IV,

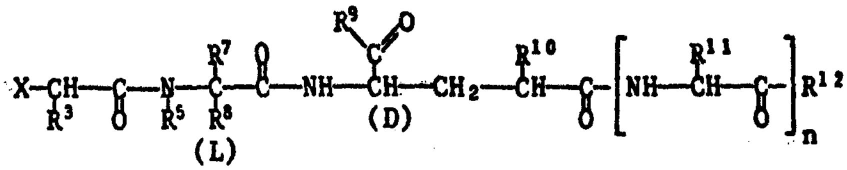

(IV)

worin X eine reaktionsfähige veresterte Hydroxygruppe bedeutet, und die restlichen Substituenten die obengenannten Bedeutungen haben, wobei in einer Verbindung der Formel IV vorhandene freie funktionelle Gruppen mit Ausnahme von X erforderlichenfalls durch leicht abspaltbare Schutzgruppen geschützt sind, umsetzt und vorhandene Schutzgruppen erforderlichenfalls abspaltet, oder

c) eine Verbindung der Formel V,

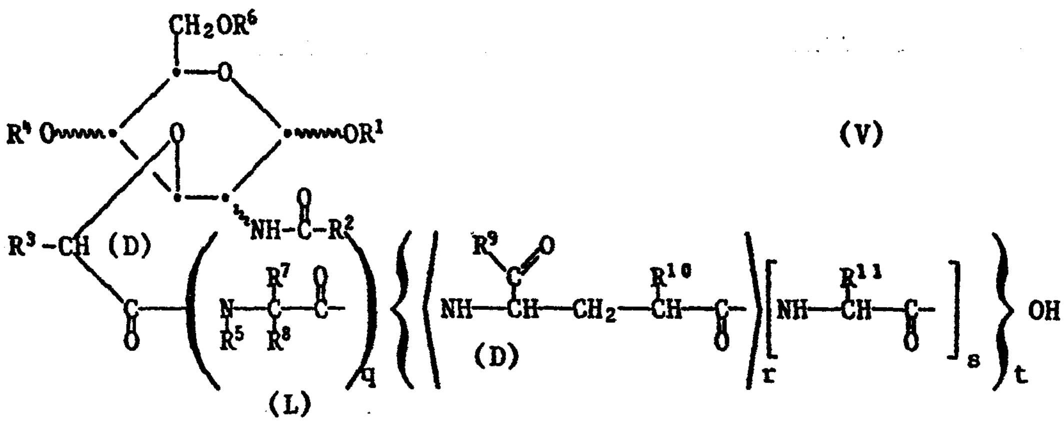

(V)

worin q, r, s und t unabhängig voneinander für 0 oder 1 stehen und worin die Substituenten die obengenannten Bedeutungen haben, wobei in einer Verbindung der Formel V vorhandene freie funktionelle Gruppen mit Ausnahme der Gruppe, die an der Reaktion teilnehmen soll, erforderlichenfalls durch leicht abspaltbare Schutzgruppen geschützt sind, oder ein reaktionsfähiges Carbonsäurederivat davon mit einer Verbindung der Formel VI,

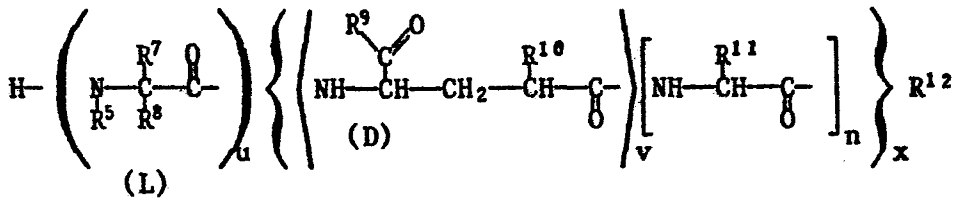

(VI)

worin u, v und x unabhängig voneinander für 0 oder 1 stehen und die übrigen Symbole und Substituenten die obengenannten Bedeutungen haben, wobei in einer Verbindung der Formel VI vorhandene freie funktionelle Gruppen mit Ausnahme der Gruppe, die an der Reaktion teilnehmen soll, erforderlichenfalls durch leicht abspaltbare Schutzgruppen geschützt sind, wobei u, v und x für 1 stehen, wenn im Reaktionspartner der Formel V q und t für 0 stehen, oder u für 0 und v und x für 1 stehen, wenn q für 1 und t für 0 stehen, oder u und v für 0 und x für 1 stehen, wenn q, r und t für 1 und s für 0 stehen oder (zur Herstellung von Verbindungen der Formel I, worin n für 1 steht) u und x für 0 stehen, wenn q, r, s und t für 1 stehen, oder mit einem reaktionsfähigen Derivat davon umsetzt und vorhandene Schutzgruppen erforderlichenfalls abspaltet, oder

d) zur Herstellung einer Verbindung der Formel I, worin $R^9$ eine der obengenannten Bedeutungen ausser Hydroxy und Amino hat und/oder $R^{10}$ für Niederalkoxycarbonyl oder Arylniederalkoxycarbonyl steht und die übrigen Substituenten die obengenannten Bedeutungen haben, eine Verbindung der Formel VII,

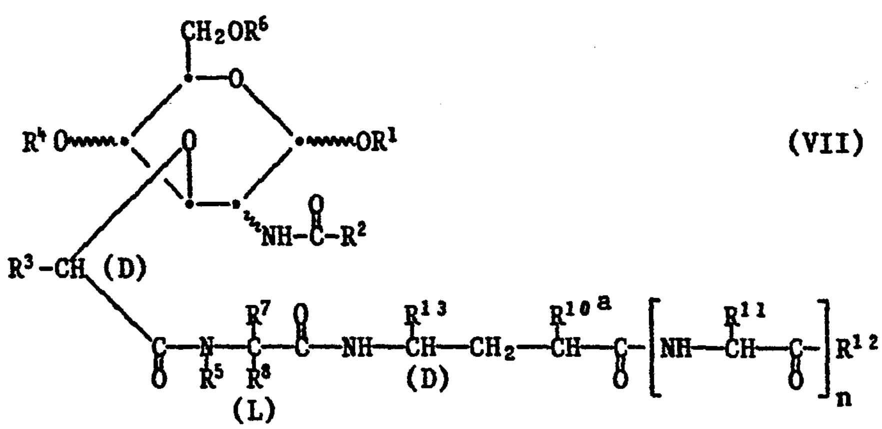

(VII)

worin mindestens einer der Reste $R^{10a}$ und $R^{13}$ für Carboxy steht und der andere der Reste $R^{10a}$ und $R^{13}$ die obengenannte Bedeutung von $R^{10}$ beziehungsweise von der Gruppe $R^9$-C(=O)- hat, und worin die übrigen Substituenten die obengenannten Bedeutungen haben, wobei in einer Verbindung der Formel VII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon verestert und vorhandene Schutzgruppen erforderlichenfalls abspaltet, oder

e) in einer Verbindung der Formel I, worin mindestens einer der Reste $R^8$, C(=O)-$R^9$, $R^{10}$, $R^{11}$ und C(=O)-$R^{12}$ in einer geschützten Form vorliegt, die nicht der Definition des gewünschten Endstoffes entspricht, die entsprechende(n) Schutzgruppe(n) abspaltet, oder

f) zur Herstellung einer Verbindung der Formel I, worin $R^9$ Amino bedeutet und die übrigen Substituenten die obengenannten Bedeutungen haben, eine Verbindung der Formel VIII,

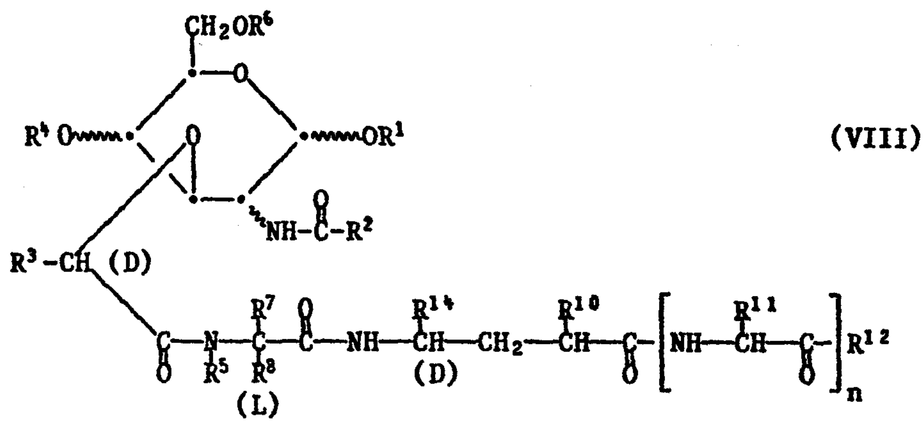

(VIII)

worin der Rest $R^{14}$ für Carboxy steht und die übrigen Substituenten die obengenannten Bedeutungen haben, wobei in einer Verbindung der Formel VIII vorhandene freie funktionelle Gruppen mit Ausnahme der Gruppen, die an der Reaktion teilnehmen sollen, erforderlichenfalls durch leicht abspaltbare Schutzgruppen geschützt sind, oder ein reaktionsfähiges Carbonsäurederivat davon amidiert und vorhandene Schutzgruppen erforderlichenfalls abspaltet,

und, wenn erwünscht, nach Durchführung eines der Verfahren a - f) eine erhaltene Verbindung der Formel I mit mindestens einer salzbildenden Gruppe in ihr Salz überführt oder ein erhaltenes Salz einer Verbindung der Formel I in die freie Verbindung umwandelt, und/oder ein erhaltenes Isomerengemisch auftrennt.

2. Verfahren nach Anspruch 1, dadurch gekennzeichnet, dass man die Ausgangsstoffe so wählt, dass man eine Verbindung der Formel I, worin sich der Hexoseteil von D-Glucose, D-Mannose oder D-Galactose ableitet, n für 0 oder 1 steht, und $R^1$, $R^4$ und $R^6$ unabhängig voneinander Niederalkanoyl oder Benzoyl, $R^2$ Niederalkyl oder Phenyl, $R^3$, $R^5$ und $R^7$ unabhängig voneinander Wasserstoff oder Niederalkyl, oder $R^5$ mit $R^8$ zusammen Trimethylen und $R^7$ Wasserstoff, $R^8$ Wasserstoff oder unsubstituiertes oder durch Phenyl, Hydroxy, Mercapto oder Niederalkylthio substituiertes Niederalkyl, $R^9$ und $R^{12}$ unabhängig voneinander Hydroxy, Amino, $C_{1-10}$-Alkoxy, Arylniederalkoxy, Alkanoyloxyniederalkoxy mit bis zu 16 C-Atomen, Aroyloxyniederalkoxy oder 3-Cholesteryloxy, $R^{10}$ Wasserstoff, Carboxy, Niederalkoxycarbonyl oder Arylniederalkoxycarbonyl und $R^{11}$ Wasserstoff oder unsubstituiertes oder durch Amino oder Hydroxy substituiertes Niederalkyl bedeuten, mit der Massgabe, dass mindestens einer der Reste $R^9$ und $R^{12}$ von Hydroxy, Amino und Niederalkoxy oder $R^{10}$ von Wasserstoff, Carboxy oder Niederalkoxycarbonyl verschieden ist, oder ein Salz einer solchen Verbindung mit mindestens einer salzbildenden Gruppe herstellt.

3. Verfahren nach Anspruch 1 oder 2, dadurch gekennzeichnet, dass man die Ausgangsstoffe so wählt, dass man eine Verbindung der Formel I, worin $R^9$ und $R^{12}$ unabhängig voneinander Hydroxy, Amino, $C_{1-10}$-Alkoxy, Alkanoyloxyniederalkoxy mit bis zu 16 C-Atomen, 3-Cholesteryloxy oder im Phenylteil jeweils unsubstituiertes oder durch Niederalkyl, Phenyl, Halogen, Hydroxy, Niederalkoxy, Niederalkanoyloxy, Amino, Mono- oder Diniederalkylamino oder Niederalkanoylamino substituiertes Phenyl- oder Benzoyloxy-niederalkoxy, und $R^{10}$ Wasserstoff, Carboxy, Niederalkoxycarbonyl oder im Phenylteil unsubstituiertes oder durch Niederalkyl, Phenyl, Halogen,

Hydroxy, Niederalkoxy, Niederalkanoyloxy, Amino, Mono- oder Diniederalkylamino oder Niederalkanoylamino substituiertes Phenylniederalkoxycarbonyl bedeuten, und die übrigen Substituenten die obengenannten Bedeutungen haben, mit der Massgabe, dass mindestens einer der Reste $R^9$ und $R^{12}$ von Hydroxy, Amino und Niederalkoxy oder $R^{10}$ von Wasserstoff, Carboxy und Niederalkoxycarbonyl verschieden ist, oder ein Salz einer solchen Verbindung mit mindestens einer salzbildenden Gruppe herstellt.

4. Verfahren nach Anspruch 1, dadurch gekennzeichnet, dass man die Ausgangsstoffe so wählt, dass man eine Verbindung der Formel I, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-4}$-Alkyl oder Phenyl, $R^3$, $R^5$ und $R^7$ unabhängig voneinander Wasserstoff oder Methyl, oder $R^5$ mit $R^8$ zusammen Trimethylen und $R^7$ Wasserstoff, $R^8$ Wasserstoff, $C_{1-4}$-Alkyl oder durch Phenyl, Hydroxy, Mercapto oder Methylthio substituiertes $C_{1-2}$-Alkyl, $R^9$ und $R^{12}$ unabhängig voneinander Hydroxy, Amino, Niederalkoxy, Phenylniederalkoxy, Niederalkanoyloxyniederalkoxy, Benzoyloxyniederalkoxy oder 3-Cholesteryloxy, $R^{10}$ Wasserstoff, Carboxy, Niederalkoxycarbonyl oder Phenylniederalkoxycarbonyl und $R^{11}$ Wasserstoff oder unsubstituiertes oder durch Amino oder Hydroxy substituiertes $C_{1-4}$-Alkyl bedeuten, mit der Massgabe, dass mindestens einer der Reste $R^9$ und $R^{12}$ von Hydroxy, Amino und Niederalkoxy oder $R^{10}$ von Wasserstoff, Carboxy und Niederalkoxycarbonyl verschieden ist, oder ein Salz einer solchen Verbindung mit mindestens einer salzbildenden Gruppe herstellt.

5. Verfahren nach Anspruch 1, dadurch gekennzeichnet, dass man die Ausgangsstoffe so wählt, dass man eine Verbindung der Formel I, worin sich der Hexoseteil von D-Glucose oder D-Mannose ableitet, n für 0 oder 1 steht, $R^1$, $R^4$ und $R^6$ unabhängig voneinander $C_{2-4}$-Alkanoyl oder Benzoyl, $R^2$ $C_{1-2}$-Alkyl oder Phenyl, $R^3$, $R^5$ und $R^7$ unabhängig voneinander Wasserstoff oder Methyl, $R^8$ $C_{1-4}$-Alkyl, $R^9$ und $R^{12}$ unabhängig voneinander Hydroxy, Amino, $C_{1-4}$-Alkoxy, Phenylmethoxy, Niederalkanoyloxymethoxy, Benzoyloxymethoxy oder

3-Cholesteryloxy, $R^{10}$ Wasserstoff, Carboxy, Alkoxycarbonyl mit bis zu 5 C-Atomen oder Phenylmethoxycarbonyl und $R^{11}$ $C_{1-4}$-Alkyl bedeuten, mit der Massgabe, dass mindestens einer der Reste $R^9$ und $R^{12}$ von Hydroxy, Amino und $C_{1-4}$-Alkoxy oder $R^{10}$ von Wasserstoff, Carboxy und Alkoxycarbonyl mit bis zu 5 C-Atomen verschieden ist, oder ein Salz einer solchen Verbindung mit mindestens einer salzbildenden Gruppe herstellt.

6. Verfahren nach Anspruch 1, dadurch gekennzeichnet, dass man die Ausgangsstoffe so wählt, dass man eine Verbindung der Formel I, worin sich der Hexoseteil von D-Glucose ableitet, n für 0 oder 1 steht, $R^1$, $R^4$ und $R^6$ Acetyl oder Butyryl, $R^2$ $C_{1-2}$-Alkyl oder Phenyl, $R^3$ Wasserstoff oder Methyl, $R^5$ und $R^7$ Wasserstoff, $R^8$ $C_{1-3}$-Alkyl, $R^9$ Amino, $C_{1-4}$-Alkoxy, Pivaloyloxymethoxy oder Mono- oder Diphenylmethoxy, $R^{10}$ Wasserstoff, $R^{11}$ Methyl und $R^{12}$ Mono- oder Diphenylmethoxy oder 3-Cholesteryloxy bedeuten, herstellt.

7. Verfahren nach Anspruch 1, dadurch gekennzeichnet, dass man die Ausgangsstoffe so wählt, dass man eine Verbindung der Formel I, worin sich der Hexoseteil von D-Glucose ableitet, n für 0 oder 1 steht, $R^1$, $R^4$ und $R^6$ $C_{2-6}$-Alkanoyl, $R^2$ $C_{1-4}$-Alkyl oder Phenyl, $R^3$, $R^5$ und $R^7$ unabhängig voneinander Wasserstoff oder Methyl, $R^8$ $C_{1-4}$-Alkyl, $R^9$ Amino, Niederalkoxy, Pivaloyloxymethoxy, Diphenylmethoxy, Benzyloxy oder 2-Trimethylammonio-ethoxy, $R^{10}$ Wasserstoff oder Niederalkoxycarbonyl, $R^{11}$ $C_{1-4}$-Alkyl, Niederalkanoyloxymethyl, oder (2-Benzyloxycarbonylamino-ethyl)-sulfonyl-methyl und $R^{12}$ Amino, Niederalkoxy, Pivaloyloxymethoxy, Diphenylmethoxy, Benzyloxy, 2-Trimethylammonio-ethoxy, 3-Cholesteryloxy oder Benzoyloxymethoxy bedeuten, mit der Massgabe, dass mindestens einer der Reste $R^9$ und $R^{12}$ von Amino und Niederalkoxy verschieden ist, oder ein Salz einer solchen zur Salzbildung fähigen Verbindung herstellt.

8. Verfahren nach Anspruch 1 oder 7, dadurch gekennzeichnet, dass man die Ausgangsstoffe so wählt, dass man eine Verbindung der Formel I, worin mindestens einer der Reste $R^9$ und $R^{12}$ für Pivaloyloxymethoxy, Diphenylmethoxy, Benzyloxy, 2-Trimethylammonio-ethoxy,

3-Cholesteryloxy oder Benzoyloxymethoxy steht und der andere der Reste $R^9$ und $R^{12}$ die genannte Bedeutung hat, oder ein Salz einer solchen zur Salzbildung fähigen Verbindung herstellt.

9. Verfahren nach Anspruch 1, dadurch gekennzeichnet, dass man die Ausgangsstoffe so wählt, dass man
1,4,6-Tri-O-acetyl-N-propionyl-desmethylmuramyl-L-alanyl-D-isoglutamin-benzhydrylester,
1,4,6-Tri-O-acetyl-N-propionyl-desmethylmuramyl-L-alanyl-D-isoglutamin-benzylester,
1,4,6-Tri-O-acetyl-N-propionyl-desmethylmuramyl-L-alanyl-D-glutaminsäure-($C_\alpha$)-n-butylester-($C_\gamma$)-benzhydrylester,
N-Acetyl-1,4,6-tri-O-acetyl-muramyl-L-alanyl-D-glutaminsäure-($C_\alpha$)-pivaloyloxymethylester-($C_\gamma$)-benzylester,
1,4,6-Tri-O-acetyl-N-benzoyl-desmethylmuramyl-L-alanyl-D-isoglutamin-benzylester,
1,4,6-Tri-O-acetyl-N-benzoyl-desmethylmuramyl-L-alanyl-D-isoglutamin-benzhydrylester,
N-Benzoyl-1,4,6-tri-O-butyryl-desmethylmuramyl-L-alanyl-D-isoglutamin-benzhydrylester,
N-Acetyl-(1α,β),4,6-tri-O-acetyl-desmethylmuramyl-L-α-aminobutyryl-D-isoglutamin-benzhydrylester,
N-Acetyl-(1α,β),4,6-tri-O-acetyl-desmethylmuramyl-L-α-aminobutyryl-D-glutaminsäure-dibenzhydrylester,
N-Acetyl-(1α,β),4,6-tri-O-acetyl-desmethylmuramyl-L-valyl-D-isoglutamin-benzhydrylester,
N-Acetyl-(1α,β),4,6-tri-O-acetyl-muramyl-L-alanyl-D-isoglutaminyl-L-alanin-cholesteryl-3-ester,
N-Acetyl-1,4,6-tri-O-acetyl-muramyl-α-amino-isobutyryl-D-isoglutamin-benzhydrylester,
N-Acetyl-1,4,6-tri-O-acetyl-desmethylmuramyl-L-alanyl-D-γ-methoxycarbonyl-isoglutamin-benzhydrylester,
N-Acetyl-1,4,6-tri-O-acetyl-muramyl-L-alanyl-D-isoglutaminyl-L-α-([2-benzyloxy-carbonylamino-ethyl]-sulfonyl-methyl)-glycin-benzylester,

1,4,6-Tri-O-acetyl-N-benzoyl-desmethylmuramyl-L-alanyl-D-glutaminsäure-dicholinester,
N-Propionyl-1,4,6-tri-O-propionyl-desmethylmuramyl-L-alanyl-D-isoglutamin-benzhydrylester,
1,4,6-Tri-O-acetyl-N-propionyl-desmethylmuramyl-L-alanyl-D-glutaminsäure-α-n-butylester-γ-benzylester,
1,4,6-Tri-O-acetyl-N-propionyl-desmethylmuramyl-L-alanyl-D-glutaminsäure-dibenzylester,
1,4,6-Tri-O-acetyl-N-benzoyl-desmethylmuramyl-L-alanyl-D-isoglutamin-benzylester,
1,4,6-Tri-O-acetyl-N-propionyl-desmethylmuramyl-L-alanyl-D-glutaminsäure-dipivaloyloxymethylester oder
1,4,6-Tri-O-acetyl-N-propionyl-desmethylmuramyl-L-alanyl-D-isoglutaminyl-L-alanin-benzoyloxymethylester oder ein pharmazeutisch verwendbares Salz einer der obengenannten zur Salzbildung fähigen Verbindung herstellt.

FO 7.4 VBU/eg*